# EUROPEAN PATENT SPECIFICATION

(11) **EP 4 426 859 B1**
(45) Date of publication and mention of the grant of the patent: **15.04.2026**
(21) Application number: 22818972.6
(22) Date of filing: 01.11.2022
(51) Int. Cl.: C12Q 1/6869

(54) **PRIMER SETS AND METHODS AND KITS INCORPORATING THE PRIMER SETS**
PRIMERSÄTZE UND VERFAHREN UND KITS MIT DEN PRIMERSÄTZEN
ENSEMBLES D'AMORCES ET PROCÉDÉS ET KITS INCORPORANT LES ENSEMBLES D'AMORCES

(30) Priority: 02.11.2021 US 202163274768 P
(43) Date of publication of application: 11.09.2024
(73) Proprietor: Illumina, Inc., San Diego, CA 92122 (US)
(72) Inventor: ARMIJO, Esteban, San Diego, California 92122 (US); BOUTELL, Jonathan Mark, Cambridge Cambridgeshire CB21 6DF (GB); BRODIN, Jeffrey, San Diego, California 92122 (US); CARRAMI, Eli M., Cambridge Cambridgeshire CB21 6DF (GB); ROGERT BACIGALUPO, Maria Candelaria, San Diego, California 92122 (US); SMITH, Randall, San Diego, California 92122 (US); LESSARD-VIGER, Mathieu, San Diego, California 92122 (US); VINCENT, Ludovic, San Diego, California 92122 (US)
(74) Representative: Murgitroyd & Company
(86) International application number: PCT/US2022/079075
(87) International publication number: WO 2023/081654

(56) References cited:
- WO-A1-2019/222264
- WO-A1-2021/173666
- SAMUEL MYLLYKANGAS ET AL: "Efficient targeted resequencing of human germline and cancer genomes by oligonucleotide-selective sequencing", NATURE BIOTECHNOLOGY, vol. 29, no. 11, 23 October 2011 (2011-10-23), New York, pages 1024 - 1027, XP055544835, ISSN: 1087-0156, DOI: 10.1038/nbt.1996
- LIPWORTH SAMUEL ET AL: "Optimized use of Oxford Nanopore flowcells for hybrid assemblies", MICROBIAL GENOMICS, vol. 6, no. 11, 1 November 2020 (2020-11-01), XP093029220, Retrieved from the Internet <URL:https://www.ncbi.nlm.nih.gov/pmc/articles/PMC7725331/pdf/mgen-6-453.pdf> DOI: 10.1099/mgen.0.000453

## Description

### BACKGROUND

The detection of specific nucleic acids can be used for diagnostic medicine and molecular biology research. In a variety of nucleic acid detection methods and applications, such as gene sequencing, genotyping, etc., flow cells are used. The flow cell surface may be functionalized with specific surface chemistry, such as primer sets, polymerases, etc. depending upon the reaction that is to take place. In many instances, the surface chemistry is suitable for a one-time use detection method.

WO2019/222264 A1 describes methods of preparation of templates for polynucleotide sequencing. WO2021/173666 A1 describes kits for genotyping with examples including a flow cell and a genotyping probe fluid. Myllykangas et al (2011) Nature Biotechnology 29 (11) 1024-1027 describes an approach for targeted genome resequencing, called oligonucleotide-selective sequencing (OS-Seq), in which the authors modify the immobilized lawn of oligonucleotide primers of a next-generation DNA sequencer to function as both a capture and sequencing substrate Lipworth et al Microbial Genomics 2020;6 DOI 10.1099/mgen.0.000453 describes optimized use of multiplexed nanopore flowcells for hybrid assemblies.

### SUMMARY

Several primer sets are disclosed herein. Examples of the flow cells that include the respective primers sets are reusable. Some of the primer sets enable the flow cell to be used twice, with a different sample being analyzed during each of the uses. Some other of the primer sets enable the flow cell to be reused any desired number of times with any desired number of different samples being analyzed during the respective uses.

The invention provides a primer set as claimed in claim 1. Also provided is a reusable flow cell as claimed in claim 3, a method as claimed in claim 5, a flow cell as claimed in claim 9, a flow cell generation kit as claimed in claim 15, and a method as claimed in claim 17.

### BRIEF DESCRIPTION OF THE DRAWINGS

Features of examples of the present disclosure will become apparent by reference to the following detailed description and drawings, in which like reference numerals correspond to similar, though perhaps not identical, components. For the sake of brevity, reference numerals or features having a previously described function may or may not be described in connection with other drawings in which they appear.
Fig. 1A is a top view of an example of a flow cell;
Fig. 1B is an enlarged, and partially cutaway view of one example of the architecture within a flow channel of one example of the flow cell;
Fig. 1C is an enlarged, and partially cutaway view of another example of the architecture within a flow channel of another example of the flow cell;
Fig. 2 is a schematic view of an example of a primer set;

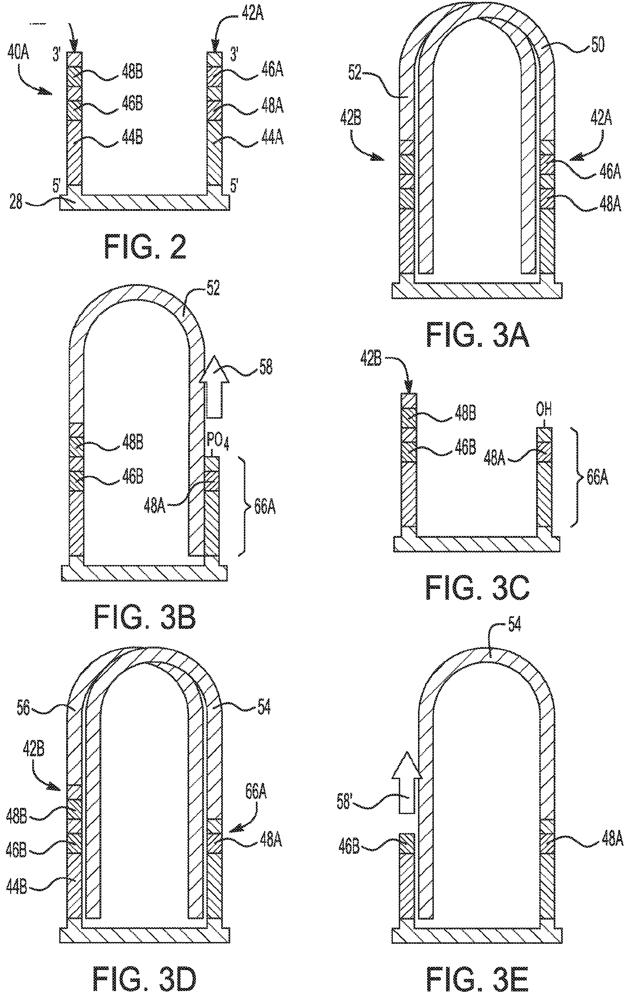

Fig. 3A through Fig. 3E are schematic views that together depict a method involving the primer set of Fig. 2, where Fig. 3A depicts first sample library fragment amplicons attached to the primers, Fig. 3B depicts the removal of a first plurality of the first sample library fragment amplicons and the sequencing of a second plurality of the first sample library fragment amplicons, Fig. 3C depicts the cleavage of the second plurality of the first sample library fragment amplicons and nascent stands resulting from sequencing, Fig. 3D depicts the generation of second sample library fragment amplicons, and Fig. 3E depicts the removal of a second plurality of the second sample library fragment amplicons and the sequencing of a first plurality of the second sample library fragment amplicons;
Fig. 4 is a schematic view of another example of a primer set;
Fig. 5A through Fig. 5D are schematic views that together depict a method involving the primer set of Fig. 4, where Fig. 5A depicts first sample library fragment amplicons attached to the primers, Fig. 5B depicts the removal of a first plurality of the first sample library fragment amplicons and the sequencing of a second plurality of the first sample library fragment amplicons, Fig. 5C depicts the cleavage of the second plurality of the first sample library fragment amplicons and nascent stands resulting from sequencing, and Fig. 5D depicts the introduction of regeneration oligonucleotides;
Fig. 6 is a schematic view of another example of a primer set;

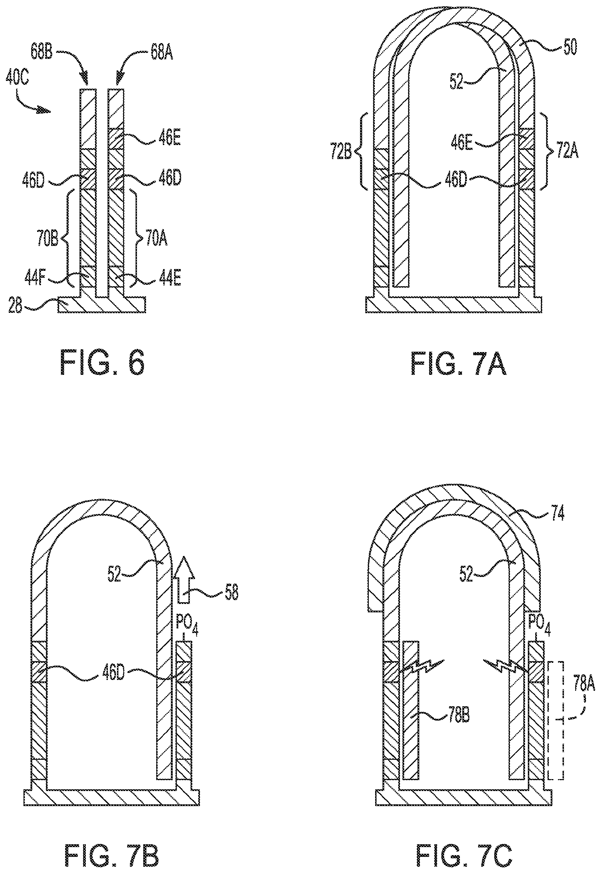

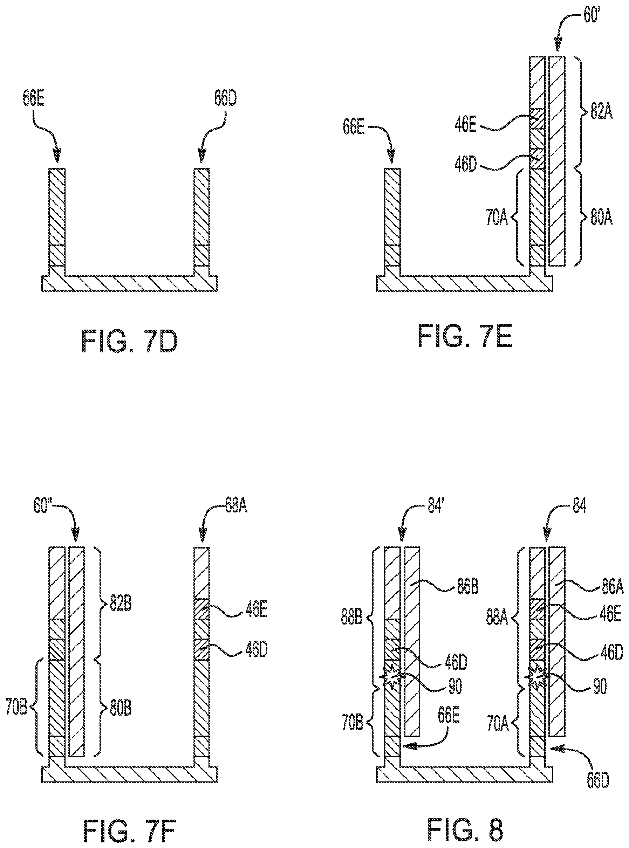

Fig. 7A through Fig. 7F are schematic views that together depict methods involving the primer set of Fig. 6, where Fig. 7A depicts first sample library fragment amplicons attached to the primers, Fig. 7B depicts the removal of a first plurality of the first sample library fragment amplicons and the sequencing of a second plurality of the first sample library fragment amplicons, Fig. 7C depicts the depicts the nascent stands resulting from sequencing, and the cleavage of the primers, Fig. 7D depicts the remaining portions of the primers after cleavage, and Fig. 7E and Fig. 7F depict the sequential regeneration of a cleaved portion of each of the primers;
Fig. 7A through Fig. 7D and Fig. 8 are schematic views that together depict another method involving the primer set of Fig. 6, where Fig. 7A depicts first sample library fragment amplicons attached to the primers, Fig. 7B depicts the removal of a first plurality of the first sample library fragment amplicons and the sequencing of a second plurality of the first sample library fragment amplicons, Fig. 7C depicts the depicts the nascent stands resulting from sequencing, and the cleavage of the primers, Fig. 7D depicts the remaining portions of the primers after cleavage, and Fig. 8 depicts the simultaneous regeneration of a cleaved portion of each of the primers;

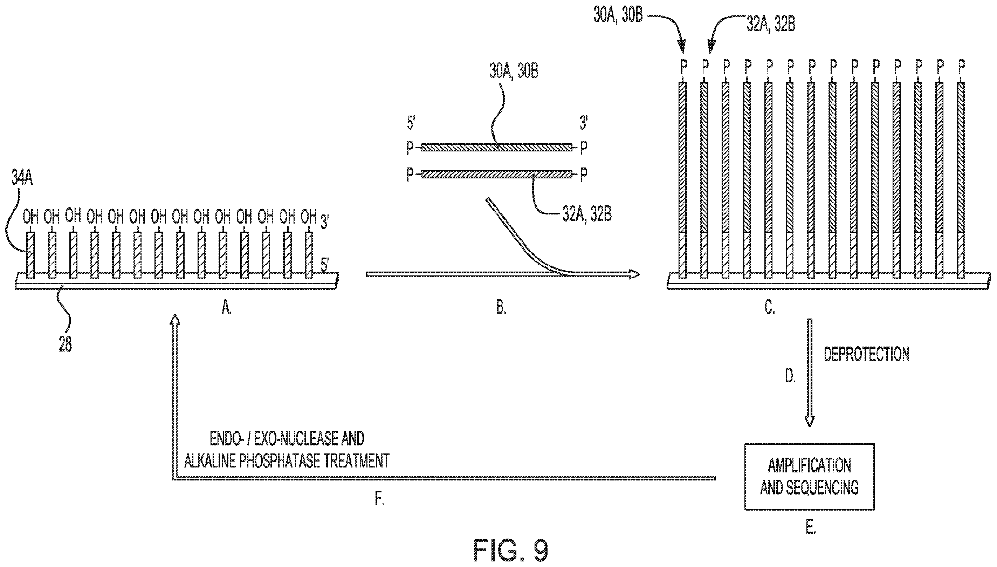

Fig. 9 is a schematic flow diagram illustrating an example method with two different types of primers and a flow cell surface with one example of the nuclease resistant anchor oligonucleotides disclosed herein;

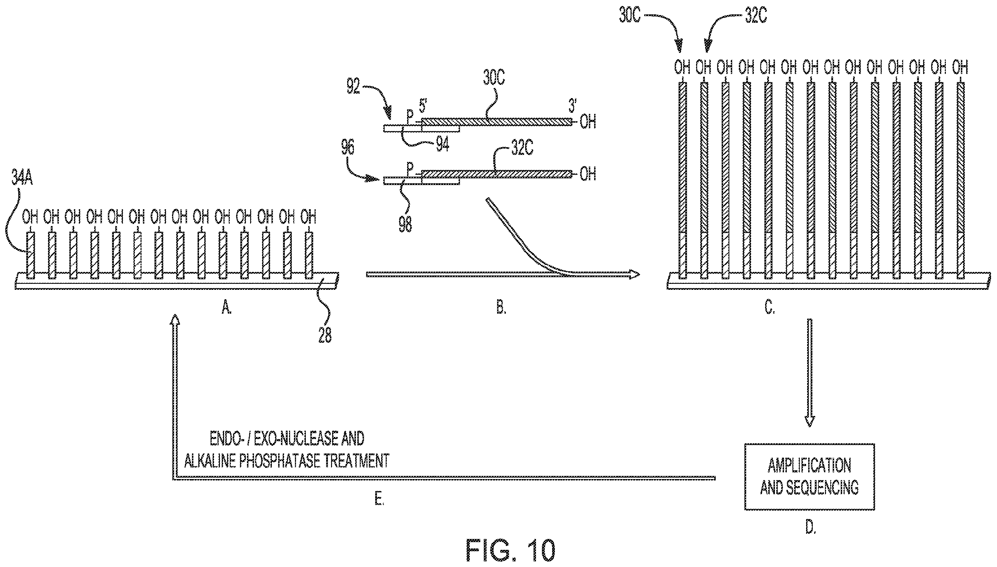

Fig. 10 is a schematic flow diagram illustrating an example method with splinted primers and the flow cell surface shown in Fig. 9; and

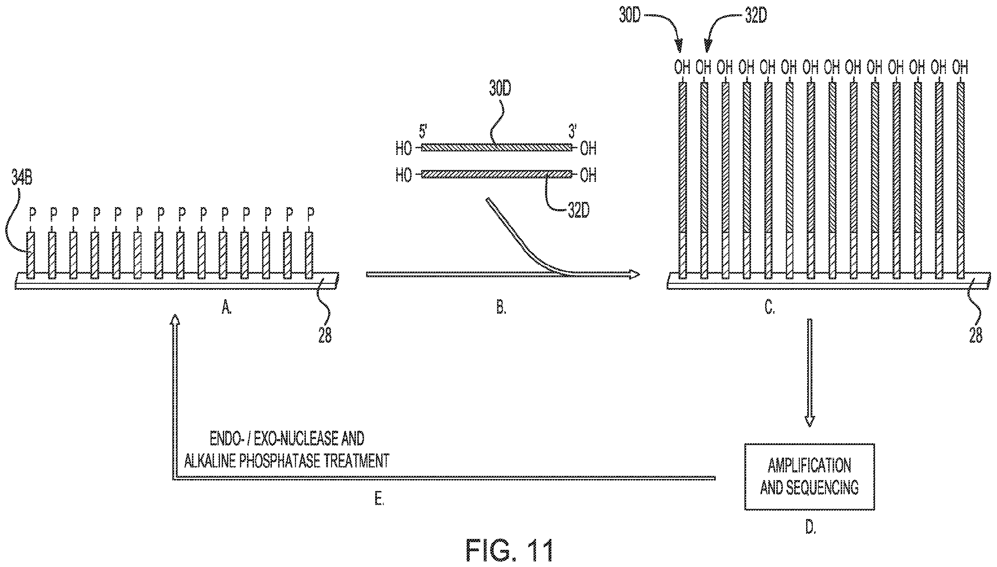

Fig. 11 is a schematic flow diagram illustrating an example method with another type of primers and a flow cell surface with another example of the nuclease resistant anchor oligonucleotides disclosed herein.

### DETAILED DESCRIPTION

Disclosed herein are several examples of oligonucleotide primer sets. Each of these primer sets may be used as part of the surface chemistry within a flow cell used for nucleic acid analysis. Each of these primer sets renders the flow cell reusable, either one time or multiple times. In some instances, the reusability of the flow cell may enable it to be part of a sequencing instrument, as opposed to part of a consumables set.

### Definitions

It is to be understood that terms used herein will take on their ordinary meaning in the relevant art unless specified otherwise. Several terms used herein and their meanings are set forth below.

The singular forms "a", "an", and "the" include plural referents unless the context clearly dictates otherwise.

The terms comprising, including, containing and various forms of these terms are synonymous with each other and are meant to be equally broad.

The terms top, bottom, lower, upper, on, etc. are used herein to describe the flow cell and/or the various components of the flow cell. It is to be understood that these directional terms are not meant to imply a specific orientation, but are used to designate relative orientation between components. The use of directional terms should not be interpreted to limit the examples disclosed herein to any specific orientation(s).

The terms first, second, etc. also are not meant to imply a specific orientation or order, but rather are used to distinguish one component from another.

It is to be understood that the ranges provided herein include the stated range and any value or sub-range within the stated range, as if such values or sub-ranges were explicitly recited. For example, a range of about 400 nm to about 1 µm (1000 nm), should be interpreted to include not only the explicitly recited limits of about 400 nm to about 1 µm, but also to include individual values, such as about 708 nm, about 945.5 nm, etc., and sub-ranges, such as from about 425 nm to about 825 nm, from about 550 nm to about 940 nm, etc.

Furthermore, when "about" and/or "substantially" are/is utilized to describe a value, they are meant to encompass minor variations (up to +/- 10%) from the stated value.

An "acrylamide monomer" is a monomer with the structure 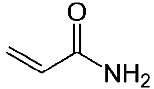 or a monomer including an acrylamide group. Examples of the monomer including an acrylamide group include azido acetamido pentyl acrylamide: 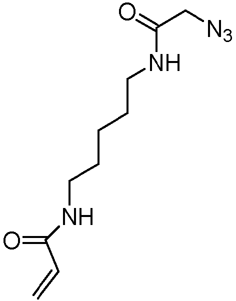 and N-isopropylacrylamide: 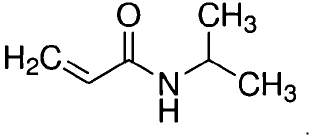 Other acrylamide monomers may be used.

The term "activation," as used herein, refers to a process that generates reactive groups at the surface of a base support or an outermost layer of a multi-layered structure. Activation may be accomplished using silanization or plasma ashing. While the figures do not depict a separate silanized layer or -OH groups from plasma ashing, it is to be understood that activation generates a silanized layer or - OH groups at the surface of the activated support or layer to covalently attach a polymeric hydrogel to the underlying support or layer.

An "aldehyde," as used herein, is an organic compound containing a functional group with the structure -CHO, which includes a carbonyl center (i.e., a carbon double-bonded to oxygen) with the carbon atom also bonded to hydrogen and an R group, such as an alkyl or other side chain. The general structure of an aldehyde is:

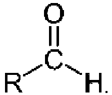

As used herein, "alkyl" refers to a straight or branched hydrocarbon chain that is fully saturated (i.e., contains no double or triple bonds). The alkyl group may have 1 to 20 carbon atoms. Example alkyl groups include methyl, ethyl, propyl, isopropyl, butyl, isobutyl, tertiary butyl, pentyl, hexyl, and the like. As an example, the designation "C1-4 alkyl" indicates that there are one to four carbon atoms in the alkyl chain, i.e., the alkyl chain is selected from the group consisting of methyl, ethyl, propyl, iso-propyl, n-butyl, isobutyl, sec-butyl, and t-butyl.

As used herein, "alkenyl" refers to a straight or branched hydrocarbon chain containing one or more double bonds. The alkenyl group may have 2 to 20 carbon atoms. Example alkenyl groups include ethenyl, propenyl, butenyl, pentenyl, hexenyl, and the like.

As used herein, "alkyne" or "alkynyl" refers to a straight or branched hydrocarbon chain containing one or more triple bonds. The alkynyl group may have 2 to 20 carbon atoms.

An "amine" or "amino" functional group refers to an -NRₐR_{b} group, where Rₐ and R_{b} are each independently selected from hydrogen (e.g., 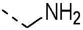 ), C1-6 alkyl, C2-6 alkenyl, C2-6 alkynyl, C3-7 carbocycle, C6-10 aryl, 5-10 membered heteroaryl, and 5-10 membered heterocyclyl, as defined herein.

"Anchor oligonucleotides" or "nuclease resistant anchor nucleotides" are single stranded DNA strands that are attached to the polymeric hydrogel, that include a nuclease resistant modification, and that are capable of ligating primers of a primer set.

As used herein, "aryl" refers to an aromatic ring or ring system (i.e., two or more fused rings that share two adjacent carbon atoms) containing only carbon in the ring backbone. When the aryl is a ring system, every ring in the system is aromatic. The aryl group may have 6 to 18 carbon atoms. Examples of aryl groups include phenyl, naphthyl, azulenyl, and anthracenyl.

The phrase "incorporated at a 3' end" or "incorporated at a 5' end" means that a nucleotide or a nucleotide analog is attached at the respective terminal end or is attached near the respective terminal end. By "near," it is meant that the nucleotide or a nucleotide analog is within 10 nucleotides from the terminal end. As examples, a cleavage site or nuclease resistant modification may be attached at the 3' end, or a cleavage site may be attached at the 3^{rd}, 5^{th} or 7^{th} nucleotide position from the 3' end, or nuclease resistant modifications may be incorporated between the last three nucleotides at the 3' end.

As used herein, the term "attached" refers to the state of two things being joined, fastened, adhered, connected or bound to each other, either directly or indirectly. For example, a nucleic acid can be attached to a polymeric hydrogel by a covalent or non-covalent bond. A covalent bond is characterized by the sharing of pairs of electrons between atoms. A non-covalent bond is a physical bond that does not involve the sharing of pairs of electrons and can include, for example, hydrogen bonds, ionic bonds, van der Waals forces, hydrophilic interactions and hydrophobic interactions.

An "azide" or "azido" functional group refers to -N₃.

As used herein, a "bonding region" refers to an area of a patterned structure that is to be bonded to another material, which may be, as examples, a spacer layer, a lid, another patterned structure, etc., or combinations thereof (e.g., a spacer layer and a lid, or a spacer layer and another patterned structure). The bond that is formed at the bonding region may be a chemical bond (as described herein), or a mechanical bond (e.g., using a fastener, etc.).

As used herein, "carbocycle" means a non-aromatic cyclic ring or ring system containing only carbon atoms in the ring system backbone. When the carbocycle is a ring system, two or more rings may be joined together in a fused, bridged or spiro-connected fashion. Carbocycles may have any degree of saturation, provided that at least one ring in a ring system is not aromatic. Thus, carbocycles include cycloalkyls, cycloalkenyls, and cycloalkynyls. The carbocycle group may have 3 to 20 carbon atoms. Examples of carbocycle rings include cyclopropyl, cyclobutyl, cyclopentyl, cyclohexyl, cyclohexenyl, 2,3-dihydro-indene, bicyclo[2.2.2]octanyl, adamantyl, and spiro[4.4]nonanyl.

As used herein, the term "carboxylic acid" or "carboxyl" as used herein refers to -COOH.

As used herein, "cycloalkylene" means a fully saturated carbocycle ring or ring system that is attached to the rest of the molecule via two points of attachment.

As used herein, "cycloalkenyl" or "cycloalkene" means a carbocycle ring or ring system having at least one double bond, wherein no ring in the ring system is aromatic. Examples include cyclohexenyl or cyclohexene and norbornenyl or norbornene. Also as used herein, "heterocycloalkenyl" or "heterocycloalkene" means a carbocycle ring or ring system with at least one heteroatom in ring backbone, having at least one double bond, wherein no ring in the ring system is aromatic.

As used herein, "cycloalkynyl" or "cycloalkyne" means a carbocycle ring or ring system having at least one triple bond, wherein no ring in the ring system is aromatic. An example is cyclooctyne. Another example is bicyclononyne. Also as used herein, "heterocycloalkynyl" or "heterocycloalkyne" means a carbocycle ring or ring system with at least one heteroatom in ring backbone, having at least one triple bond, wherein no ring in the ring system is aromatic.

The term "depositing," as used herein, refers to any suitable application technique, which may be manual or automated, and, in some instances, results in modification of the surface properties. Generally, depositing may be performed using vapor deposition techniques, coating techniques, grafting techniques, or the like. Some specific examples include chemical vapor deposition (CVD), spray coating (e.g., ultrasonic spray coating), spin coating, dunk or dip coating, doctor blade coating, puddle dispensing, flow through coating, aerosol printing, screen printing, microcontact printing, inkjet printing, or the like.

As used herein, the term "depression" refers to a discrete concave feature in a base support or a layer of a multi-layer stack having a surface opening that is at least partially surrounded by interstitial region(s) of the base support or a layer of a multi-layer stack. Depressions can have any of a variety of shapes at their opening in a surface including, as examples, round, elliptical, square, polygonal, star shaped (with any number of vertices), etc. The cross-section of a depression taken orthogonally with the surface can be curved, square, polygonal, hyperbolic, conical, angular, etc. As examples, the depression can be a well or two interconnected wells. The depression may also have more complex architectures, such as ridges, step features, etc. An example of a depression having a step feature is referred to herein as a multidepth depression, where the step feature defines the shallow portion.

The term "each," when used in reference to a collection of items, is intended to identify an individual item in the collection, but does not necessarily refer to every item in the collection. Exceptions can occur if explicit disclosure or context clearly dictates otherwise.

The term "epocy" (also referred to as a glycidyl or oxirane group) as used herein refers to

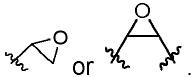

As used herein, the term "flow cell" is intended to mean a vessel having a flow channel where a reaction can be carried out, an inlet for delivering reagent(s) to the flow channel, and an outlet for removing reagent(s) from the flow channel. In some examples, the flow cell accommodates the detection of the reaction that occurs in the flow cell. For example, the flow cell can include one or more transparent surfaces allowing for the optical detection of arrays, optically labeled molecules, or the like.

As used herein, a "flow channel" or "channel" may be an area defined between two bonded components, which can selectively receive a liquid sample. In some examples, the flow channel may be defined between two patterned structures, and thus may be in fluid communication with surface chemistry of the patterned structures. In other examples, the flow channel may be defined between one patterned structure and a lid, and thus may be in fluid communication with surface chemistry of the one patterned structure.

As used herein, "heteroaryl" refers to an aromatic ring or ring system (i.e., two or more fused rings that share two adjacent atoms) that contain(s) one or more heteroatoms, that is, an element other than carbon, including but not limited to, nitrogen, oxygen and sulfur, in the ring backbone. When the heteroaryl is a ring system, every ring in the system is aromatic. The heteroaryl group may have 5-18 ring members.

As used herein, "heterocycle" means a non-aromatic cyclic ring or ring system containing at least one heteroatom in the ring backbone. Heterocycles may be joined together in a fused, bridged or spiro-connected fashion. Heterocycles may have any degree of saturation provided that at least one ring in the ring system is not aromatic. In the ring system, the heteroatom(s) may be present in either a non-aromatic or aromatic ring. The heterocycle group may have 3 to 20 ring members (i.e., the number of atoms making up the ring backbone, including carbon atoms and heteroatoms). In some examples, the heteroatom(s) are O, N, or S.

The term "hydrazine" or "hydrazinyl" as used herein refers to a -NHNH₂ group.

As used herein, the term "hydrazone" or "hydrazonyl" as used herein refers to a 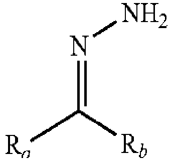 group in which Rₐ and R_{b} are each independently selected from hydrogen, C1-6 alkyl, C2-6 alkenyl, C2-6 alkynyl, C3-7 carbocycle, C6-10 aryl, 5-10 membered heteroaryl, and 5-10 membered heterocycle, as defined herein.

As used herein, "hydroxy" or "hydroxyl" refers to an -OH group.

As used herein, the term "interstitial region" refers to an area, e.g., of a base support or a layer of a multi-layer stack that separates depressions (concave regions). For example, an interstitial region can separate one depression of an array from another depression of the array. The two depressions that are separated from each other can be discrete, i.e., lacking physical contact with each other. In many examples, the interstitial region is continuous, whereas the depressions are discrete, for example, as is the case for a plurality of depressions defined in an otherwise continuous surface. In other examples, the interstitial regions and the features are discrete, for example, as is the case for a plurality of depressions in the shape of trenches, which are separated by respective interstitial regions. The separation provided by an interstitial region can be partial or full separation. Interstitial regions may have a surface material that differs from the surface material of the depressions. For example, depressions can have a polymeric hydrogel and primer sets therein, and the interstitial regions can be free of the polymeric hydrogel and primer sets.

A "library fragment" or "library template strand" is a single stranded deoxyribonucleic acid sequence that is to be sequenced. The library template strand may be prepared from a larger DNA sample, and may be modified to include adapters at each end that are respectively complementary to at least a portion of the primers in a primer set. The library template strand serves as a template for cluster generation, which generates a plurality of amplicons.

"Nitrile oxide," as used herein, means a "RₐC≡N⁺O⁻" group in which Rₐ is defined herein. Examples of preparing nitrile oxide include *in situ* generation from aldoximes by treatment with chloramide-T or through action of base on imidoyl chlorides [RC(Cl)=NOH] or from the reaction between hydroxylamine and an aldehyde.

"Nitrone," as used herein, means a 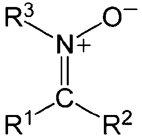 group in which R¹, R², and R³ may be any of the Rₐ and R_{b} groups defined herein, except that R³ is not hydrogen (H).

As used herein, a "nucleotide" includes a nitrogen containing heterocyclic base, a sugar, and one or more phosphate groups. Nucleotides are monomeric units of a nucleic acid sequence. In RNA, the sugar is a ribose, and in DNA, the sugar is a deoxyribose, i.e. a sugar lacking a hydroxyl group that is present at the 2' position in ribose. The nitrogen containing heterocyclic base (i.e., nucleobase) can be a purine base or a pyrimidine base. Purine bases include adenine (A) and guanine (G), and modified derivatives or analogs thereof. Pyrimidine bases include cytosine (C), thymine (T), and uracil (U), and modified derivatives or analogs thereof. The C-1 atom of deoxyribose is bonded to N-1 of a pyrimidine or N-9 of a purine. A nucleic acid analog may have any of the phosphate backbone, the sugar, or the nucleobase altered. Examples of nucleic acid analogs include, for example, universal bases or phosphate-sugar backbone analogs, such as peptide nucleic acids (PNA), phosphorothioate backbone modification, etc.

In some examples, the term "over" may mean that one component or material is positioned directly on another component or material. When one is directly on another, the two are in contact with each other.

In other examples, the term "over" may mean that one component or material is positioned indirectly on another component or material. By indirectly on, it is meant that a gap or an additional component or material may be positioned between the two components or materials.

A "patterned structure" refers to a single layer base support that includes, or a multi-layer stack with a layer that includes, surface chemistry positioned in a pattern, e.g., in depressions, across the single layer base support or the layer. In contrast, a "non-patterned structure" refers to a single layer base support that includes, or a multi-layer stack with a layer that includes, surface chemistry positioned in no particular pattern across the single layer base support or layer. The surface chemistry may include a polymeric hydrogel and one example of the primer sets disclosed herein.

As used herein, the term "polyhedral oligomeric silsesquioxane" refers to a chemical composition that is a hybrid intermediate (e.g., RSiO_{1.5}) between that of silica (SiO₂) and silicone (R₂SiO). An example of polyhedral oligomeric silsesquioxane may be that described in Kehagias et al., Microelectronic Engineering 86 (2009), pp. 776-778. In an example, the composition is an organosilicon compound with the chemical formula [RSiO_{3/2}]ₙ, where the R groups can be the same or different. Example R groups for polyhedral oligomeric silsesquioxane include epoxy, azide/azido, a thiol, a poly(ethylene glycol), a norbornene, a tetrazine, acrylates, and/or methacrylates, or further, for example, alkyl, aryl, alkoxy, and/or haloalkyl groups.

As used herein, the term "primer set" is defined as two single stranded nucleic acid sequences (e.g., single strand DNA) that together enable the amplification of a library fragment. The primers in a primer set may be referred to herein as amplification primers because they serve as a starting point for library fragment amplification and cluster generation. The primers in a primer set may include a sequence that is complementary to a portion (e.g., an adapter) of the library fragment such that it is able to hybridize to, and thus capture, the library fragment. The 5' terminus of the primers in any examples of the primer set may be modified to allow a coupling reaction with a functional group of a polymeric hydrogel. The length of each primer in a primer set can be any number of bases long and can include a variety of natural and/or non-natural nucleotides.

Some of the primer sets disclosed herein include nuclease resistant primers. A "nuclease resistant primer" is a single stranded nucleic acid sequence that includes a nuclease resistant modification at some point along the sequence. A "nuclease resistant modification" is a bond, a nucleotide analog, or some other alteration to the nucleic acid sequence that confers the nuclease resistant primers 42A, 42B. The resistance may be to endonucleases and/or exonucleases.

Other primers, such as "sequencing primers" are not part of a primer set, but rather serve as a starting point for DNA synthesis. In an example, the sequencing primer is a short strand, ranging from 10 to 60 bases, or from 20 to 40 bases.

A "spacer layer," as used herein refers to a material that bonds two components together. In some examples, the spacer layer can be a radiation absorbing material that aids in bonding, or can be put into contact with a radiation absorbing material that aids in bonding.

The term "substrate" refers to the single layer base support or a multi-layer structure upon which surface chemistry is introduced.

A "thiol" functional group refers to -SH.

As used herein, the terms "tetrazine" and "tetrazinyl" refer to six-membered heteroaryl group comprising four nitrogen atoms. Tetrazine can be optionally substituted.

"Tetrazole," as used herein, refer to five-membered heterocyclic group including four nitrogen atoms. Tetrazole can be optionally substituted.

### Flow Cells

Some examples of the flow cell disclosed herein include a substrate, a polymeric hydrogel attached to at least a portion of the substrate, and an example of the primer set disclosed herein attached to the polymeric hydrogel. In one example of the flow cell, the primer set is selected from the group consisting of i) a first nuclease resistant primer including a first cleavage site, and a second nuclease resistant primer including a second cleavage site that is different from the first cleavage site; and ii) a first nuclease resistant primer including a cleavage site, and a second nuclease resistant primer without any cleavage site. In another example of the flow cell, the primer set includes a first primer having a first cleavage site and a second cleavage site that is different from the first cleavage site; and a second primer having the first cleavage site without the second cleavage site.

Other examples of the flow cell disclosed herein include a substrate, a polymeric hydrogel attached to at least a portion of the substrate, and first and second nuclease resistant anchor oligonucleotides attached to the polymeric hydrogel.

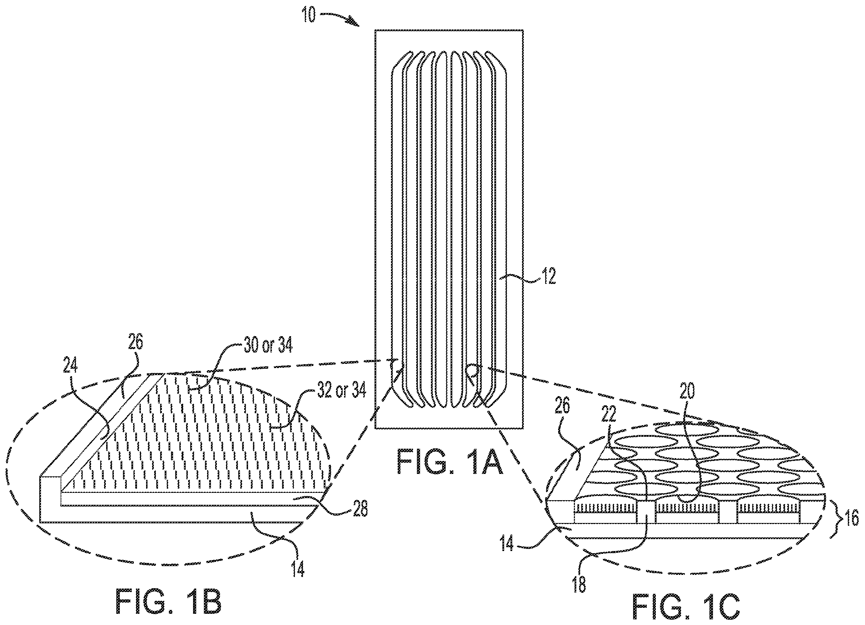

The substrate and the polymeric hydrogel of the flow cells will be described in reference to Fig. 1A through Fig. 1C, while the different primer sets will be described in reference to Fig. 2 through Fig. 8 and the anchor oligonucleotides will be described in reference to Fig. 9 through Fig. 11.

One example of the flow cell 10 is shown in Fig. 1A from a top view. The flow cell 10 may include two patterned or non-patterned structures bonded together or one patterned or non-patterned structure bonded to a lid (lid not shown). Between the two patterned or non-patterned structures or the one patterned or non-patterned structure and the lid is a flow channel 12. The example shown in Fig. 1A includes eight flow channels 12. While eight flow channels 12 are shown in Fig. 1A, it is to be understood that any number of flow channels 12 may be included in the flow cell 10 (e.g., a single flow channel 12, four flow channels 12, etc.). Each flow channel 12 may be isolated from each other flow channel 12 so that fluid introduced into a flow channel 12 does not flow into adjacent flow channel(s) 12. Some examples of the fluids introduced into the flow channel 12 may introduce reaction components (e.g., primer fluids, DNA sample, polymerases, sequencing primers, nucleotides, etc.), washing solutions, deblocking agents, etc.

Each flow channel 12 is in fluid communication with an inlet and an outlet (not shown). The inlet and outlet of each flow channel 12 may be positioned at opposed ends of the flow cell 10. The inlets and outlets of the respective flow channels 12 may alternatively be positioned anywhere along the length and width of the flow channel 12 that enables desirable fluid flow.

The inlet allows fluids to be introduced into the flow channel 12, and the outlet allows fluid to be extracted from the flow channel 12. Each of the inlets and outlets is fluidly connected to a fluidic control system (including, e.g., reservoirs, pumps, valves, waste containers, and the like) which controls fluid introduction and expulsion.

The flow channel 12 is at least partially defined by a patterned structure or a non-patterned structure. Each of these structures includes a substrate, such as the single layer base support 14 (as shown in Fig. 1B), or a multi-layered structure 16 (as shown in Fig. 1C).

Examples of suitable single layer base supports 14 include epoxy siloxane, glass, modified or functionalized glass, plastics (including acrylics, polystyrene and copolymers of styrene and other materials, polypropylene, polyethylene, polybutylene, polyurethanes, polytetrafluoroethylene (such as TEFLON^{®} from Chemours), cyclic olefins/cyclo-olefin polymers (COP) (such as ZEONOR^{®} from Zeon), polyimides, etc.), nylon (polyamides), ceramics/ceramic oxides, silica, fused silica, or silica-based materials, aluminum silicate, silicon and modified silicon (e.g., boron doped p+ silicon), silicon nitride (Si₃N₄), silicon oxide (SiO₂), tantalum pentoxide (Ta₂O₅) or other tantalum oxide(s) (TaOₓ), hafnium oxide (HfO₂), carbon, metals, inorganic glasses, or the like.

Examples of the multi-layered structure 16 include the base support 14 and at least one other layer 18 on the base support 16. Some examples of the multi-layered structure 16 include glass or silicon as the base support 14, with a coating layer 18 of tantalum oxide (e.g., tantalum pentoxide or another tantalum oxide(s) (TaOₓ)) or another ceramic oxide at the surface. Other examples of the multi-layered structure 16 include the base support 14 (e.g., glass, silicon, tantalum pentoxide, or any of the other base support materials) and a patterned resin as the other layer 18. It is to be understood that any material that can be selectively deposited, or deposited and patterned to form depressions 20 (see Fig. 1B) and interstitial regions 22 may be used for the patterned resin.

As one example of the patterned resin, an inorganic oxide may be selectively applied to the base support 14 via vapor deposition, aerosol printing, or inkjet printing. Examples of suitable inorganic oxides include tantalum oxide (e.g., Ta₂O₅), aluminum oxide (e.g., Al₂O₃), silicon oxide (e.g., SiO₂), hafnium oxide (e.g., HfO₂), etc.

As another example of the patterned resin, a polymeric resin may be applied to the base support 14 and then patterned. Suitable deposition techniques include chemical vapor deposition, dip coating, dunk coating, spin coating, spray coating, puddle dispensing, ultrasonic spray coating, doctor blade coating, aerosol printing, screen printing, microcontact printing, etc. Suitable patterning techniques include photolithography, nanoimprint lithography (NIL), stamping techniques, embossing techniques, molding techniques, microetching techniques, etc. Some examples of suitable resins include a polyhedral oligomeric silsesquioxane resin based resin, an epoxy resin not based on a polyhedral oligomeric silsesquioxane, a poly(ethylene glycol) resin, a polyether resin (e.g., ring opened epoxies), an acrylic resin, an acrylate resin, a methacrylate resin, an amorphous fluoropolymer resin (e.g., CYTOP^{®} from Bellex), and combinations thereof.

In an example, the single base support 14 (whether used singly or as part of the multi-layered structure 16) may be a circular sheet, a panel, a wafer, a die etc. having a diameter ranging from about 2 mm to about 300 mm, e.g., from about 200 mm to about 300 mm, or may be a rectangular sheet, panel, wafer, die etc. having its largest dimension up to about 10 feet (~ 3 meters). For example, a die may have a width ranging from about 0.1 mm to about 10 mm. While example dimensions have been provided, it is to be understood that a single base support with any suitable dimensions may be used.

In an example, the flow channel 12 has a substantially rectangular configuration (e.g., with curved ends as shown in Fig. 1A). The length and width of the flow channel 12 may be selected so a portion of the base support 14 or the multi-layer structure 16 of the flow cell 10 surrounds the flow channel 12 and is available for attachment to the lid (not shown) or another patterned or non-patterned structure.

The depth of the flow channel 12 can be as small as a monolayer thick when microcontact, aerosol, or inkjet printing is used to deposit a separate material (not shown) that defines the flow channel 12 walls. For other examples, the depth of the flow channel 12 can be about 1 µm, about 10 µm, about 50 µm, about 100 µm, or more. In an example, the depth may range from about 10 µm to about 100 µm. In another example, the depth may range from about 10 µm to about 30 µm. In still another example, the depth is about 5 µm or less. It is to be understood that the depth of the flow channel 12 may be greater than, less than or between the values specified above.

Fig. 1B and Fig. 1C depict examples of the architecture within the flow channel 12.

The architecture shown in Fig. 1B is a non-patterned structure. The substrate of the non-patterned structure may be the single layer base support 14 or the multi-layer structure 16. In the example shown in Fig. 1B, a lane 24 is defined in the surface of the single layer base support 14. Alternatively, the lane 24 may be defined in the surface, e.g., in layer 18, of the multi-layer structure 16.

The lane 24 is surrounded by edge regions 26 of the substrate in which it is formed. The edge regions 26 provide bonding regions where two non-patterned structures can be attached to one another or where one non-patterned structure can be attached to a lid.

The lane 24 may be formed using any suitable patterning techniques, such as photolithography, nanoimprint lithography (NIL), stamping techniques, embossing techniques, molding techniques, microetching techniques, etc.

The lane 24 provides a designated area for the polymeric hydrogel 28.

In an example, the polymeric hydrogel 28 includes an acrylamide copolymer. In this example, the acrylamide copolymer has a structure: 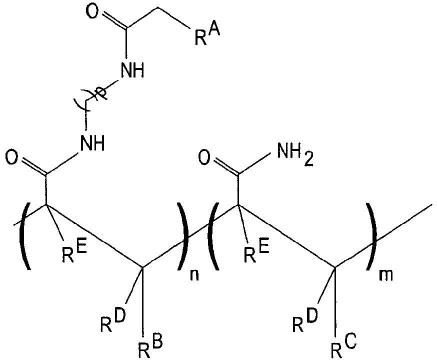 wherein:
R^{A} is selected from the group consisting of azido, optionally substituted amino, optionally substituted alkenyl, optionally substituted alkyne, halogen, optionally substituted hydrazone, optionally substituted hydrazine, carboxyl, hydroxy, optionally substituted tetrazole, optionally substituted tetrazine, nitrile oxide, nitrone, sulfate, and thiol;
R^{B} is H or optionally substituted alkyl;
R^{C}, R^{D}, and R^{E} are each independently selected from the group consisting of H and optionally substituted alkyl;
each of the -(CH₂)ₚ- can be optionally substituted;
p is an integer in the range of 1 to 50;
n is an integer in the range of 1 to 50,000; and
m is an integer in the range of 1 to 100,000.

One specific example of the acrylamide copolymer represented by structure (I) is poly(N-(5-azidoacetamidylpentyl)acrylamide-co-acrylamide, PAZAM.

One of ordinary skill in the art will recognize that the arrangement of the recurring "n" and "m" features in structure (I) are representative, and the monomeric subunits may be present in any order in the polymer structure (e.g., random, block, patterned, or a combination thereof).

The molecular weight of the acrylamide copolymer may range from about 5 kDa to about 1500 kDa or from about 10 kDa to about 1000 kDa, or may be, in a specific example, about 312 kDa.

In some examples, the acrylamide copolymer is a linear polymer. In some other examples, the acrylamide copolymer is a lightly cross-linked polymer.

In some examples, the polymeric hydrogel 28 may be a variation of structure (I). In one example, the acrylamide unit may be replaced with N,N-dimethylacrylamide ( 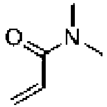 ). In another example, the acrylamide unit in structure (I) may be replaced with, 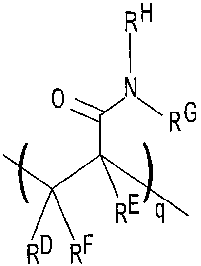 where R^{D}, R^{E}, and R^{F} are each H or a C1-C6 alkyl, and R^{G} and R^{H} are each a C1-C6 alkyl (instead of H as is the case with the acrylamide). In this example, q may be an integer in the range of 1 to 100,000. In another example, the N,N-dimethylacrylamide may be used in addition to the acrylamide unit. In this example, structure (I) may include 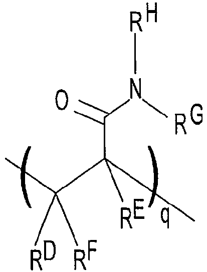 in addition to the recurring "n" and "m" features, where R^{D}, R^{E}, and R^{F} are each H or a C1-C6 alkyl, and R^{G} and R^{H} are each a C1-C6 alkyl. In this example, q may be an integer in the range of 1 to 100,000.

As another example of the polymeric hydrogel 28, the recurring "n" feature in structure (I) may be replaced with a monomer including a heterocyclic azido group having structure (II): 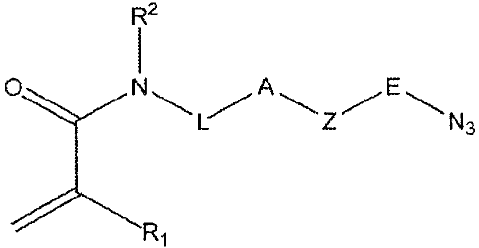 wherein R¹ is H or a C1-C6 alkyl; R₂ is H or a C1-C6 alkyl; L is a linker including a linear chain with 2 to 20 atoms selected from the group consisting of carbon, oxygen, and nitrogen and 10 optional substituents on the carbon and any nitrogen atoms in the chain; E is a linear chain including 1 to 4 atoms selected from the group consisting of carbon, oxygen and nitrogen, and optional substituents on the carbon and any nitrogen atoms in the chain; A is an N substituted amide with an H or a C1-C4 alkyl attached to the N; and Z is a nitrogen containing heterocycle. Examples of Z include 5 to 10 carbon-containing ring members present as a single cyclic structure or a fused structure. Some specific examples of Z include pyrrolidinyl, pyridinyl, or pyrimidinyl. As still another example, the polymeric hydrogel 28 may include a recurring unit of each of structure (III) and (IV): 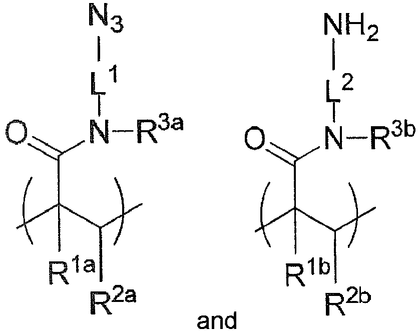 wherein each of R^{1a}, R^{2a}, R^{1b} and R^{2b} is independently selected from hydrogen, an optionally substituted alkyl or optionally substituted phenyl; each of R^{3a} and R^{3b} is independently selected from hydrogen, an optionally substituted alkyl, an optionally substituted phenyl, or an optionally substituted C7-C14 aralkyl; and each L¹ and L² is independently selected from an optionally substituted alkylene linker or an optionally substituted heteroalkylene linker.

In still another example, the acrylamide copolymer is formed using nitroxide mediated polymerization, and thus at least some of the copolymer chains have an alkoxyamine end group. In the copolymer chain, the term "alkoxyamine end group" refers to the dormant species -ONR₁R₂, where each of R₁ and R₂ may be the same or different, and may independently be a linear or branched alkyl, or a ring structure, and where the oxygen atom is attached to the rest of the copolymer chain. In some examples, the alkoxyamine may also be introduced into some of the recurring acrylamide monomers, e.g., at position R^{A} in structure (I). As such, in one example, structure (I) includes an alkoxyamine end group; and in another example, structure (I) includes an alkoxyamine end group and alkoxyamine groups in at least some of the side chains.

It is to be understood that other molecules may be used to form the polymeric hydrogel 28 as long as they are capable of being functionalized with the desired chemistry, e.g., a primer set or anchor oligonucleotides. Some examples of suitable materials for the polymeric hydrogel 28 include functionalized silanes, such as norbornene silane, azido silane, alkyne functionalized silane, amine functionalized silane, maleimide silane, or any other silane having functional groups that can respectively attach the desired chemistry.

Still other examples of suitable materials for the polymeric hydrogel 28 include those having a colloidal structure, such as agarose; or a polymer mesh structure, such as gelatin; or a cross-linked polymer structure, such as polyacrylamide polymers and copolymers, silane free acrylamide (SFA), or an azidolyzed version of SFA. Examples of suitable polyacrylamide polymers may be synthesized from acrylamide and an acrylic acid or an acrylic acid containing a vinyl group, or from monomers that form [2+2] photo-cycloaddition reactions. Still other examples of suitable materials for the polymeric hydrogel 28 include mixed copolymers of acrylamides and acrylates. A variety of polymer architectures containing acrylic monomers (e.g., acrylamides, acrylates etc.) may be utilized in the examples disclosed herein, such as branched polymers, including dendrimers (e.g., multi-arm or star polymers). For example, the monomers (e.g., acrylamide, etc.) may be incorporated, either randomly or in block, into the branches (arms) of a dendrimer.

The attachment of the polymeric hydrogel 28 to the underlying single layer base support 14 or multi-layer structure 16 may be through covalent bonding. In some instances, the single layer base support 14 or multi-layer structure 16 may first be activated, e.g., through silanization or plasma ashing. Covalent linking is helpful for maintaining the primer set or anchor oligonucleotides in the desired regions throughout the lifetime of the flow cell 10 during a variety of uses.

To introduce the polymeric hydrogel 28 into the lane 24, a mixture of the polymeric hydrogel 28 may be generated and then applied to the single layer base support 14 or the multi-layered structure 16. In one example, the polymeric hydrogel 28 may be present in a mixture (e.g., with water or with ethanol and water). The mixture may then be applied to the substrate surface using spin coating, or dipping or dip coating, or flow of the material under positive or negative pressure, or another suitable technique. These types of techniques blanketly deposit the polymeric hydrogel 28 in the lane 24 and on the edge regions 26. Other selective deposition techniques (e.g., involving a mask, controlled printing techniques, etc.) may be used to specifically deposit the polymeric hydrogel 28 in the lane 24 and not on the edge regions 26.

Depending upon the chemistry of the polymeric hydrogel 28, the applied mixture may be exposed to a curing process. In an example, curing may take place at a temperature ranging from room temperature (e.g., about 25°C) to about 95°C for a time ranging from about 1 millisecond to about several days.

Polishing may then be performed in order to remove the polymeric hydrogel 28 from the edge regions 26, while leaving the polymeric hydrogel 28 on the surface in the lane 24 at least substantially intact. The polishing process may be performed with a chemical slurry (including, e.g., an abrasive, a buffer, a chelating agent, a surfactant, and/or a dispersant) which can remove the polymeric hydrogel 28 from the edge regions 26 without deleteriously affecting the underlying substrate at those regions 26. Alternatively, polishing may be performed with a solution that does not include the abrasive particles.

The chemical slurry may be used in a chemical mechanical polishing system to polish the surface of the edge regions 26. The polishing head(s)/pad(s) or other polishing tool(s) is/are capable of polishing the polymeric hydrogel 28 that may be present over the edge regions 26 while leaving the polymeric hydrogel 28 in the lane 24 at least substantially intact. As an example, the polishing head may be a Strasbaugh ViPRR II polishing head.

Cleaning and drying processes may be performed after polishing. The cleaning process may utilize a water bath and sonication. The water bath may be maintained at a relatively low temperature ranging from about 22°C to about 30°C. The drying process may involve spin drying, or drying via another suitable technique.

In some examples, the polymeric hydrogel 28 includes primers 30, 32 of a primer set attached thereto (described further in reference to Fig. 2, Fig. 4 and Fig. 6). In other examples, the polymeric hydrogel 28 includes anchor oligonucleotides 34 attached thereto (described further in reference to Fig. 9 through Fig. 11).

In some examples, the primers 30, 32 or anchor oligonucleotides 34 may be pre-grafted to the polymeric hydrogel 28. In these examples, additional primer grafting is not performed. In other examples, the primers 30, 32 or anchor oligonucleotides 34 are not pre-grafted to the polymeric hydrogel 28. In these examples, the primers 30, 32 or anchor oligonucleotides 34 may be grafted after the polymeric hydrogel 28 is applied to the lane 24.

When grafting is performed after the polymeric hydrogel 28 is applied, grafting may be accomplished using any suitable grafting techniques. As examples, grafting may be accomplished by flow through deposition (e.g., using a temporarily bound lid), dunk coating, spray coating, puddle dispensing, or by another suitable method. Some examples utilize a primer solution or mixture, which may include the primers 30, 32, water, a buffer, and a catalyst. Other examples utilize an anchor oligonucleotide solution or mixture, which may include the anchor oligonucleotides 34, water, a buffer, and a catalyst. With any of the grafting methods, the primers 30, 32 or anchor oligonucleotides 34 attach to the reactive groups of the polymeric hydrogel 28, and have no affinity for the edge region 26.

Referring now to Fig. 1C, another example of the architecture within the flow channel 12 is depicted. In this example, the architecture includes a patterned structure. The patterned structure includes a plurality of depressions 20 defined in the substrate surface, which are isolated from each other by interstitial regions 22. Each of the plurality of depressions 20 has the polymeric hydrogel 28 applied therein.

In some examples, the substrate of the patterned structure is the multi-layered structure 16, and the depressions 20 are defined in the layer 18. While the substrate shown in Fig. 1C is the multi-layer structure 16, it is to be understood that the single layer base support 14 may be used (where the depressions 20 would be formed in the lane 24 defined in the single layer base support 14).

The depressions 20 may be formed using any suitable patterning techniques, such as photolithography, nanoimprint lithography (NIL), stamping techniques, embossing techniques, molding techniques, microetching techniques, etc.

Many different layouts of the depressions 20 and interstitial regions 22 may be envisaged, including regular, repeating, and non-regular patterns. In an example, the plurality of depressions 20 and interstitial regions 22 are disposed to create a hexagonal grid for close packing and improved density. Other layouts may include, for example, rectangular layouts, triangular layouts, and so forth. In some examples, the layout or pattern can be an x-y format in rows and columns. In some other examples, the layout or pattern can be a repeating arrangement of the depressions 20 and the interstitial regions 22. In still other examples, the layout or pattern can be a random arrangement of the depressions 20 and the interstitial regions 22.

The layout or pattern may be characterized with respect to the density (number) of the depressions 20 within a defined area. For example, the plurality of depressions 20 may be present at a density of approximately 2 million per mm². The density may be tuned to different densities including, for example, a density of about 100 per mm², about 1,000 per mm², about 0.1 million per mm², about 1 million per mm², about 2 million per mm², about 5 million per mm², about 10 million per mm², about 50 million per mm², or more, or less. It is to be further understood that the density can be between one of the lower values and one of the upper values selected from the ranges above, or that other densities (outside of the given ranges) may be used. As examples, a high density array may be characterized as having the plurality of depressions 20 separated by less than about 100 nm, a medium density array may be characterized as having the plurality of depressions 20 separated by about 400 nm to about 1 µm, and a low density array may be characterized as having the plurality of depressions 20 separated by greater than about 1 µm.

The layout or pattern of the plurality of depressions 20 may also or alternatively be characterized in terms of the average pitch, or the spacing from the center of one of the plurality of depression 20 to the center of an adjacent depression 20 (center-to-center spacing) or from the right edge of one of the plurality of depressions 20 to the left edge of an adjacent depression 20 (edge-to-edge spacing). The pattern can be regular, such that the coefficient of variation around the average pitch is small, or the pattern can be non-regular in which case the coefficient of variation can be relatively large. In either case, the average pitch can be, for example, about 50 nm, about 0.15 µm, about 0.5 µm, about 1 µm, about 5 µm, about 10 µm, about 100 µm, or more or less. The average pitch for a particular pattern of can be between one of the lower values and one of the upper values selected from the ranges above. In an example, the depressions 20 have a pitch (center-to-center spacing) of about 1.5 µm. While example average pitch values have been provided, it is to be understood that other average pitch values may be used.

The size of each depression 20 may be characterized by its volume, opening area, depth, and/or diameter (when the depression 20 is circular) and/or length and width. For example, the volume can range from about 1×10⁻³µm³ to about 100 µm³, e.g., about 1×10⁻² µm³, about 0.1 µm³, about 1 µm³, about 10 µm³, or more, or less. For another example, the opening area can range from about 1x10⁻³µm² to about 100 µm², e.g., about 1×10⁻² µm², about 0.1 µm², about 1 µm², at least about 10 µm², or more, or less. For still another example, the depth can range from about 0.1 µm to about 100 µm, e.g., about 0.5 µm, about 1 µm, about 10 µm, or more, or less. For another example, the depth can range from about 0.1 µm to about 100 µm, e.g., about 0.5 µm, about 1 µm, about 10 µm, or more, or less. For yet another example, the diameter or each of the length and width can range from about 0.1 µm to about 100 µm, e.g., about 0.5 µm, about 1 µm, about 10 µm, or more, or less.

The architectures in both Fig. 1B and Fig. 1C may include edge regions 26 that define interstitial like regions that extend the length of the flow channels 12 and separate one flow channel 12 from an adjacent flow channel 12 and/or define a perimeter of the flow cell 10. The edge regions 26 provide bonding regions where two non-patterned structures can be attached to one another or where one non-patterned structure can be attached to a lid.

The depressions 20 provide a designated area for the polymeric hydrogel 28. Any example of the polymeric hydrogel 28 may be used and may be formed via the techniques described herein in reference to Fig. 1B.

To introduce the polymeric hydrogel 28 into the depressions 20, a mixture of the polymeric hydrogel 28 may be generated and then applied to the multi-layered structure 16. In one example, the polymeric hydrogel 28 may be present in a mixture (e.g., with water or with ethanol and water). The mixture may then be applied to the substrate surface using spin coating, or dipping or dip coating, or flow of the material under positive or negative pressure, or another suitable technique. These types of techniques blanketly deposit the polymeric hydrogel 28 in the depressions 20 and on the interstitial regions 22. Other selective deposition techniques (e.g., involving a mask, controlled printing techniques, etc.) may be used to specifically deposit the polymeric hydrogel 28 in the depressions 20 and not on the interstitial regions 22.

Depending upon the chemistry of the polymeric hydrogel 28, the applied mixture may be exposed to a curing process. In an example, curing may take place at a temperature ranging from room temperature (e.g., about 25°C) to about 95°C for a time ranging from about 1 millisecond to about several days.

Polishing may then be performed in order to remove the polymeric hydrogel 28 from the interstitial regions 22, while leaving the polymeric hydrogel 28 on the surface in the depressions 20 at least substantially intact. The polishing process may be performed with a chemical slurry (including, e.g., an abrasive, a buffer, a chelating agent, a surfactant, and/or a dispersant) which can remove the polymeric hydrogel 28 from the interstitial regions 22 without deleteriously affecting the underlying substrate at those regions 22. Alternatively, polishing may be performed with a solution that does not include the abrasive particles.

The chemical slurry may be used in a chemical mechanical polishing system to polish the surface of the interstitial regions 22. The polishing head(s)/pad(s) or other polishing tool(s) is/are capable of polishing the polymeric hydrogel 28 that may be present over the interstitial regions 22 while leaving the polymeric hydrogel 28 in the depressions 20 at least substantially intact. As an example, the polishing head may be a Strasbaugh ViPRR II polishing head.

Cleaning and drying processes may be performed after polishing. The cleaning process may utilize a water bath and sonication. The water bath may be maintained at a relatively low temperature ranging from about 22°C to about 30°C. The drying process may involve spin drying, or drying via another suitable technique.

Prior to depositing and polishing the polymeric hydrogel 28, the single layer base support 14 or the multi-layer structure 16 may be activated as described herein.

The polymeric hydrogel 28 in the depressions 20 also includes primers 30, 32 or anchor oligonucleotides 34 attached thereto. The primers 30, 32 or anchor oligonucleotides 34 may be pre-grafted or may be grafted via any suitable technique as described in reference to Fig. 1B.

Different examples of the primers 30, 32 are described herein in reference to Fig. 2 through Fig. 8. Some of the primers 30, 32 are nuclease resistant primers (e.g., primers 42A, 42B or 42C, 42D) and some other of the primers may or may not be nuclease resistant (e.g., primers 68A, 68B).

Different examples of the anchor oligonucleotides 34 are described herein in reference to Fig. 9 through Fig. 11.

### Primer Set #1 and Method of Use

Fig. 2 illustrates an example of one of the primer sets 40A that may be used in the examples of the flow cell 10 disclosed herein.

The primer set 40A includes a first nuclease resistant primer 42A including a first sequence 44A, a first cleavage site 46A attached at a 3' end of the first sequence 44A, and a first nuclease resistant modification 48A incorporated between the first sequence 44A and the first cleavage site 46A; and a second nuclease resistant primer 42B including a second sequence 44B that is different from the first sequence 44A, a second nuclease resistant modification 48B incorporated at a 3' end of the second sequence 44B, and a second cleavage site 46B attached between the second sequence 44B and the second nuclease resistant modification 48B, wherein the second cleavage site 46B is different from the first cleavage site 46A.

The sequence 44A, 44B of the nuclease resistant primers 42A, 42B may include P5 and P7 primer sequences, P15 and P7 primer sequences, or any combination of the PA primer sequences, the PB primer sequences, the PC primer sequences, and the PD primer sequences set forth herein. As examples, the nuclease resistant primers 42A, 42B may include any combination of one PA primer sequence and one PB, PC, or PD primer sequence, or any combination of one PB primer sequence and one PC or PD primer sequence, or any combination of one PC primer sequence and one PD primer sequence.

Any two of these primer sequences may be used, respectively, as the main or primary sequence (i.e., sequence 44A, 44B) of the nuclease resistant primers 42A, 42B, and the respective cleavage sites 46A, 46B and nuclease resistant modifications 48A, 48B may be incorporated at desirable positions into the respective nucleic acid sequences 44A, 44B.

The cleavage sites 46A, 46B render the nuclease resistant primers 42A, 42B cleavable at the respective position at which the cleavage site 46A, 46B is incorporated. Examples of suitable cleavage sites 46A, 46B include enzymatically cleavable nucleobases or chemically cleavable nucleobases, modified nucleobases, or linkers (e.g., between nucleobases), as described herein. Some specific examples of the cleavage sites 46A, 46B include a uracil, an 8-oxoguanine, or an allyl-nucleotide (e.g., allyl-T, allyl-A, allyl-C, allyl-G). The cleavage sites 46A, 46B are different from one another, and thus are cleaved via different cleaving chemistries. This renders the cleavage of the nuclease resistant primers 42A, 42B orthogonal, meaning that one nuclease resistant primer 42A can be cleaved while the other nuclease resistant primers 42B remains uncleaved until a suitable cleaving agent is introduced.

The following examples illustrate some example sequences 44A, 44B, where P5, P7, and P15 illustrate example cleavage sites 46A, 46B.

The P5 primer sequence is:
P5: 5' → 3'
AATGATACGGCGACCACCGAGAUCTACAC (SEQ. ID. NO. 1)

The P7 primer sequence may be:
P7 #1: 5' → 3'
   CAAGCAGAAGACGGCATACGAnAT (SEQ. ID. NO. 2)
P7 #2: 5' → 3'
   CAAGCAGAAGACGGCATACnAGAT (SEQ. ID. NO. 3)
where n in both sequences is 8-oxoguanine. Thus, when the nucleotide in the 5^{th} position from the 3' end is guanine, n in the 3^{rd} position from the 3' end is 8-oxoguanine (SEQ. ID. NO. 2), and when the nucleotide in the 3^{rd} position from the 3' end is guanine, n in the 5^{th} position from the 3' end is 8-oxoguanine (SEQ. ID. NO. 3).

The P15 primer sequence is:
P15: 5' → 3'
AATGATACGGCGACCACCGAGAnCTACAC (SEQ. ID. NO. 4)
where n is allyl-T.

The other primer sequences (PA-PD) mentioned above include:
PA 5' → 3'
   GCTGGCACGTCCGAACGCTTCGTTAATCCGTTGAG (SEQ. ID. NO. 5)
cPA (PA') 5' → 3'
   CTCAACGGATTAACGAAGCGTTCGGACGTGCCAGC (SEQ. ID. NO. 6)
PB 5' → 3'
   CGTCGTCTGCCATGGCGCTTCGGTGGATATGAACT (SEQ. ID. NO. 7)
cPB (PB') 5' → 3'
   AGTTCATATCCACCGAAGCGCCATGGCAGACGACG (SEQ. ID. NO. 8)
PC 5' → 3'
   ACGGCCGCTAATATCAACGCGTCGAATCCGCAACT (SEQ. ID. NO. 9)
cPC (PC') 5' → 3'
   AGTTGCGGATTCGACGCGTTGATATTAGCGGCCGT (SEQ. ID. NO. 10)
PD 5' → 3'
   GCCGCGTTACGTTAGCCGGACTATTCGATGCAGC (SEQ. ID. NO. 11)
cPD (PD') 5' → 3'
   GCTGCATCGAATAGTCCGGCTAACGTAACGCGGC (SEQ. ID. NO. 12)

Any of the cleavage sites 46A, 46B disclosed herein may be incorporated into a suitable position in SEQ. ID. NO. 5 through SEQ. ID. NO. 12. In any of the examples disclosed herein, the position of the cleavage site 46A, 46B depends, at least in part, on whether the nuclease resistant primer 42A, 42B is to be used in an additional amplification and sequencing process, for regeneration, etc. In these instances, enough of the sequence 44A, 44B should remain, after cleavage, for hybridization, amplification, splint ligation, extension, and/or whatever other process is to be performed.

The nuclease resistant modifications 48A, 48B render the nuclease resistant primers 42A, 42B resistant to the 3'→5' enzymatic activity of an exonuclease and/or an endonuclease. Examples of suitable nuclease resistant modifications 48A, 48B include a phosphorothioate bond, methyl phosphonate, an inverted nucleotide, a peptide nucleic acid, a morpholino, 2'-O-methyl, and a nucleotide including a phosphoramidite C3 spacer. Some of these nuclease resistant modifications 48A, 48B, e.g., inverted nucleotide positioned at the 3' end of the nuclease resistant primers 42A, 42B, may also inhibit the extension activity of some polymerases. When these types of nuclease resistant modifications 48A, 48B are used, a polymerase that can read over such inverse nucleotides may be used in the method described in reference to Fig. 3A through Fig. 3E.

As mentioned, any of the primer sequences (P5, P7, PA, etc.) may be used as the main or primary sequence 44A of the nuclease resistant primer 42A. The cleavage site 46A is incorporated at the 3' end of the primer sequence 44A, and thus may be attached at the terminal end or near the terminal end. The specific location may depend upon the main or primary sequence 44A and the cleavage site 46A that are used; and, as noted above, the process(es) in which the remaining portion of the sequence 44A will be involved (e.g., another round of amplification and sequencing). As examples, uracil may be incorporated into the P5 sequence at the seventh position from the 3' terminal end of the primer sequence 44A (see SEQ. ID. NO. 1), or 8-oxoguanine may be incorporated into the P7 sequence at the third or fifth position from the 3' terminal end of the primer sequence 44A (see SEQ. ID. NOS. 2 and 3), or allyl-nucleotide may be incorporated into the P15 sequence at the 3' terminal end of the primer sequence 44A. In the nuclease resistant primer 42A, the nuclease resistant modification 48A is positioned 5' with respect to the cleavage site 46A. The position of the nuclease resistant modification 48A depends, at least in part, on whether the nuclease resistant primer 42A, 42B is to be used in an additional amplification and sequencing process, for regeneration, etc. In these instances, enough of the sequence 44A should remain, after nuclease digestion/cleavage, for hybridization, amplification, splint ligation, extension, etc. It is to be understood that a single nuclease resistant modification 48A or multiple nuclease resistant modifications 48A may be incorporated into the sequence 44A. As one example, three or more phosphorothioate bonds may be incorporated in a row in the primer sequence 44A beginning at a position that is 5' with respect to the position of the cleavage site 46A. This number of phosphorothioate bonds may increase the nuclease resistance of the nuclease resistant primer 42A.

Also as mentioned, any of the primer sequences (P5, P7, PA, etc.) may be used as the main or primary sequence 44B of the nuclease resistant primer 42B. The nuclease resistant modification 48B is incorporated at the 3' end of the primer sequence 44B, and thus may be attached at the terminal end or near the terminal end. The specific location may depend upon the nuclease resistant modification 48B that is used; and, as noted above, the process(es) in which the remaining portion of the sequence 44B will be involved (e.g., another round of amplification and sequencing). As one example, three phosphorothioate bonds may be incorporated in the first through third nucleotide positions beginning at the 3' terminal end of the primer sequence 44B. As another example, an inverted nucleotide may be incorporated at the second position from the 3' terminal end of the primer sequence 44B. In the nuclease resistant primer 42B, the cleavage site 46B is positioned 5' with respect to the nuclease resistant modification 48B. As examples, in P5, uracil may be incorporated at the seventh position from the 3' terminal end of the primer sequence 44B, or, in P7, 8-oxoguanine may be incorporated at the third or fifth position from the 3' terminal end of the primer sequence 44B as long as the nuclease resistant modification 48B is positioned 3' with respect to the cleavage site 46B.

In some examples of the primer set 40A, the first nuclease resistant modification 48A and the second nuclease resistant modification 48B are the same type of nuclease resistant modification. In contrast, the cleavage sites 46A, 46B are different. In an example, both the first and second nuclease resistant modifications 48A, 48B are selected from the group consisting of a phosphorothioate bond, methyl phosphonate, an inverted nucleotide, a peptide nucleic acid, a morpholino, 2'-O-methyl, and a nucleotide including a phosphoramidite C3 spacer; and each of the first and second cleavage sites 46A, 46B includes a nucleobase independently selected from the group consisting of a uracil, an 8-oxoguanine, and an allyl-nucleotide.

The 5' terminal end of each of the nuclease resistant primers 42A, 42B may also include a linker having a terminal alkyne group or another suitable terminal functional group that can attach to the surface functional groups of the polymeric hydrogel 28. In one example, the nuclease resistant primers 42A, 42B are 5' terminated with hexynyl. In addition to the desirable terminal functional group, the linker may include 10 nucleotides or less, a polyethylene glycol chain, an alkyl group or a carbon chain, an aliphatic linker with vicinal diols, a peptide linker, etc. An example of a linker is a polyT spacer, although other nucleotides can also be used. In one example, the spacer is a 6T to 10T spacer with a terminal alkyne group.

As mentioned, an example of the method involving the primer set 40A is shown schematically in Fig. 3A through Fig. 3E. While a single primer set 40A is shown in these figures, it is to be understood that the flow cell 10 will include several primer sets 40A and that all or some of the primer sets 40A may be utilized during the method.

The method shown in Fig. 3A through Fig. 3E enables single reads from two different samples. This example method includes introducing a first sample into an example of the flow cell 10 disclosed herein including the primer set 40A; amplifying a first library fragment of the first sample, thereby generating a first plurality of first sample library fragment amplicons 50 (Fig. 3A) respectively attached to a plurality of the first nuclease resistant primers 42A and a second plurality of first sample library fragment amplicons 52 respectively attached to a plurality of the second nuclease resistant primers 42B; introducing a first cleavage site cleaving fluid into the flow cell 10, thereby cleaving the first nuclease resistant primers 42A at the first cleavage sites 46A, whereby the first plurality of first sample library fragment amplicons 50 are removed (Fig. 3B); sequencing the second plurality of first sample library fragment amplicons 52 (Fig. 3B), thereby generating a plurality of first sample library fragment nascent strands (not shown); introducing a nuclease enzyme to the flow cell 10, thereby cleaving the second plurality of first sample library fragment amplicons 52 and the plurality of nascent strands (Fig. 3C); introducing a second sample into the flow cell 10; amplifying a second library fragment of the second sample, thereby generating a first plurality of second sample library fragment amplicons 54 respectively attached to a second plurality of the first nuclease resistant primers 42A and a second plurality of second sample library fragment amplicons 56 attached to a second plurality of the second nuclease resistant primers 42B (Fig. 3D); introducing a second cleavage site cleaving fluid into the flow cell 10, thereby cleaving the second nuclease resistant primers at the second cleavage sites 46B, whereby the second plurality of second sample library fragment amplicons 56 are removed; and sequencing the first plurality of second sample library fragment amplicons 54 (Fig. 3E), thereby generating a plurality of second sample library fragment nascent strands (not shown).

Prior to initiation of the method shown in Fig. 3A through Fig. 3E, respective library fragments may be prepared from the first and second nucleic acid samples (e.g., a DNA sample or an RNA sample). The DNA nucleic acid samples may be fragmented into single-stranded, similarly sized (e.g., < 1000 bp) DNA fragments. The RNA nucleic acid samples may be used to synthesize complementary DNA (cDNA), and the cDNA may be fragmented into single-stranded, similarly sized (e.g., < 1000 bp) cDNA fragments. During preparation, adapters may be added to the ends of any of the fragments. Through reduced cycle amplification, different motifs may be introduced in the adapters, such as sequencing primer binding sites, indices, and regions that are complementary to at least a portion of the nuclease resistant primers 42A, 42B. In some examples, the fragments from a single nucleic acid sample have the same adapters added thereto. The final library fragments include the DNA or cDNA fragment and adapters at both ends. The DNA or cDNA fragments from the respective samples represent the portions that are to be sequenced.

At the outset of the method shown in Fig. 3A through Fig. 3E, the first sample, including first sample library fragments, is introduced into the flow cell 10. Multiple first sample library templates are hybridized, for example, to one of two types of nuclease resistant primers 42A, 42B immobilized on the polymeric hydrogel 28.

Amplification, i.e., cluster generation, may then be performed. In one example of amplification, the library fragments are copied from the hybridized primers by 3' extension using a high-fidelity DNA polymerase (not shown). The original library fragments are denatured, leaving the copies (e.g., the first plurality of first sample library fragment amplicons 50) immobilized on the polymeric hydrogel 28. Isothermal bridge amplification or some other form of amplification may be used to amplify the immobilized copies. For example, the amplicons 50 loop over to hybridize to an adjacent, complementary nuclease resistant primer 42A, 42B and a polymerase copies the copied templates to form the second plurality of first sample library fragment amplicons 52. The first and second pluralities of first sample library fragment amplicons 50, 52 form double stranded bridges, as shown in Fig. 3A. While not shown, these double stranded bridges are denatured to form two single stranded first sample library fragment amplicons 50, 52. The single stranded first sample library fragment amplicons 50, 52 loop over and hybridize to adjacent, complementary primers 42A, 42B and are extended again to form two new double stranded loops (not shown). The process is repeated on each first library fragment amplicon 50, 52 by cycles of isothermal denaturation and amplification to create dense clonal clusters. Each cluster of double stranded bridges is denatured.

Amplification results in the formation of several amplicons 50, 52 immobilized on the polymeric hydrogel 28. This example of amplification/clustering is referred to as bridge amplification, and is one example of the amplification that may be performed. It is to be understood that other amplification techniques may be used, such as the exclusion amplification (Examp) workflow (Illumina Inc.).

The first cleavage site cleaving fluid is then introduced into the flow cell 10. This cleaving fluid is capable of removing the first plurality of first sample library fragment amplicons 50 without removing the second plurality of first sample library fragment amplicons 52. The active component of the cleaving fluid will depend upon the cleavage site 46A of the nuclease resistant primer 42A. As examples, when the first cleavage site 46A is uracil, the first cleavage site cleaving fluid may include the USER^{™} enzyme, which is a mixture of uracil DNA glycosylase (UDG) and the DNA glycosylase-lyase Endonuclease VIII (which leaves a 3' phosphate) or an enzyme isolated from Pyrococcus furiosus (which leaves a deprotected 3' end group); when the first cleavage site 46A is 8-oxoguanine, the first cleavage site cleaving fluid may include formamidopyrimidine [fapy]-DNA glycosylase (FPG); and when the first cleavage site 46A is an allyl-nucleotide, the first cleavage site cleaving fluid may include palladium and tris(hydroxypropyl)phosphine (THP). After cleavage, a portion 66A of the nuclease resistant primer 42A that is 5' of the cleavage site 46A remains attached to the polymeric hydrogel 28.

In an example, the first plurality of first sample library fragment amplicons 50 are reverse amplicons that are removed by specific base cleavage, leaving the second plurality of first sample library fragment amplicons 52, which are forward amplicons.

The second plurality of first sample library fragment amplicons 52 is then sequenced. Sequencing primers 58 are introduced into the flow cell 10 that hybridize, respectively, to a complementary portion of the sequence of the amplicons 52. During hybridization of the sequencing primer 58, the amplicons 50 may also rehybridize to the portion 66A of the primer 42A that remains, as shown in Fig. 3B.

The sequencing primers 58 render the amplicons 52 ready for sequencing using an incorporation mix. The incorporation mix may include a plurality of fully functional nucleotides (FFNs), a polymerase, and a liquid carrier. The liquid carrier of the incorporation mix may be water and/or an ionic salt buffer fluid, such as saline citrate at milli-molar to molar concentrations, sodium chloride, potassium chloride, phosphate buffered saline, etc., and other buffers, such as tris(hydroxymethyl)aminomethane (TRIS) or (4-(2-hydroxyethyl)-1-piperazineethanesulfonic acid) (HEPES). The liquid carrier may also include catalytic metal(s) intended for the incorporation reaction, such as Mg²⁺, Mn²⁺, Ca²⁺, etc. A single catalytic metal or a combination of catalytic metals may be used, and the total amount may range from about 0.01 mM to about 100 mM.

The fully functional nucleotide includes the nucleotide, a 3' OH blocking group attached to the sugar of the nucleotide, and a fluorophore attached to the base of the nucleotide.

The nucleotide may be any nucleotide described herein.

The 3' OH blocking group may be linked to the 3' oxygen atom of the sugar molecule in the nucleotide. The 3' OH blocking group may be a reversible terminator that allows only a single-base incorporation to occur in each sequencing cycle. The reversible terminator stops additional bases from being incorporated into a nascent strand that is complementary to the amplicon 52. This enables the detection and identification of a single incorporated base. The 3' OH blocking group can subsequently be removed, enabling additional sequencing cycles to take place at each amplicon 52. Examples of different 3' OH blocking groups include a 3'-ONH₂ reversible terminator, a 3'-O-allyl reversible terminator (i.e., -CH=CHCH₂), and 3'-*O-*azidomethyl reversible terminator (i.e., -CH₂N₃). Other suitable reversible terminators include o-nitrobenzyl ethers, alkyl o-nitrobenzyl carbonate, ester moieties, other allyl-moieties, acetals (e.g., tert-butoxy-ethoxy), MOM (-CH₂OCH₃) moieties, 2,4-dinitrobenzene sulfenyl, tetrahydrofuranyl ether, 3' phosphate, ethers, -F, -H₂ , - OCH₃, -N₃, -HCOCH₃, and 2-nitrobenzene carbonate.

The fluorophore of the fully functional nucleotide is attached to the base of the nucleotide. The fluorophore may be any optically detectable moiety, including luminescent, chemiluminescent, fluorescent, fluorogenic, chromophoric and/or chromogenic moieties. Some examples of suitable optically detectable moieties include fluorescein labels, rhodamine labels, cyanine labels (e.g., Cy3, Cy5, and the like), and the ALEXA^{®} family of fluorescent dyes and other fluorescent and fluorogenic dyes. The fluorophore may be attached to the base of the nucleotide using any suitable linker molecule. In an example, the linker molecule is a spacer group of formula -((CH₂)₂O)ₙ- wherein n is an integer between 2 and 50. The linker molecule includes a cleavage site.

In one example, the incorporation mix includes a mixture of different FFNs, which include different bases, e.g., A, T, G, C (as well as U or I). It may also be desirable to utilize a different type of fluorophore for the different FFNs. For example, the fluorophores may be selected so that each fluorophore absorbs excitation radiation and/or emits fluorescence, at a distinguishable wavelength from the other fluorophores. Such distinguishable analogs provide an ability to monitor the presence of different fluorophores simultaneously in the same reaction mixture. In some examples, one of the four FFNs in the incorporation mix may include no fluorophore, while the other three labeled FFNs include different fluorophores.

Any polymerase that can accept the fully functional nucleotide, and that can successfully incorporate the base of the fully functional nucleotide into a nascent strand along the amplicon 52 may be used. In some instances, the polymerase may also be selected to read over the nuclease resistant modifications 48A, 48B. For example, lesion bypass polymerases, such as human polymerase δ, may be selected to read over some nuclease resistant modifications 48A, 48B, including morpholino and inverted nucleotides. Other example polymerases include those polymerases from family A, such as Bsu Polymerase, Bst Polymerase, Taq Polymerase, T7 Polymerase, and many others; polymerases from families B and B2, such as Phi29 polymerase and other highly processive polymerases (family B2), Pfu Polymerase (family B), KOD Polymerase (family B), 9oN (family B), and many others; polymerases from family C, such as Escherichia coli DNA Pol III, and many others, polymerases from family D, such as Pyrococcus furiosus DNA Pol II, and many others; polymerases from family X, such as DNA Pol µ, DNA Pol β, DNA Pol σ, and many others.

In this example method, any example of the incorporation mix is introduced into the flow cell 10, e.g., via the inlet. When the incorporation mix is introduced into the flow cell 10, the mix enters the flow channel 12, and contacts the surface chemistry where the amplicons 52 are present. One or more of these polymerases may be used to copy over phosphorothioate modifications.

The incorporation mix is allowed to incubate in the flow cell 10, and FFNs are incorporated by a polymerase into a nascent strand (not shown) generated along the amplicon 52. During incorporation, one of FFNs is incorporated, by a respective polymerase, into one nascent strand that extends one sequencing primer 58 and that is complementary to one of the amplicons 52. Incorporation is performed in a template strand dependent fashion, and thus detection of the order and type of FFNs added to the nascent strand can be used to determine the sequence of the amplicon 52. Incorporation occurs in at least some of the amplicons 52 across the lane 24 or depressions 20 during a single sequencing cycle. As such, in at least some of the amplicons 52 across the flow cell 10, respective polymerases extend the hybridized sequencing primer 58 by one of the FFNs in the incorporation mix.

The incorporated FFNs include the reversible termination property due to the presence of the 3' OH blocking group, which terminates further sequencing primer extension on the nascent strand once the FFN has been added. After a desired time for incubation and incorporation, the incorporation mix may be removed from the flow cell 10 during a wash cycle. The wash cycle may involve a flow-through technique, where a washing solution (e.g., buffer) is directed into, through, and then out of flow channel 12, e.g., by a pump or other suitable mechanism.

Without further incorporation taking place, the most recently incorporated FFNs can be detected through an imaging event. During the imaging event, an illumination system (not shown) may provide an excitation light to the flow cell surfaces containing the surface chemistry. The fluorophore of the incorporated FFNs emit optical signals in response to the excitation light.

After imaging is performed, a mix is introduced into the flow cell 10 that is capable of i) removing the 3' OH blocking group from the incorporated FFNs, and ii) cleaving the fluorophore from the FFNs. Examples of 3' OH blocking groups and suitable de-blocking agents/components in the mix may include: ester moieties that can be removed by base hydrolysis; allyl-moieties that can be removed with Nal, chlorotrimethylsilane and Na₂S₂O₃ or with Hg(II) in acetone/water; azidomethyl which can be cleaved with phosphines, such as tris(2-carboxyethyl)phosphine (TCEP) or tri(hydroxypropyl)phosphine (THP); acetals, such as tert-butoxy-ethoxy which can be cleaved with acidic conditions; MOM (-CH₂OCH₃) moieties that can be cleaved with LiBF₄ and CH₃CN/H₂O; 2,4-dinitrobenzene sulfenyl which can be cleaved with nucleophiles such as thiophenol and thiosulfate; tetrahydrofuranyl ether which can be cleaved with Ag(I) or Hg(II); and 3' phosphate which can be cleaved by phosphatase enzymes (e.g., polynucleotide kinase). Examples of suitable fluorophore cleaving agents/components in the cleavage mix may include: sodium periodate, which can cleave a vicinal diol; phosphines, such as tris(2-carboxyethyl)phosphine (TCEP) or tri(hydroxypropyl)phosphine (THP), which can cleave azidomethyl linkages; palladium and THP, which can cleave an allyl; bases, which can cleave ester moieties; or any other suitable cleaving agent.

Wash(es) may take place between the various fluid delivery steps. The sequencing cycle can then be repeated n times to extend the sequencing primer 58 by n nucleotides, thereby detecting a sequence of length n.

After the amplicons 52 are sequenced, a nuclease enzyme is introduced into the flow cell 10 to cleave the second plurality of first sample library fragment amplicons 52 and the plurality of nascent strands (Fig. 3C). The nuclease enzyme may be an exonuclease or an endonuclease. The nuclease enzyme has 3'→5' activity. In an example, the nuclease enzyme is selected from the group consisting of Exonuclease I, Thermolabile Exonuclease I, Exonuclease T, Exonuclease VII, Mung Bean Nuclease, Nuclease P1, Exonuclease III, Exonuclease V, Nuclease BAL-31, DNase I, and Micrococcal Nuclease. As shown in Fig. 3C, the nuclease enzyme removes the first sample strands (e.g., amplicons 52 and nascent strands attached thereto), but does not remove the nuclease resistant primer 42B or the remaining portion of the nuclease resistant primer 42A. This is due to the fact that the nuclease resistant modifications 48A, 48B are not susceptible to, and thus protect against nuclease digestion/cleavage.

After the first sample strands are removed, an enzyme with 3'-phosphatase activity (e.g., DNA phosphatase, T4 phage polynucleotide kinase (PNK), etc.) may be introduced into the flow cell 10. This is desirable when the 3' end is a phosphate. This type of enzyme processes the 3' phosphate ends, converting them into 3'-hydroxyls. The remaining portions of the nuclease resistant primers 42A, 42B are ready for the second sample.

The second sample, including second sample library fragments, is introduced into the flow cell 10. Amplification of the second library fragments may be performed as described herein, which generates the first plurality of second sample library fragment amplicons 54 respectively attached to a second plurality of the first nuclease resistant primers 42A and a second plurality of second sample library fragment amplicons 56 respectively attached to a second plurality of the second nuclease resistant primers 42B. This is depicted in Fig. 3D.

The second cleavage site cleaving fluid is then introduced into the flow cell 10. This cleaving fluid is capable of removing the second plurality of second sample library fragment amplicons 56 without removing the first plurality of second sample library fragment amplicons 54. The active component of the cleaving fluid will depend upon the cleavage site 46B of the nuclease resistant primer 42B. As examples, when the second cleavage site 46B is uracil, the second cleavage site cleaving fluid may include the USER^{™} enzyme, which is a mixture of uracil DNA glycosylase (UDG) and the DNA glycosylase-lyase Endonuclease VIII (which leaves a 3' phosphate) or an enzyme isolated from Pyrococcus furiosus (which leaves a deprotected 3' end group); when the second cleavage site 46B is 8-oxoguanine, the second cleavage site cleaving fluid may include formamidopyrimidine [fapy]-DNA glycosylase (FPG); and when the second cleavage site 46B is an allyl-nucleotide, the second cleavage site cleaving fluid may include palladium and tris(hydroxypropyl)phosphine (THP).

Another sequencing primer 58' is introduced into the flow cell 10, and the first plurality of second sample library fragment amplicons 54 are sequenced as described herein.

In one specific example of the method shown in Fig. 3A through Fig. 3E, each of the first cleavage sites 46A includes a uracil nucleobase; the first cleavage site cleaving fluid includes a mixture of uracil DNA glycosylase and the DNA glycosylase-lyase Endonuclease VIII or an enzyme isolated from Pyrococcus furiosus; each of the second cleavage sites 46B includes an 8-oxoguanine nucleobase; the second cleavage site cleaving fluid includes formamidopyrimidine [fapy]-DNA glycosylase; and the nuclease enzyme is selected from the group consisting of Exonuclease I, Thermolabile Exonuclease I, Exonuclease T, Exonuclease VII, Mung Bean Nuclease, Nuclease P1, Exonuclease III, Exonuclease V, Nuclease BAL-31, DNase I, and Micrococcal Nuclease.

### Primer Set #2 and Method of Use

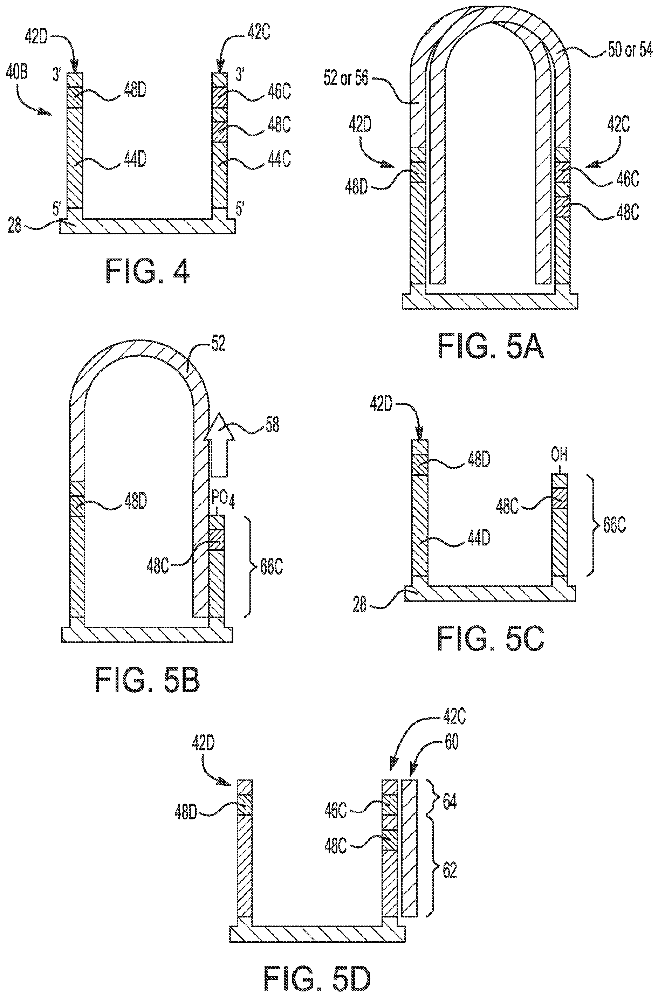

Fig. 4 illustrates an example of another primer set 40B that may be used in the examples of the flow cell 10 disclosed herein. The primer set 40B includes two nuclease resistant primers 42C, 42D, one of which includes a cleavage site 46C and other of which does not include a cleavage site. The primer set 40B includes a first nuclease resistant primer 42C including a first sequence 44C, a cleavage site 46C attached at a 3' end of the first sequence 44C, and a first nuclease resistant modification 48C incorporated between the first sequence 44C and the cleavage site 46C; and a second nuclease resistant primer 42D including a second sequence 44D that is different from the first sequence 44C, and a second nuclease resistant modification 48B incorporated at a 3' end of the second sequence 44D.

The sequence 44C, 44D of the nuclease resistant primers 42C, 42D may include any of the sequences set forth herein, with the caveat that the cleavage site 46C is not included in P5, P7, or P15 when used as the primer 42D. Any two of these primer sequences may be used, respectively, as the main or primary sequence (i.e., sequence 44C, 44D) of the nuclease resistant primers 42C, 42D. The cleavage site 46C and nuclease resistant modification 48C may be incorporated at desirable positions into the nucleic acid sequence 44C, while the nuclease resistant modification 48D may be incorporated at a desirable position into the nucleic acid sequence 44D.

The cleavage site 46C renders the nuclease resistant primer 42C cleavable at the position at which the cleavage site 46C is incorporated. Any of the examples set forth herein for cleavage sites 46A, 46B may be used for the cleavage site 46C except for the allyl-nucleotide because polymerase extension is used to generate the nuclease resistant primer 42C. The nuclease resistant primer 42D does not include a cleavage site.

The nuclease resistant modifications 48C, 48D render the nuclease resistant primers 42C, 42D resistant to the 3'→5' enzymatic activity of an exonuclease and/or an endonuclease. Any of the examples of nuclease resistant modifications 48A, 48B may be used for the nuclease resistant modifications 48C, 48D.

As mentioned, any of the primer sequences (P5, P7, PA, etc.) may be used as the main or primary sequence 44C of the nuclease resistant primer 42C. The cleavage site 46C is incorporated at the 3' end of the primer sequence 44C, and thus may be attached at the terminal end or near the terminal end. The specific location may depend upon the cleavage site 46C that is used and the process(es) in which the remaining portion of the sequence 44C will be involved (e.g., regeneration), e.g., as described herein in reference to the cleavage site 46A. In the nuclease resistant primer 42C, the nuclease resistant modification 48C is positioned 5' with respect to the cleavage site 46C. It is to be understood that a single nuclease resistant modification 48C or multiple nuclease resistant modifications 48C may be incorporated into the sequence 44C. As one example, three or more phosphorothioate bonds may be incorporated in a row in the primer sequence 44C beginning at a position that is 5' with respect to the position of the cleavage site 46C.

Also as mentioned, any of the primer sequences (P5, P7, PA, etc.) may be used as the main or primary sequence 44D of the nuclease resistant primer 42D. The nuclease resistant modification 48D is incorporated at the 3' end of the primer sequence 44D, and thus may be attached at the terminal end or near the terminal end. The specific location may depend upon the nuclease resistant modification 48D that is used and the process(es) in which the remaining portion of the sequence 44D will be involved (e.g., another round of amplification and sequencing). As one example, three phosphorothioate bonds may be incorporated in the first through third nucleotide positions beginning at the 3' terminal end of the primer sequence 44D. As another example, an inverted nucleotide may be incorporated at the second position from the 3' terminal end of the primer sequence 44D.

In some examples of the primer set 40B, the first nuclease resistant modification 48C and the second nuclease resistant modification 48D are the same type of nuclease resistant modification. In an example, the first nuclease resistant modification 48C and the second nuclease resistant modification 48D are a same type of nuclease resistant modification and are selected from the group consisting of a phosphorothioate bond, methyl phosphonate, an inverted nucleotide, a peptide nucleic acid, a morpholino, 2'-O-methyl, and a nucleotide including a phosphoramidite C3 spacer; and the cleavage site 46C of the first nuclease resistant primer 42C includes a nucleobase independently selected from the group consisting of a uracil, an 8-oxoguanine, and an allyl-nucleotide.

The 5' terminal end of each of the nuclease resistant primers 42C, 42D may also include a linker (e.g., a polyT spacer) having a terminal alkyne group or another suitable terminal functional group that can attach to the surface functional groups of the polymeric hydrogel 28.

An example of the method involving the primer set 40B is shown schematically in Fig. 5A through Fig. 5D. While a single primer set 40B is shown in these figures, it is to be understood that the flow cell 10 will include several primer sets 40B and that all or some of the primer sets 40B may be utilized during the method.

The method shown in Fig. 5A through Fig. 5D enables single reads from any number of samples. This example method includes introducing a first sample into an example of the flow cell 10 disclosed herein including the primer set 40B; amplifying a library fragment of the first sample, thereby generating a first plurality of first sample library fragment amplicons 50 (Fig. 5A) respectively attached to a plurality of the first nuclease resistant primers 42C and a second plurality of first sample library fragment amplicons 52 respectively attached to a plurality of the second nuclease resistant primers 42D; introducing a cleaving fluid into the flow cell 10, thereby cleaving the first nuclease resistant primers 42C at the cleavage sites 46C, whereby the first plurality of first sample library fragment amplicons 50 are removed (Fig. 5B); sequencing the second plurality of first sample library fragment amplicons 52 (Fig. 5B), thereby generating a plurality of first sample library fragment nascent strands (not shown); introducing a nuclease enzyme to the flow cell 10, thereby cleaving the second plurality of first sample library fragment amplicons 52 and the plurality of first sample library fragment nascent strands (Fig. 5C); introducing a plurality of regeneration oligonucleotides 60 into the flow cell 10 (Fig. 5D), whereby first portions 62 of at least some of the regeneration oligonucleotides 60 respectively hybridize to remaining portions 66C of at least some of the first nuclease resistant primers 42C, wherein each regeneration oligonucleotide 60 further includes a second portion 64 that is complementary to a cleaved portion of the first nuclease resistant primer 42C; and initiating polymerase extension along respective second portions 64 of the hybridized regeneration oligonucleotides 60 to regenerate the cleaved portion of the at least some of the first nuclease resistant primers 42C.

The library fragments used in the method shown in Fig. 5A through Fig. 5D may be prepared used any suitable technique.

At the outset of the method shown in Fig. 5A through Fig. 5D, the first sample, including first sample library fragments, is introduced into the flow cell 10. Multiple first sample library templates are hybridized, for example, to one of two types of nuclease resistant primers 42C, 42D immobilized on the polymeric hydrogel 28.

Amplification, i.e., cluster generation, may then be performed as described herein. Amplification results in the formation of several amplicons 50, 52 immobilized on the polymeric hydrogel 28.

A cleaving fluid is then introduced into the flow cell 10. This cleaving fluid is capable of removing the first plurality of first library fragment amplicons 50 without removing the second plurality of first library fragment amplicons 52 because the primers 42D do not include a cleavage site. The active component of the cleaving fluid will depend upon the cleavage site 46C of the nuclease resistant primer 42C. Any examples of the cleaving fluid disclosed herein may be used. After cleavage, a portion 66C of the nuclease resistant primer 42C that is 5' of the cleavage site 46C remains attached to the polymeric hydrogel 28.

The sequencing primers 58 are introduced and the second plurality of first library fragment amplicons 52 is sequenced as described herein.

After the amplicons 52 are sequenced, a nuclease enzyme is introduced into the flow cell 10 to cleave the second plurality of first library fragment amplicons 52 and the plurality of nascent strands attached thereto (Fig. 5C). The nuclease enzyme may be an exonuclease or an endonuclease. The nuclease enzyme has 3'→5' activity. In an example, the nuclease enzyme is selected from the group consisting of Exonuclease I, Thermolabile Exonuclease I, Exonuclease T, Exonuclease VII, Mung Bean Nuclease, Nuclease P1, Exonuclease III, Exonuclease V, Nuclease BAL-31, DNase I, and Micrococcal Nuclease. As shown in Fig. 5C, the nuclease enzyme removes the first sample strands (e.g., amplicons 52 and nascent strands attached thereto), but does not remove the nuclease resistant primers 42D or the remaining portion 66C of the nuclease resistant primers 42C. This is due to the fact that the nuclease resistant modifications 48C, 48D are not susceptible to, and thus protect against nuclease digestion/cleavage.

After the first sample strands are removed and when the 3' ends are phosphates, an enzyme with 3'-phosphatase activity (e.g., DNA phosphatase, T4 phage polynucleotide kinase (PNK), etc.) may be introduced into the flow cell 10. This enzyme processes the phosphate ends, converting them into 3'-hydroxyls.

In this example method, the remaining portion 66C of the nuclease resistant primer 42C is exposed to processing that reintroduces the cleavage site 46C and thus regenerates the nuclease resistant primer 42C. To regenerate the nuclease resistant primer 42C, regeneration oligonucleotides 60 are introduced into the flow cell 10. Each regeneration oligonucleotide 60 includes a first portion 62 that is complementary to the remaining portion 66C of the nuclease resistant primer 42C and a second portion 64 that is complementary to the portion of the nuclease resistant primer 42C that was cleaved by the cleaving fluid. Thus, the sequence of the regeneration oligonucleotide 60 depends upon the sequence of the nuclease resistant primer 42C.

The regeneration oligonucleotides 60 are introduced at conditions that enable the first portions 62 of the regeneration oligonucleotides 60 to respectively hybridize to the remaining portions 66C of at least some of the first nuclease resistant primers 42C.

A mixture containing the cleavage site 46C, nucleotides, and a polymerase are then introduced into the flow cell 10. The temperature of the flow cell 10 may be adjusted to initiate polymerase chain reaction (PCR). The polymerase enables the PCR extension of the 3' end of the remaining portion 66C using the second portion 64 of the regeneration oligonucleotide 60 as a template. Because the PCR extension reaction is guided by the second portion 64 of the regeneration oligonucleotides 60 and the second portion 64 is complementary to the portion of the nuclease resistant primer 42C that was cleaved by the cleaving fluid, the polymerase extension along the second portion 64 regenerates at least the cleavage site 46C of the first nuclease resistant primer 42C. In an example, the PCR extension incorporates the cleavage site 46C and the nucleotide(s) from the mixture to regenerate the first nuclease resistant primer 42C.

Once the first nuclease resistant primers 42C are regenerated, the regeneration oligonucleotides 60 are denatured, and the flow cell 10 surface can be exposed to another sample for amplification and sequencing. Thus, the method shown in Fig. 5A through Fig. 5D may be repeated with a new sample. One example of the method involves: introducing a second sample into the flow cell 10 after the polymerase extension (moving from Fig. 5D back to Fig. 5A); amplifying a library fragment of the second sample, thereby generating a first plurality of second sample library fragment amplicons (e.g., amplicons 54) respectively attached to a second plurality of the first nuclease resistant primers 42C and a second plurality of second sample library fragment amplicons (e.g., amplicons 56) attached to a second plurality of the second nuclease resistant primers 42D; introducing the cleaving fluid into the flow cell 10, thereby cleaving the (regenerated) first nuclease resistant primers 42C at the (regenerated) cleavage sites 46C, whereby the first plurality of second sample library fragment amplicons 54 are removed; and sequencing the second plurality of second sample library fragment amplicons 56, thereby generating a plurality of second sample library fragment nascent strands (not shown).

After the amplicons 56 are sequenced, a nuclease enzyme is introduced into the flow cell 10 to cleave the second plurality of second library fragment amplicons 56 and the plurality of nascent strands attached thereto (Fig. 5C). After the second sample strands are removed, an enzyme with 3'-phosphatase activity (e.g., T4 phage polynucleotide kinase (PNK)) may be introduced into the flow cell 10 to generate 3'-hydroxyls. The remaining portion 66C of the nuclease resistant primer 42C is then exposed to processing that again reintroduces the cleavage site 46C and regenerates the nuclease resistant primer 42C. The method shown in Fig. 5A through Fig. 5D may be repeated for any desirable number of samples.

In one specific example of the method shown in Fig. 5A through Fig. 5D, the first cleavage site 46C includes a uracil nucleobase; the cleaving fluid includes a mixture of uracil DNA glycosylase and the DNA glycosylase-lyase Endonuclease VIII or an enzyme isolated from Pyrococcus furiosus; and the nuclease enzyme is selected from the group consisting of Exonuclease I, Thermolabile Exonuclease I, Exonuclease T, Exonuclease VII, Mung Bean Nuclease, Nuclease P1, Exonuclease III, Exonuclease V, Nuclease BAL-31, DNase I, and Micrococcal Nuclease.

### Primer Set #3 and Methods of Use

Fig. 6 illustrates an example of another primer set 40C that may be used in the examples of the flow cell 10 disclosed herein. The primer set 40C includes two primers 68A, 68B, one of which includes different first and second cleavage sites 46D, 46E, and the other of which includes the first cleavage site 46D without the second cleavage site 46E. The cleavage site 46D is the same in each of the primers 68A, 68B.

The primer 68A includes a first sequence 44E, a cleavage site 46E attached at a 3' end of the first sequence 44E, and another cleavage site 46D incorporated between the first sequence 44E and the cleavage site 46E. The first sequence 44E may be any of the primer sequences set forth herein (e.g., P5, P7, P15, etc.), and at least a portion 70A of the first sequence 44E is configured to hybridize to a regeneration oligonucleotide 60' (see Fig. 7E) or a first regeneration splint 84 (see Fig. 8) during examples of the method. This primer 68A may be made nuclease resistant by incorporating any example of the nuclease resistant modifications at the terminal position of the 3' end of the primer 68A or at the 3' end of the portion 70A. At the terminal position of the 3' end of the primer 68A, the nuclease resistant modification may enable digestion of the amplified and sequenced strands, while leaving the remainder of the primer 68A intact for regeneration. At the 3' end of the portion 70A, the nuclease resistant modification may enable endonuclease resistance for all of the nucleotides of the portion 70A.

The primer 68B includes a second sequence 44F that is different than the first sequence 44E and the cleavage site 46D attached at a 3' end of the second sequence 44F. The second sequence 44F may be any of the primer sequences set forth herein (e.g., P5, P7, P15, etc.) that is different from the first sequence 44E, and at least a portion 70B of the second sequence 44F is configured to hybridize to a regeneration oligonucleotide 60" (see Fig. 7F) or a second regeneration splint 84' (see Fig. 8) during examples of the method. This primer 68B may be made nuclease resistant by incorporating any example of the nuclease resistant modification at the terminal position of the 3' end of the primer 68B or at the 3' end of the portion 70B. At the terminal position of the 3' end of the primer 68B, the nuclease resistant modification may enable digestion of the amplified and sequenced strands, while leaving the remainder of the primer 68B intact for regeneration. At the 3' end of the portion 70B, the nuclease resistant modification may enable endonuclease resistance for all of the nucleotides of the portion 70B.

In one example of the primer set 40C, the first cleavage site 46D (in each of the primers 68A, 68B) is a restriction site; and the second cleavage site 46E (in the primer 68A) includes a nucleobase selected from the group consisting of a uracil, an 8-oxoguanine, and an allyl-nucleotide. Some examples of the restriction site are sensitive to a restriction enzyme selected from the group consisting of a ≥ 6 base cutter restriction endonuclease, a nicking enzyme, or the like. Some example 6 base cutters include Acll, Afel, Nspl, Haell, Tatl, and others that are commercially available, for example, from New England BioLabs Inc., ThermoFisher Scientific, etc. Some example 7 base cutters include BspQI and Sapl, and some example 8 base cutters include Notl, Fsel, and Pacl.

In another example of the primer set 40C, the first cleavage site 46D (in each of the primers 68A, 68B) and the second cleavage site 46E (in the primer 68A) each include a nucleobase independently selected from the group consisting of a uracil, an 8-oxoguanine, and an allyl-nucleotide. When the primers 68A, 68B are to be regenerated by polymerase extension (Fig. 7E), the cleavage sites 46D, 46E are not allyl-nucleotides. When the primers 68A, 68B are to be regenerated by splint ligation (Fig. 8), the cleavage sites 46D, 46E may be allyl-nucleotides.

Whether the first cleavage site 46D is a restriction site or a cleavage site that is orthogonal to the second cleavage site 46E, in the primer 68A, the first cleavage site 46D is positioned 5' relative to the second cleavage site 46E, and the portion 70A is positioned 5' relative to the first cleavage site 46D. In the primer 68B, the portion 70A is positioned 5' relative to the first cleavage site 46D. With this primer set 40C, the second cleavage site 46E is utilized for linearization after amplification and the first cleavage sites 46D are for the removal of amplicons 54 and nascent strands attached thereto after sequencing.

The 5' terminal end of each of the primers 68A, 68B may also include a linker (e.g., a polyT spacer) having a terminal alkyne group or another suitable terminal functional group that can attach to the surface functional groups of the polymeric hydrogel 28.

Two examples of the method involving the primer set 40C are shown schematically in Fig. 7A through Fig. 7F. Another example of the method involving the primer set 40C is shown schematically in Fig. 7A through Fig. 7D and Fig. 8. While a single primer set 40C is shown in these figures, it is to be understood that the flow cell 10 will include several primer sets 40C and that all or some of the primer sets 40C may be utilized during the method.

The methods shown in Fig. 7A through Fig. 7F and in Fig. 7A through Fig. 7D and Fig. 8 enable single reads from any number of samples. These example methods include introducing a first sample into an example of the flow cell 10 disclosed herein including the primer set 40C; amplifying a first library fragment of the first sample, thereby generating a first plurality of first sample library fragment amplicons 50 respectively attached to a plurality of the first primers 68A and a second plurality of first sample library fragment amplicons 52 respectively attached to a plurality of the second primers 68B (Fig. 7A); introducing a cleaving fluid into the flow cell 10, thereby cleaving the first primers 68A at the second cleavage sites 46E, whereby the first plurality of first sample library fragment amplicons 50 are removed (Fig. 7B); sequencing the second plurality of first sample library fragment amplicons 52 (Fig. 7B), thereby generating a plurality of first sample library fragment nascent strands 74 (Fig. 7C); introducing a second cleaving fluid into the flow cell 10, thereby cleaving each of the first primers 68A and the second primers 68B at their respective first cleavage sites 46D and leaving remaining portions 66D, 66E of the first primers 68A and of the second primers 68B attached to the polymeric hydrogel 28 (Fig. 7C and Fig. 7D); and sequentially (Fig. 7E and Fig. 7F) or simultaneously (Fig. 8) regenerating i) a cleaved portion 72A (see Fig. 7A) of at least some of the first primers 68A at the remaining portions 66D of the at least some of the first primers 68A, and ii) a cleaved portion 72B (see Fig. 7A) of at least some of the second primers 68B at the remaining portions 78B of the at least some of the second primers 68B. The methods shown in Fig. 7A through Fig. 7F depict sequential regeneration of the primer set 40C, and the method shown in Fig. 7A through Fig. 7D and Fig. 8 depict simultaneous regeneration of the primer set 40C.

The methods of Fig. 7A through Fig. 7F will now be described.

In one example method, each of the first cleavage sites 46D, 46E is a restriction site.

In another example method, each of the first cleavage sites 46D, 46E is a nucleobase independently selected from the group consisting of a uracil, an 8-oxoguanine, and an allyl-thymine (where the second cleavage site 46E is different from the first cleavage sites 46D, 46E).

Each of these examples is described in reference to Fig. 7A through Fig. 7F.

The library fragments used in the methods shown in Fig. 7A through Fig. 7F may be prepared used any suitable technique.

At the outset of the methods shown in Fig. 7A through Fig. 7F, the first sample, including first sample library fragments, is introduced into the flow cell 10. Multiple first sample library templates are hybridized, for example, to one of two types of primers 68A, 68B immobilized on the polymeric hydrogel 28.

In both examples of the method shown in Fig. 7A through Fig. 7F, amplification, i.e., cluster generation, may then be performed as described herein. Amplification results in the formation of several amplicons 50, 52 immobilized on the polymeric hydrogel 28.

In both examples of the method shown in Fig. 7A through Fig. 7F, a cleaving fluid is then introduced into the flow cell 10. The active component of the cleaving fluid will depend upon the second cleavage site 46E of the primer 68A. This cleaving fluid is capable of removing the first plurality of first library fragment amplicons 50 without removing the second plurality of first library fragment amplicons 52 because the primers 68B do not include the cleavage site 46E which is present in the primer 68A. Because the cleavage sites 46D of both primers 68A, 68B are chemically orthogonal to the second cleavage site 46E of the primer 68A, the cleavage sites 46D are not susceptible to or affected by the cleaving fluid introduced into the flow cell 10 at this point in the methods. Any examples of the cleaving fluid disclosed herein may be used. After cleavage, a portion of the primer 68A that is 5' of the second cleavage site 46E remains attached to the polymeric hydrogel 28, as shown in Fig. 7B.

The sequencing primers 58 are introduced and the second plurality of first library fragment amplicons 52 is sequenced as described herein. Fig. 7C illustrates an example of the nascent strand 74 that is generated along the amplicon 52 during sequencing. As depicted, the primer 68A may remain single stranded after sequencing is complete.

After at least some of the amplicons 52 are sequenced, a second cleaving fluid is introduced into the flow cell 10 to cleave each of the first primers 68A and the second primers 68B at their respective first cleavage sites 46D, which leaves remaining portions 66D, 66E of the first primers 68A and of the second primers 68B attached to the polymeric hydrogel 28. This second cleaving fluid also cleaves the second plurality of first library fragment amplicons 52 and the plurality of nascent strands 74 attached thereto. The flow cell surface after exposure to the second cleaving fluid is shown in Fig. 7D. The components of the second cleaving fluid will depend upon the first cleavage site 46D of the primers 68A, 68B.

Fig. 7C schematically illustrates the flow cell surface during exposure to the second cleaving fluid.

In one example shown in Fig. 7C, each of the first cleavage sites 46D is a restriction site, and the second cleaving fluid includes a plurality of first oligonucleotides 78A, each first oligonucleotide 78A being complementary to at least a portion of the first primer 68A; a plurality of second oligonucleotides 78B, each second oligonucleotide 78B being complementary to at least a portion of the second primer 68B; and a restriction enzyme (not shown).

In this example, the restriction enzyme in the second cleaving fluid will depend upon the restriction site used as the cleavage sites 46D. As noted herein, the restriction enzyme may be selected from the group consisting of a ≥ 6 base cutter restriction endonuclease, a nicking enzyme, or the like. Some example 6 base cutters include Acll, Afel, Nspl, Haell, Tatl, and others that are commercially available, for example, from New England BioLabs Inc., ThermoFisher Scientific, etc.

The first and second oligonucleotides 78A, 78B in this example of the second cleaving fluid are respectively complementary to the cleavage sites 46D (i.e., the restriction sites) of the first and second primers 68A, 68B and to at least a portion of each of the first and second primers 68A, 68B that is 5' to the cleavage sites 46D. The sequences of the first and second oligonucleotides 78A, 78B enable them to respectively hybridize to at least some of the first and second primers 68A, 68B that are single stranded after sequencing. The primer 68B is single stranded after the amplicon 52 is sequenced, and thus the second oligonucleotides 78B hybridize to at least a portion of at least some of the primers 68B. Some of the primers 68A are hybridized to respective amplicons 52, and thus have a double stranded section at the cleavage sites 46D, which provides a substrate for the restriction enzyme cleavage. Others of the primers 68A do not participate in amplification and thus are not hybridized to respective amplicons 52. These primers 68A remain single stranded after amplification and sequencing, and thus the first oligonucleotides 78A hybridize to at least a portion of at least some of these primers 68A. Fig. 7C illustrates both of the possible hybridizations that may take place when this example of the second cleavage fluid is used.

While this example of the second cleaving fluid is in the flow cell 10, the flow cell 10 is exposed to a first temperature i) to initiate respective hybridization of: at least some of the plurality of first oligonucleotides 78A to at least some of the first primers 68A that are single stranded after sequencing, at least some of the plurality of second oligonucleotides 78B to at least some of the second primers 68B that are single stranded after sequencing, or both, and a second temperature ii) to initiate double stranded cleaving activity of the restriction enzyme. The first and second temperatures may be the same or different. In one example, the hybridization and digestion temperature may both be about 37°C. When different, the first temperature is higher than the second temperature, and the second temperature (i.e., the digestion temperature) is low enough to prevent dehybridization (denaturing) of the hybridized primers 68A and amplicons 52, the hybridized primers 68A and first oligonucleotides 78A, and the hybridized primers 68B and the second oligonucleotides 78B. As examples, the hybridization may be performed at about 60°C and the digestion may be performed at about 37°C, or the hybridization may be performed at about 40°C and the digestion may be performed at about 25°C.

The cleaving activity of the restriction enzyme cleaves the double stranded section at the cleavage sites 46D, represented by the lightning bolts in Fig. 7C. After cleavage, the temperature of the flow cell 10 may be raised to denature strand portions (e.g., portions of the amplicon 52 and/or of the oligonucleotides 78A, 78B) that remain hybridized to remaining portions of the primers 68A, 68B. Denaturing renders the remaining portions 66D of the at least some of the first primers 68A and the remaining portions 66E of at least some of the second primers 68B single stranded, as shown in Fig. 7D. The flow cell 10 may be rinsed with a washing fluid to remove all of the cleaved and denatured strands.

In another example shown in Fig. 7C, each of the first cleavage sites 46D includes a nucleobase selected from the group consisting of a uracil, an 8-oxoguanine, and an allyl-nucleobase, and the second cleaving fluid includes a plurality of first oligonucleotides 78A, each first oligonucleotide 78A being complementary to at least a portion of the first primer 68A; a plurality of second oligonucleotides 78B, each second oligonucleotide 78B being complementary to at least a portion of the second primer 68B; and an excision enzyme (not shown).

In this example, the excision enzyme in the second cleaving fluid will depend upon the nucleobase used as the cleavage sites 46D. Examples of suitable excision enzymes include the USER^{™} enzyme, an enzyme isolated from Pyrococcus furiosus, FPG, etc.

The first and second oligonucleotides 78A, 78B in this example of the second cleaving fluid are respectively complementary to the cleavage sites 46D (i.e., the nucleobase) of the first and second primers 68A, 68B and to at least a portion of each of the first and second primers 68A, 68B that is 5' to the cleavages sites 46D. The sequences of the first and second oligonucleotides 78A, 78B enable them to respectively hybridize to at least some of the first and second primers 68A, 68B that are single stranded after sequencing. The primer 68B is single stranded after the amplicon 52 is sequenced, and thus the second oligonucleotides 78B hybridize to at least a portion of at least some of the primers 68B. Some of the primers 68A are hybridized to respective amplicons 52, and thus have a double stranded section at the cleavage sites 46D, which provides a substrate for the excision enzyme cleavage. Others of the primers 68A do not participate in amplification and thus are not hybridized to respective amplicons 52. These primers 68A remain single stranded after amplification and sequencing, and thus the first oligonucleotides 78A hybridize to at least a portion of at least some of these primers 68A. Fig. 7C illustrates both of the possible hybridizations that may take place when this example of the second cleavage fluid is used.

While this example of the second cleaving fluid is in the flow cell 10, the flow cell 10 is exposed to a first temperature i) to initiate respective hybridization of: at least some of the plurality of first oligonucleotides 78A to at least some of the first primers 68A that are single stranded after sequencing, at least some of the plurality of second oligonucleotides 78B to at least some of the second primers 68B that are single stranded after sequencing, or both, and a second temperature ii) to initiate double stranded cleaving activity of the excision enzyme. The first and second temperatures may be the same or different. In one example, the hybridization and excision temperature may both be about 37°C. When different, the first temperature is higher than the second temperature, and the second temperature (i.e., the excision temperature) is low enough to prevent dehybridization (denaturing) of the hybridized primers 68A and amplicons 52, the hybridized primers 68A and first oligonucleotides 78A, and the hybridized primers 68B and the second oligonucleotides 78B.

The cleaving activity of the excision enzyme cleaves the primers 68A, 68B at the cleavage sites 46D, represented by the lightning bolts in Fig. 7C. After cleavage, the temperature of the flow cell 10 may be raised to denature strands (e.g., amplicons 52 and/or oligonucleotides 78A, 78B) that remain hybridized to remaining portions of the primers 68A, 68B. Denaturing renders the remaining portions 66D of the at least some of the first primers 68A and the remaining portions 66E of the at least some of the second primers 68B single stranded, as shown in Fig. 7D. The flow cell 10 may be rinsed with a washing fluid to remove all of the cleaved and denatured strands.

When the cleaving fluid with the excision enzyme is used, an additional enzyme with 3'-phosphatase activity (e.g., DNA phosphatase, T4 phage polynucleotide kinase (PNK)) may be introduced into the flow cell 10. This enzyme processes the phosphate ends, converting them into 3'-hydroxyls.

The methods shown in Fig. 7A through Fig. 7F then involve the sequential regeneration of the cleaved portions 72A, 72B (see Fig. 7A) of the primers 68A, 68B. Sequential regeneration of the cleaved portion 72A of the at least some of the first primers 68A and the cleaved portion 72B of the at least some of the second primers 68B involves: as shown in Fig. 7E, introducing a plurality of first regeneration oligonucleotides 60' into the flow cell 10, whereby first portions 80A of at least some of the first regeneration oligonucleotides 60' respectively hybridize to the remaining portions 66D of the at least some of the first primers 68A, and wherein each first regeneration oligonucleotide 60' further includes a second portion 82A that is complementary to the cleaved portion 72A of the first primer 68A; and initiating polymerase extension along respective second portions 82A of the hybridized first regeneration oligonucleotides 60' to regenerate the cleaved portion 72A of the at least some of the first primers 68A; and as shown in Fig. 7F, introducing a plurality of second regeneration oligonucleotides 60" into the flow cell, whereby first portions 80B of at least some of the second regeneration oligonucleotides 60" respectively hybridize to the remaining portions 66E of the at least some of the second primers 68B, wherein each second regeneration oligonucleotide 60" further includes a second portion 82B that is complementary to the cleaved portion 72B of the second primer 68B; and initiating polymerase extension along respective second portions 82B of the hybridized second regeneration oligonucleotides 60" to regenerate the cleaved portion 72B of the at least some of the second primers 68B.

As shown in Fig. 7E, each regeneration oligonucleotide 60' includes the first portion 80A that is complementary to at least a portion 70A of the remaining portion 66D of the primer 68A and the second portion 82A that is complementary to the portions of the primer 68A that were cleaved by the cleaving fluids. Thus, the sequence of the regeneration oligonucleotide 60' depends upon the original sequence of the first primer 68A.

The regeneration oligonucleotides 60' are introduced at conditions that enable the first portions 80A of the regeneration oligonucleotides 60' to respectively hybridize to at least the portion 70A of the remaining portions 66D of at least some of the primers 68A. The regeneration oligonucleotides 60' will not hybridize to the remaining portions 66E of the second primers 68B as these portions 66E have different sequences than the remaining portions 66D of the first primers 68A.

A mixture containing the cleavage sites 46D, 46E, nucleotides, and a polymerase are then introduced into the flow cell 10. The temperature of the flow cell 10 may be adjusted to initiate polymerase chain reaction (PCR). The polymerase enables the PCR extension of the 3' end of the remaining portion 66D using the second portion 82A of the regeneration oligonucleotide 60' as a template. Because the PCR extension reaction is guided by the second portion 82A of the regeneration oligonucleotide 60' and the second portion 82A is complementary to the portions of the primer 68A that were cleaved by the cleaving fluids, the polymerase extension along the second portion 82A regenerates at least the cleavage sites 46D, 46E of the first primer 68A. In an example, the PCR extension incorporates the cleavage sites 46D, 46E and the nucleotide(s) from the mixture to regenerate the first primer 68A.

Once the first primers 68A are regenerated, the regeneration oligonucleotides 60' are denatured, and the second primers 68B are regenerated.

As shown in Fig. 7F, each regeneration oligonucleotide 60" includes the first portion 80B that is complementary to at least a portion 70B of the remaining portion 66E of the primer 68B and the second portion 82B that is complementary to the portion of the primer 68A that were cleaved by the second cleaving fluid. Thus, the sequence of the regeneration oligonucleotide 60" depends upon the original sequence of the first primer 68B.

The regeneration oligonucleotides 60" are introduced at conditions that enable the first portions 80B of the regeneration oligonucleotides 60" to respectively hybridize to at least the portion 70B of the remaining portions 66E of at least some of the primers 68B. The regeneration oligonucleotides 60" will not hybridize to the regenerated first primers 68A, as these primers 68A have different sequences than the remaining portions 66E of the second primers 68B.

A mixture containing the cleavage site 46D, nucleotides, and a polymerase are then introduced into the flow cell 10. The temperature of the flow cell 10 may be adjusted to initiate polymerase chain reaction (PCR). The polymerase enables the PCR extension of the 3' end of the remaining portion 66E using the second portion 82B of the regeneration oligonucleotide 60" as a template. Because the PCR extension reaction is guided by the second portion 82B of the regeneration oligonucleotide 60" and the second portion 82B is complementary to the portions of the primer 68B that were cleaved by the second cleaving fluid, the polymerase extension along the second portion 82B regenerates at least the cleavage site 46D of the second primer 68B. In an example, the PCR extension incorporates the cleavage sites 46D and the nucleotide(s) from the mixture to regenerate the second primer 68B.

Once the second primers 68B are regenerated, the regeneration oligonucleotides 60" are denatured, and the flow cell 10 surface can be exposed to another sample for amplification and sequencing. Thus, the method shown in Fig. 7A through Fig. 7F may be repeated with a new sample. One example of the method involves: introducing a second sample into the flow cell 10 (moving from Fig. 7F back to Fig. 7A) and performing amplification, introduction of the first cleaving fluid, sequencing, introduction of the second cleaving fluid, and sequential primer 68A, 68B regeneration as described herein.

The method of Fig. 7A through Fig. 7D and Fig. 8 will now be described. In this example method, sample introduction and amplification may be performed as described in reference to Fig. 7A, cleavage of the amplicons 50 and sequencing of the amplicons 52 may be performed as described in reference to Fig. 7B, and strand cleavage (with any example of the second cleaving fluid) and removal to generate the remaining portions 66D, 66E may be performed as described in reference to Fig. 7C and Fig. 7D.

The method of Fig. 7A through Fig. 7D and Fig. 8 then involves the simultaneous regeneration of the cleaved portions 72A, 72B (see Fig. 7A) of the primers 68A, 68B. Simultaneous regeneration of the cleaved portion 72A of the at least some of the first primers 68A and the cleaved portion 72B of the at least some of the second primers 68B involves: introducing a plurality of regeneration splints 84, 84', including first regeneration splints 84 and second regeneration splints 84', into the flow cell 10, whereby splint portions 86A of the first regeneration splints 84 respectively hybridize to the remaining portions 66D of the at least some of the first primers 68A and splint portions 86B of the second regeneration splints 84' respectively hybridize to the remaining portions 66D of the at least some of the second primers 68B, each first regeneration splint 84 further including a first ligate portion 88A hybridized to the splint portion 86A and having a sequence of the cleaved portion 72A of the first primer 68A, and each second regeneration splint 84' further including a second ligate portion 88B hybridized to the splint portion 86B and having a sequence of the cleaved portion 72B of the second primer 68B; and initiating ligation of at least some of the first ligate portions 88A to the remaining portions 66D of the at least some of the first primers 68A and at least some of the second ligate portions 88B to the remaining portions 66E of the at least some of the second primers 68B.

As shown in Fig. 8, each of the regeneration splints 84, 84' includes the splint portion 86A, 86B and the ligate portions 88A, 88B respectively hybridized at the 5' end of the splint portions 86A, 86B. In the regeneration splint 84, the portion of the sequence of the splint portion 86A at the 5' end is complementary to the ligate portion 88A, and the portion of the sequence of the splint portion 86A at the 3' end is complementary to at least a portion 70A of the remaining portion 66D of the first primer 68A. The portion of the sequence of the splint portion 86A at the 3' end hybridizes to the portion 70A and thus guides the ligate portion 88A into position for ligation to the remaining portion 66D of the first primer 68A.

Similarly, in the regeneration splint 84', the portion of the sequence of the splint portion 86B at the 5' end is complementary to the ligate portion 88B, and the portion of the sequence of the splint portion 86A at the 3' end is complementary to at least a portion 70B of the remaining portion 66E of the second primer 68B. The portion of the sequence of the splint portion 86B at the 3' end hybridizes to the portion 70B and thus guides the ligate portion 88B into position for ligation to the remaining portion 66E of the second primer 68B.

The sequence of the ligate portion 88A of the regeneration splint 84 is identical to the portions of the first primer 68A that were cleaved during the method shown in Fig. 7A through Fig. 7D. Similarly, the sequence of the ligate portion 88B of the regeneration splint 84' is identical to the portion of the second primer 68B that was cleaved during the method shown in Fig. 7A through Fig. 7D. Thus, the respective ligation of the ligate portions 88A, 88B to the remaining portions 66D, 66E regenerates the primers 68A, 68B.

The regeneration splints 84, 84' are introduced at conditions that enable respective hybridization of the regeneration splints 84, 84' to the portions 70A, 70B. A mixture containing the regeneration splints 84, 84' and a ligase 90 is introduced into the flow cell 10. Examples of suitable ligases 90 include E. Coli ligase (centaur protocol), T4DNA ligase, Taq DNA ligase, etc. The temperature of the flow cell 10 may be adjusted to initiate hybridization and ligation. The ligase enables the respective splint assisted ligation of the 5' end of the ligate portions 88A, 88B to the 3' end of the remaining portions 66D, 66E.

Once the first and second primers 68A, 68B are regenerated, the splint portions 86A, 86B are denatured and the flow cell is ready for exposure to a second (or subsequent) sample.

### Flow Cell Generation Kits

Some examples of the flow cell surfaces include anchor oligonucleotides 34 attached to the polymeric hydrogel 28. These flow cells 10 may be included in a kit that enables the desired primers 30, 32 to be grafted prior to sequencing and removed post-sequencing. Thus, the flow cell surfaces are reusable.

In an example, the flow cell regeneration kit includes a flow cell 10 including: the substrate (e.g., 14 or 16); the polymeric hydrogel 28 attached to at least a portion of the substrate; and nuclease resistant anchor oligonucleotides 34 (examples of which are shown in Fig. 9, Fig. 10, and Fig. 11); and a primer fluid including: a plurality of first primers 30 to be respectively ligated to at least some of the first nuclease resistant oligonucleotides 34; and a plurality of second primers 32 to be respectively ligated to at least some of the second nuclease resistant oligonucleotides 34.

All of the examples of the nuclease resistant oligonucleotides 34 include a short nucleotide sequence (e.g., containing from about 10 nucleotides to about 50 nucleotides), which contains any example of the nuclease resistant modification incorporated at the 3' end. In an example, each of the nuclease resistant anchor oligonucleotides 34 includes a 3' end nuclease resistant modification selected from the group consisting of a phosphorothioate bond, methyl phosphonate, an inverted nucleotide, a peptide nucleic acid, a morpholino, 2'-O-methyl, a 3' end phosphorylated nucleotide, and a nucleotide including a phosphoramidite C3 spacer.

The 3' end of the short nucleotide sequence is also capable of being ligated to the first and second primers 30, 32. The short nucleotide sequence is also selected so that it is not capable of hybridizing to the first and second primers 30, 32. Each of the nuclease resistant oligonucleotides 34 also includes a functional group at the 5' end that can be grafted to the polymeric hydrogel 28.

The primer fluid includes the primers 30, 32 in a fluid carrier. The fluid carrier may include water, a buffer, and a catalyst. The buffer may be an ionic salt buffer fluid, such as saline citrate at milli-molar to molar concentrations, sodium chloride, potassium chloride, phosphate buffered saline, etc., and other buffers, such as tris(hydroxymethyl)aminomethane (TRIS) or (4-(2-hydroxyethyl)-1-piperazineethanesulfonic acid) (HEPES). The liquid carrier may also include catalytic metal(s) intended for the ligation reaction, such as Mg²⁺, Mn²⁺, Ca²⁺, etc. A single catalytic metal or a combination of catalytic metals may be used, and the total amount may range from about 0.01 mM to about 100 mM.

In these examples, the primers 30 and 32 may respectively include the P5 and P7 primer sequences, the P15 and P7 primer sequences, or any combination of the PA primer sequences, the PB primer sequences, the PC primer sequences, and the PD primer sequences set forth herein. The 5' end of the primers 30 and 32 is also capable of being ligated to the 3' end of the nuclease resistant oligonucleotides 34.

In these examples, the primers 30 and 32 may include orthogonal cleavage sites (e.g., uracil and oxo-guanine) so that forward and reverse amplicons can be removed via exposure to different cleaving agents.

Several examples of combinations of the nuclease resistant oligonucleotides 34 and the primers 30, 32 will now be described in reference to Fig. 9, Fig. 10, and

Fig. 11. The methods shown in Fig. 9, Fig. 10, and Fig. 11, which utilize the combinations of the nuclease resistant oligonucleotides 34 and the primers 30, 32, involve introducing a primer fluid into the flow cell 10 including the substrate, the polymeric hydrogel 28 attached to at least a portion of the substrate, and nuclease resistant anchor oligonucleotides 34 attached to the polymeric hydrogel 28 (shown at A and B in each of the figures); initiating ligation of a first primer 30 in the primer fluid to at least some of the nuclease resistant anchor oligonucleotides 34 and a second primer 32 in the primer fluid to at least some other of the nuclease resistant anchor oligonucleotides 34 (shown at C in each of the figures); performing a sequencing operation using the first and second primers 30, 32 (shown at D in each of the figures); and cleaving the first and second primers 30, 32 after the sequencing operation to expose the nuclease resistant anchor oligonucleotides 34 (shown at E and A in each of the figures).

Two examples combinations are shown in Fig. 9.

In one example shown in Fig. 9, each of the nuclease resistant anchor oligonucleotides 34A is 3' end hydroxyl terminated (see A in Fig. 9), and each of the first primers 30A and each of the second primers 32A is 3' end phosphate terminated and 5' end adenylated (see B in Fig. 9). The 5' adenylated end can be ligated to 3'-hydroxyl of the nuclease resistant anchor oligonucleotides 34A (see C in Fig. 9). Ligation of these primers 30A, 32A may be initiated by introducing a ligase with the primer fluid, the ligase being selected from the group consisting of T4 RNA Ligase I and Thermostable 5' App Ligase, and adjusting a temperature of the flow cell 10 to a reaction temperature for the ligase. As examples, the temperature may be about 25°C when T4 RNA Ligase I is used and may be about 65°C when Thermostable 5' App Ligase is used. The flow cell 10 may be exposed to a wash cycle to remove any non-ligated primers 30A, 32A.

Prior to performing the sequencing operation, the method further includes deprotecting the first and second primers 30A, 32A at the 3' ends (see D in Fig. 9). The phosphate terminated 3' ends may be deprotected by introducing an enzyme with 3'-phosphatase activity (e.g., T4 phage polynucleotide kinase (PNK) or alkaline phosphatase (AP or ALP)). These enzymes process the phosphate ends, converting them into 3'-hydroxyls and render them ready for amplification and sequencing as described herein.

As shown at "E" in Fig. 9, amplification and sequencing may be performed as described herein. A sample containing library fragments may be introduced, amplified, linearized (e.g., with a suitable cleaving agent for one of the generated amplicons 50, 52), and sequenced as described herein.

Once sequencing is complete and any desirable wash cycles are performed, the flow cell 10 may be exposed to a nuclease enzyme and an alkaline phosphatase treatment to cleave the first and second primers 30A, 32A (see F in Fig. 9). The nuclease enzyme has 3'→5' activity. In the examples involving 3' end phosphate terminated and 5' end adenylated primers 30A, 32A, cleaving may be performed by introducing DNAse I and Exonuclease I. As the nuclease resistant anchor oligonucleotides 34A are resistant to the exonuclease activity, cleavage of the first and second primers 30A, 32A exposes the nuclease resistant anchor oligonucleotides 34A (see A in Fig. 9).

The flow cell surface shown in Fig. 9 may be reused in one or more additional rounds of primer 30A, 32A ligation, amplification, sequencing, and cleaving as described herein.

In another example shown in Fig. 9, each of the nuclease resistant anchor oligonucleotides 34A is 3' end hydroxyl terminated (see A in Fig. 9), and each of the first and second primers 30B, 32B is 3' end phosphate terminated and 5' end phosphate terminated (see B in Fig. 9). The 5' end phosphate can be ligated to 3'-hydroxyl of the nuclease resistant anchor oligonucleotides 34A (see C in Fig. 9). Ligation of these primers 30B, 32B may be initiated by introducing T4 RNA Ligase I with the primer fluid, and adjusting a temperature of the flow cell 10 to a reaction temperature for the T4 RNA Ligase I (e.g., about 25°C). The flow cell 10 may be exposed to a wash cycle to remove any non-ligated primers 30B, 32B.

Prior to performing the sequencing operation, the method further includes deprotecting the first and second primers 30B, 32B at the 3' ends (see D in Fig. 9). The phosphate terminated 3' ends may be deprotected by introducing an enzyme with 3'-phosphatase activity (e.g., T4 phage polynucleotide kinase (PNK) or alkaline phosphatase (AP or ALP)). These enzymes process the phosphodiester ends, converting them into 3'-hydroxyls and render them ready for amplification and sequencing as described herein.

As shown at "E" in Fig. 9, amplification and sequencing may be performed as described herein. A sample containing library fragments may be introduced, amplified, linearized (e.g., with a suitable cleaving agent for one of the generated amplicons 50, 52), and sequenced as described herein.

Once sequencing is complete and any desirable wash cycles are performed, the flow cell 10 may be exposed to a nuclease enzyme and an alkaline phosphatase treatment to cleave the first and second primers 30B, 32B (see F in Fig. 9). The nuclease enzyme has 3'→5' activity. In the examples involving 3' end phosphate terminated and 5' end phosphate terminated primers 30B, 32B, cleaving may be performed by introducing DNAse I and Exonuclease I. As the nuclease resistant anchor oligonucleotides 34A are resistant to the nuclease activity, cleavage of the first and second primers 30B, 32B exposes the nuclease resistant anchor oligonucleotides 34A (see A in Fig. 9).

The flow cell surface shown in Fig. 9 may be reused in one or more additional rounds of primer 30B, 32B ligation, amplification, sequencing, and cleaving as described herein.

Referring now to Fig. 10, each of the nuclease resistant anchor oligonucleotides 34A is 3' end hydroxyl terminated (see A in Fig. 10); each of the first primers 30C is 3' end hydroxyl terminated and 5' end phosphate terminated and is hybridized to a respective splint 92 having a portion 94 that is to hybridize to one of the nuclease resistant anchor oligonucleotides 34A (see B in Fig. 10); and each of the second primers 32C is 3' end hydroxyl terminated and 5' end phosphate terminated and is hybridized to a respective splint 96 having a portion 98 that is to hybridize to one of the nuclease resistant anchor oligonucleotides 34A (see B in Fig. 10). The splint 92 includes a second portion that is hybridized at the 5' end of the primer 30C and the splint 96 includes a second portion that is hybridized at the 5' end of the primer 32C.

The portions 94 and 98 of the splints 92 and 96 may have the same sequence, which is complementary to the sequence of the nuclease resistant anchor oligonucleotides 34A. When the primer fluid is introduced, the flow cell 10 may be heated to initiate respective hybridization of the portions 94 and 98 to different nuclease resistant anchor oligonucleotides 34A. Thus, the portions 94, 96 can guide the respective primers 30C, 32C into position for ligation to the nuclease resistant anchor oligonucleotides 34A.

The phosphate terminated 5' ends of the primers 30C, 32C can be respectively ligated to 3'-hydroxyl of the nuclease resistant anchor oligonucleotides 34A (see C in Fig. 10). Ligation of these primers 30C, 32C may be initiated by introducing a ligase with the primer fluid, the ligase being selected from the group consisting of T4 RNA Ligase II and PBCV-1 DNA Ligase, and adjusting a temperature of the flow cell 10 to a reaction temperature for the ligase. As examples, a suitable temperature for T4 RNA Ligase II is about 37°C and for PBCV-1 DNA Ligase is about 37°C. Once ligation is complete, the flow cell 10 may be brought to a suitable temperature to denature the splints 92, 96. The flow cell 10 may be exposed to a wash cycle to remove non-attached primers 30C, 32C (having the splints 92, 96 attached thereto) and denatured splints 92, 96.

As shown at "D" in Fig. 10, amplification and sequencing may be performed as described herein. Because the primers 30C, 32C are 3' hydroxyl terminated, no additional deprotection is performed prior to amplification and sequencing. A sample containing library fragments may be introduced, amplified, linearized (e.g., with a suitable cleaving agent for one of the generated amplicons 50, 52), and sequenced as described herein.

Once sequencing is complete and any desirable wash cycles are performed, the flow cell 10 may be exposed to a nuclease enzyme and an alkaline phosphatase treatment to cleave the first and second primers 30C, 32C (see E in Fig. 10). The nuclease enzyme has 3'→5' activity. In the examples involving 3' end hydroxyl terminated and 5' end phosphate terminated primers 30C, 32C, cleaving may be performed by introducing DNAse I and Exonuclease I. As the nuclease resistant anchor oligonucleotides 34A are resistant to the nuclease activity, cleavage of the first and second primers 30C, 32C exposes the nuclease resistant anchor oligonucleotides 34A (see A in Fig. 10).

The flow cell surface shown in Fig. 10 may be reused in one or more additional rounds of splint hybridization and primer 30C, 32C ligation, amplification, sequencing, and cleaving as described herein.

In the example shown in Fig. 11, each of the nuclease resistant anchor oligonucleotides 34B is 3' end phosphate terminated (see A in Fig. 11); and each of the first primers 30D and each of the second primers 32D is 3' end hydroxyl terminated and 5' end hydroxyl terminated (see B in Fig. 11). The 5' end hydroxyl can be ligated to 3'-phosphate of the nuclease resistant anchor oligonucleotides 34B (see C in Fig. 11). Ligation of these primers 30D, 32D may be initiated by introducing an RtcB ligase with the primer fluid, and adjusting a temperature of the flow cell 10 to a reaction temperature for the RtcB ligase (e.g., 37°C). The flow cell 10 may be exposed to a wash cycle to remove any non-ligated primers 30D, 32D.

As shown at "D" in Fig. 11, amplification and sequencing may be performed as described herein. Because the primers 30D, 32D are 3' end hydroxyl terminated, no additional deprotection is performed prior to amplification and sequencing. A sample containing library fragments may be introduced, amplified, linearized (e.g., with a suitable cleaving agent for one of the generated amplicons 50, 52), and sequenced as described herein.

Once sequencing is complete and any desirable wash cycles are performed, the flow cell 10 may be exposed to a nuclease enzyme and an alkaline phosphatase treatment to cleave the first and second primers 30D, 32D (see E in Fig. 11). The nuclease enzyme has 3'→5' activity. In the examples involving 3' end hydroxyl terminated and 5' end phosphate terminated primers 30D, 32D, cleaving may be performed by introducing DNAse II or MNase. As the nuclease resistant anchor oligonucleotides 34A are resistant to the nuclease activity, cleavage of the first and second primers 30D, 32D exposes the nuclease resistant anchor oligonucleotides 34A (see A in Fig. 11).

The flow cell surface shown in Fig. 11 may be reused in one or more additional rounds of primer 30D, 32D ligation, amplification, sequencing, and cleaving as described herein.

In any of the examples shown in Fig. 9 through Fig. 11, it is to be understood that i) a 3' end of each of the nuclease resistant anchor oligonucleotides 34A, 34B may include an RNA oligonucleotide or a 2'-O-methyl; or ii) a 5' end of each of the plurality of first primers 30A, 30B and the plurality of second primers 32A, 32B includes an RNA oligonucleotide or a 2'-O-methyl; or iii) both i) and ii). The addition of the RNA oligonucleotide(s) or the 2'-O-methyl at the 3' termini of the nuclease resistant anchor oligonucleotides 34A, 34B or the 5' termini of the first primers 30A, 30B and the plurality of second primers 32A, 32B may improve the efficiency of the ligation.

In any examples with the flow cell surfaces described in reference to Fig. 9, Fig. 10, and Fig. 11, the method may involve introducing a second primer fluid into the flow cell 10; initiating ligation of a third primer (similar to 30A, 30B, 30C, or 30D) in the second primer fluid to at least some of the nuclease resistant anchor oligonucleotides 34A, 34B and a fourth primer (similar to 32A, 32B, 32C, or 32D) in the second primer fluid to the some other of the nuclease resistant anchor oligonucleotides 34A, 34B; and performing a second sequencing operation using the third and fourth primers.

### Additional Notes

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any referenced disclosure should be accorded a meaning most consistent with the particular concepts disclosed herein.

Reference throughout the specification to "one example", "another example", "an example", and so forth, means that a particular element (e.g., feature, structure, and/or characteristic) described in connection with the example is included in at least one example described herein, and may or may not be present in other examples. In addition, it is to be understood that the described elements for any example may be combined in any suitable manner in the various examples unless the context clearly dictates otherwise.

While several examples have been described in detail, it is to be understood that the disclosed examples may be modified. Therefore, the foregoing description is to be considered non-limiting.

## Claims

1. A primer set, comprising:
a first nuclease resistant primer including:
a first sequence;
a first cleavage site attached at a 3' end of the first sequence; and
a first nuclease resistant modification incorporated between the first sequence and the first cleavage site; and
a second nuclease resistant primer including:
a second sequence that is different from the first sequence;
a second nuclease resistant modification incorporated at a 3' end of the second sequence; and
a second cleavage site attached between the second sequence and the second nuclease resistant modification, wherein the second cleavage site is different from the first cleavage site.

2. The primer set as defined in claim 1, wherein the first nuclease resistant modification and the second nuclease resistant modification are a same type of nuclease resistant modification, optionally wherein:
the first and second nuclease resistant modifications are selected from the group consisting of a phosphorothioate bond, methyl phosphonate, an inverted nucleotide, a peptide nucleic acid, a morpholino, 2'-O-methyl, and a nucleotide including a phosphoramidite C3 spacer; and each of the first and second cleavage sites includes a nucleobase independently selected from the group consisting of a uracil, an 8-oxoguanine, and an allyl-nucleotide.

3. A reusable flow cell, comprising:
a substrate;
a polymeric hydrogel attached to at least a portion of the substrate; and
a primer set attached to the polymeric hydrogel, the primer set selected from the group consisting of:
i) a first nuclease resistant primer including a first cleavage site; and
a second nuclease resistant primer including a second cleavage site that is different from the first cleavage site; and
ii) a first nuclease resistant primer including a cleavage site; and
a second nuclease resistant primer without any cleavage site.

4. The reusable flow cell as defined in claim 3, wherein:
(a) the primer set is i);
the first nuclease resistant primer includes:
a first sequence;
the first cleavage site attached at a 3' end of the first sequence; and
a first nuclease resistant modification incorporated between the first sequence and the first cleavage site; and
the second nuclease resistant primer includes:
a second sequence that is different from the first sequence;
a second nuclease resistant modification incorporated at a 3' end of the second sequence; and
the second cleavage site attached between the second sequence and
the second nuclease resistant modification;
optionally wherein:
the first nuclease resistant modification and the second nuclease resistant modification are a same type of nuclease resistant modification;
the first and second nuclease resistant modifications are selected from the group consisting of a phosphorothioate bond, methyl phosphonate, an inverted nucleotide, a peptide nucleic acid, a morpholino, 2'-O-methyl, , and a nucleotide including a phosphoramidite C3 spacer; and
each of the first and second cleavage sites includes a nucleobase independently selected from the group consisting of a uracil, an 8-oxoguanine, and an allyl-nucleotide;
or
(b) the primer set is ii);
the first nuclease resistant primer includes:
a first sequence;
the cleavage site attached at a 3' end of the first sequence; and
a first nuclease resistant modification incorporated between the first sequence and the cleavage site; and
the second nuclease resistant primer includes:
a second sequence that is different from the first sequence; and
a second nuclease resistant modification incorporated at a 3' end of the second sequence;
optionally wherein:
the first nuclease resistant modification and the second nuclease resistant modification are a same type of nuclease resistant modification;
the first and second nuclease resistant modifications are selected from the group consisting of a phosphorothioate bond, methyl phosphonate, an inverted nucleotide, a peptide nucleic acid, a morpholino, 2'-O-methyl, and a nucleotide including a phosphoramidite C3 spacer; and
the cleavage site of the first nuclease resistant primer includes a nucleobase selected from the group consisting of a uracil, an 8-oxoguanine, and an allyl-nucleotide.

5. A method, comprising:
(a) introducing a first sample into a flow cell including:
a substrate;
a polymeric hydrogel attached to at least a portion of the substrate;
a plurality of primer sets attached to the polymeric hydrogel, each primer set in the plurality including a first primer and a second primer;
(b) amplifying a library fragment of the first sample, thereby generating a first plurality of first sample library fragment amplicons respectively attached to a plurality of the first primers and a second plurality of first sample library fragment amplicons respectively attached to a plurality of the second primers;
(c) introducing a cleaving fluid into the flow cell, thereby cleaving the first primers at cleavage sites, whereby the first plurality of first sample library fragment amplicons are removed; and
(d) sequencing the second plurality of first sample library fragment amplicons, thereby generating a plurality of first sample library fragment nascent strands;
wherein either option (I), (II) or (III) applies:
(I) in step (a) said first primer is a nuclease resistant primer including a first cleavage site and said second primer is a second nuclease resistant primer including a second cleavage site that is different from the first cleavage site, said library fragment amplified in step (b) is a first library fragment, in step (c) said cleaving fluid is a first cleaving site cleaving fluid and the cleavage sites at which the first primers are cleaved are the first cleavage sites; wherein the method further comprises:
(e) introducing a nuclease enzyme to the flow cell, thereby cleaving the second plurality of first sample library fragment amplicons and the plurality of first sample library fragment nascent strands;
(f) introducing a second sample into the flow cell;
(g) amplifying a second library fragment of the second sample, thereby generating a first plurality of second sample library fragment amplicons respectively attached to a second plurality of the first nuclease resistant primers and a second plurality of second sample library fragment amplicons attached to a second plurality of the second nuclease resistant primers;
(h) introducing a second cleavage site cleaving fluid into the flow cell, thereby cleaving the second nuclease resistant primers at the second cleavage sites, whereby the second plurality of second sample library fragment amplicons are removed; and
(i) sequencing the first plurality of second sample library fragment amplicons, thereby generating a plurality of second sample library fragment nascent strands;
OR
(II) in step (a) said first primer is a nuclease resistant primer including a cleavage site and said second primer is a second nuclease resistant primer without any cleavage site, wherein the method further comprises:
(e) introducing a nuclease enzyme into the flow cell, thereby cleaving the second plurality of first sample library fragment amplicons and the plurality of first sample library fragment nascent strands;
(f) introducing a plurality of regeneration oligonucleotides into the flow cell, whereby first portions of at least some of the regeneration oligonucleotides respectively hybridize to remaining portions of at least some of the first nuclease resistant primers, wherein each regeneration oligonucleotide further includes a second portion that is complementary to a cleaved portion of the first nuclease resistant primer; and
(g) initiating polymerase extension along respective second portions of the hybridized regeneration oligonucleotides to regenerate the cleaved portion of the at least some of the first nuclease resistant primers;
OR
(III) in step (a) said first primer has a first cleavage site and a second cleavage site that is different from the first cleavage site, and said second primer has the first cleavage site without the second cleavage site; in step (c) the cleavage sites at which the first primers are cleaved are the second cleavage sites; wherein the method further comprises:
(e) introducing a second cleaving fluid into the flow cell, thereby cleaving each of the first primers and the second primers at their respective first cleavage sites and leaving remaining portions of the first primers and of the second primers attached to the polymeric hydrogel; and
(f) sequentially or simultaneously regenerating i) a cleaved portion of at least some of the first primers at the remaining portions of the at least some of the first primers, and ii) a cleaved portion of at least some of the second primers at the remaining portions of the at least some of the second primers.

6. The method as defined in claim 5, wherein the method is the method of option (I) and wherein:
each of the first cleavage sites includes a uracil nucleobase;
the first cleavage site cleaving fluid includes a mixture of uracil DNA glycosylase and the DNA glycosylase-lyase Endonuclease VIII or an enzyme isolated from Pyrococcus furiosus;
each of the second cleavage sites includes an 8-oxoguanine nucleobase;
the second cleavage site cleaving fluid includes formamidopyrimidine [fapy]-DNA glycosylase; and
the nuclease enzyme is selected from the group consisting of Exonuclease I, Thermolabile Exonuclease I, Exonuclease T, Exonuclease VII, Mung Bean Nuclease, Nuclease P1, Exonuclease III, Exonuclease V, Nuclease BAL-31, DNase I, and Micrococcal Nuclease.

7. The method according to claim 5, wherein the method is the method of option (II).

8. The method as defined in claim 7, wherein:
(i) the cleavage site includes a uracil nucleobase;
the cleaving fluid includes a mixture of uracil DNA glycosylase and the DNA glycosylase-lyase Endonuclease VIII; and
the nuclease enzyme is selected from the group consisting of Exonuclease I, Thermolabile Exonuclease I, Exonuclease T, Exonuclease VII, Mung Bean Nuclease, Nuclease P1, Exonuclease III, Exonuclease V, Nuclease BAL-31, DNase I, and Micrococcal Nuclease;
or
(ii) the polymerase extension along the second portion of the regeneration oligonucleotide regenerates at least the cleavage site of the first nuclease resistant primer;
or
(iii) the method further comprises:
introducing a second sample into the flow cell after the polymerase extension;
amplifying a library fragment of the second sample, thereby generating a first plurality of second sample library fragment amplicons respectively attached to a second plurality of the first nuclease resistant primers and a second plurality of second sample library fragment amplicons attached to a second plurality of the second nuclease resistant primers;
introducing the cleaving fluid into the flow cell, thereby cleaving the first nuclease resistant primers at the cleavage sites, whereby the first plurality of second sample library fragment amplicons are removed; and
sequencing the second plurality of second sample library fragment amplicons, thereby generating a plurality of second sample library fragment nascent strands.

9. A flow cell, comprising:
a substrate;
a polymeric hydrogel attached to at least a portion of the substrate; and
a primer set attached to the polymeric hydrogel, the primer set including:
a first primer having a first cleavage site and a second cleavage site that is different from the first cleavage site; and
a second primer having the first cleavage site without the second cleavage site.

10. The flow cell as defined in claim 9, wherein:
(i) the first cleavage site is a restriction site; and
the second cleavage site includes a nucleobase selected from the group consisting of a uracil, an 8-oxoguanine, and an allyl-nucleotide;
or
(ii) the first cleavage site and the second cleavage site each include a nucleobase independently selected from the group consisting of a uracil, an 8-oxoguanine, and an allyl-nucleotide;
or
(iii) the first and second primers are nuclease resistant primers.

11. The method according to claim 5, wherein the method is the method of option (III).

12. The method as defined in claim 11, wherein:
each of the first cleavage sites is a restriction site;
the second cleaving fluid includes:
a plurality of first oligonucleotides, each first oligonucleotide being complementary to the remaining portion of the first primer;
a plurality of second oligonucleotides, each second oligonucleotide being complementary to the remaining portion of the second primer; and
a restriction enzyme; and
while the second cleaving fluid is in the flow cell, the flow cell is exposed to a first temperature i) to initiate respective hybridization of: at least some of the plurality of first oligonucleotides to at least some of the first primers that are single stranded after sequencing, at least some of the plurality of second oligonucleotides to at least some of the second primers that are single stranded after sequencing, or both, and a second temperature ii) to initiate double stranded cleaving activity of the restriction enzyme.

13. The method as defined in claim 12, further comprising denaturing to render the remaining portions of the at least some of the first primers and the remaining portions of at least some of the second primers single stranded;
optionally wherein:
(i) the method involves sequential regeneration of the cleaved portions; and
sequentially regenerating the cleaved portion of the at least some of the first primers and the cleaved portion of the at least some of the second primers involves:
introducing a plurality of first regeneration oligonucleotides into the flow cell, whereby first portions of at least some of the first regeneration oligonucleotides respectively hybridize to the remaining portions of the at least some of the first primers, wherein each first regeneration oligonucleotide further includes a second portion that is complementary to the cleaved portion of the first primer;
initiating polymerase extension along respective second portions of the hybridized first regeneration oligonucleotides to regenerate the cleaved portion of the at least some of the first primers;
introducing a plurality of second regeneration oligonucleotides into the flow cell, whereby first portions of at least some of the second regeneration oligonucleotides respectively hybridize to the remaining portions of the at least some of the second primers, wherein each second regeneration oligonucleotide further includes a second portion that is complementary to the cleaved portion of the second primer; and
initiating polymerase extension along respective second portions of the hybridized second regeneration oligonucleotides to regenerate the cleaved portion of the at least some of the second primers;
or
(ii) the method involves simultaneous regeneration of the cleaved portions; and
simultaneously regenerating the cleaved portion of the at least some of the first primers and the cleaved portion of the at least some of the second primers involves:
introducing a plurality of regeneration splints, including first regeneration splints and second regeneration splints, into the flow cell, whereby splint portions of the first regeneration splints respectively hybridize to the remaining portions of the at least some of the first primers and splint portions of the second regeneration splints respectively hybridize to the remaining portions of the at least some of the second primers, each first regeneration splint further including a first ligate portion hybridized to the splint portion and having a sequence of the cleaved portion of the first primer, and each second regeneration splint further including a second ligate portion hybridized to the splint portion and having a sequence of the cleaved portion of the second primer; and
initiating ligation of at least some of the first ligate portions to the remaining portions of the at least some of the first primers and at least some of the second ligate portions to the remaining portions of the at least some of the second primers.

14. The method as defined in claim 11, wherein:
the method involves sequential regeneration of the cleaved portions;
each of the first cleavage sites includes a nucleobase selected from the group consisting of a uracil, an 8-oxoguanine, and an allyl-nucleotide; and
the second cleaving fluid includes:
a plurality of first oligonucleotides, each first oligonucleotide being complementary to the remaining portion of the first primer;
a plurality of second oligonucleotides, each second oligonucleotide being complementary to the remaining portion of the second primer; and
an excision enzyme; and
while the second cleaving fluid is in the flow cell, the flow cell is exposed to a first temperature i) to initiate respective hybridization of: at least some of the plurality of first oligonucleotides to at least some of the first primers that are single stranded after sequencing, at least some of the plurality of second oligonucleotides to at least some of the second primers that are single stranded after sequencing, or both, and a second temperature ii) to initiate double stranded cleaving activity of the excision enzyme;
optionally wherein:
(a) the method further comprises denaturing to render the remaining portions of the at least some of the first primers and the remaining portions of at least some of the second primers single stranded; or
(b) the method further comprises denaturing to render the remaining portions of the at least some of the first primers and the remaining portions of at least some of the second primers single stranded, further wherein sequentially regenerating the cleaved portion of the at least some of the first primers and the cleaved portion of the at least some of the second primers involves:
introducing a plurality of first regeneration oligonucleotides into the flow cell, whereby first portions of at least some of the first regeneration oligonucleotides respectively hybridize to the remaining portions of the at least some of the first primers, wherein each first regeneration oligonucleotide further includes a second portion that is complementary to the cleaved portion of the first primer;
initiating polymerase extension along respective second portions of the hybridized first regeneration oligonucleotides to regenerate the cleaved portion of the at least some of the first primers;
introducing a plurality of second regeneration oligonucleotides into the flow cell, whereby first portions of at least some of the second regeneration oligonucleotides respectively hybridize to the remaining portions of the at least some of the second primers, wherein each second regeneration oligonucleotide further includes a second portion that is complementary to the cleaved portion of the second primer; and
initiating polymerase extension along respective second portions of the hybridized second regeneration oligonucleotides to regenerate the cleaved portion of the at least some of the second primers.

15. A flow cell generation kit, comprising:
a flow cell including:
a substrate;
a polymeric hydrogel attached to at least a portion of the substrate; and
nuclease resistant anchor oligonucleotides attached to the polymeric hydrogel; and
a primer fluid including:
a plurality of first primers to be respectively ligated to at least some of the nuclease resistant anchor oligonucleotides; and
a plurality of second primers to be respectively ligated to at least some other of the nuclease resistant anchor oligonucleotides.

16. The flow cell generation kit as defined in claim 15, wherein:
(a) each of the nuclease resistant anchor oligonucleotides is 3' end hydroxyl terminated; and
each of the first primers and each of the second primers is 3' end phosphate terminated and 5' end adenylated;
or
(b) each of the nuclease resistant anchor oligonucleotides is 3' end hydroxyl terminated;
each of the first primers is 3' end hydroxyl terminated and 5' end phosphate terminated, and is hybridized to a respective splint having a portion that is to hybridize to one of the nuclease resistant anchor oligonucleotides; and
each of the second primers is 3' end hydroxyl terminated and 5' end phosphate terminated, and is hybridized to a respective splint having a portion that is to hybridize to one of the nuclease resistant anchor oligonucleotides;
or
(c) each of the nuclease resistant anchor oligonucleotides is 3' end phosphate terminated; and
each of the first primers and each of the second primers is 3' end hydroxyl terminated and 5' end hydroxyl terminated;
or
(d) each of the nuclease resistant anchor oligonucleotides is 3' end hydroxyl terminated; and
each of the first primers and each of the second primers is 3' end phosphate terminated and 5' end phosphate terminated;
or
(e) each of the nuclease resistant anchor oligonucleotides includes a 3' end nuclease resistant modification selected from the group consisting of a phosphorothioate bond, methyl phosphonate, an inverted nucleotide, a peptide nucleic acid, a morpholino, 2'-O-methyl, a 3' end phosphorylated nucleotide, and a nucleotide including a phosphoramidite C3 spacer;
or
(f) (i) a 3' end of each of the nuclease resistant anchor oligonucleotides includes an RNA oligonucleotide or a 2'-O-methyl; or ii) a 5' end of each of the plurality of first primers and the plurality of second primers includes an RNA oligonucleotide or a 2'-O-methyl; or iii) both i) and ii).

17. A method, comprising:
introducing a primer fluid into a flow cell including:
a substrate;
a polymeric hydrogel attached to at least a portion of the substrate; and
nuclease resistant anchor oligonucleotides attached to the polymeric hydrogel;
initiating ligation of a first primer in the primer fluid to at least some of the nuclease resistant anchor oligonucleotides and a second primer in the primer fluid to at least some other of the nuclease resistant anchor oligonucleotides;
performing a sequencing operation using the first and second primers; and
cleaving the first and second primers after the sequencing operation to expose the nuclease resistant anchor oligonucleotides.

18. The method as defined in claim 17, wherein:
(i) each of the nuclease resistant anchor oligonucleotides is 3' end hydroxyl terminated;
each of the first primers and each of the second primers is 3' end phosphate terminated and 5' end adenylated;
initiating ligation of the first and second primers involves:
introducing a ligase with the primer fluid, the ligase being selected from the group consisting of T4 RNA Ligase I and Thermostable 5' App Ligase; and
adjusting a temperature of the flow cell to a reaction temperature for the ligase; and
prior to performing the sequencing operation, the method further includes deprotecting the first and second primers at the 3' ends;
or
(ii) each of the nuclease resistant anchor oligonucleotides is 3' end hydroxyl terminated;
each of the first primers is 3' end hydroxyl terminated and 5' end phosphate terminated, and is hybridized to a respective splint having a portion that is to hybridize to one of the nuclease resistant anchor oligonucleotides;
each of the second primers is 3' end hydroxyl terminated and 5' end phosphate terminated, and is hybridized to a respective splint having a portion that is to hybridize to one of the nuclease resistant anchor oligonucleotides; and
initiating ligation of the first and second primers involves:
introducing a ligase with the primer fluid, the ligase being selected from the group consisting of T4 RNA Ligase II and PBCV-1 DNA Ligase; and
adjusting a temperature of the flow cell to a splint ligation reaction temperature for the ligase;
or
(iii) each of the nuclease resistant anchor oligonucleotides is 3' end phosphate terminated;
each of the first primers and each of the second primers is 3' end hydroxyl terminated and 5' end hydroxyl terminated; and
initiating ligation of the first and second primers involves:
introducing an RtcB ligase with the primer fluid; and
adjusting a temperature of the flow cell to a reaction temperature for the RtcB ligase;
or
(iv) each of the nuclease resistant anchor oligonucleotides is 3' end hydroxyl terminated;
each of the first primers and each of the second primers is 3' end phosphate terminated and 5' end phosphate terminated; and
initiating ligation of the first and second primers involves:
introducing T4 RNA Ligase I with the primer fluid; and
adjusting a temperature of the flow cell to a reaction temperature for the T4 RNA Ligase I;
or
(v) introducing a second primer fluid into a flow cell;
initiating ligation of a third primer in the second primer fluid to at least some of the nuclease resistant anchor oligonucleotides and a fourth primer in the second primer fluid to some other of the nuclease resistant anchor oligonucleotides; and
performing a second sequencing operation using the third and fourth primers.

## Patentansprüche

1. Ein Primer-Set, das Folgendes beinhaltet:
einen ersten nukleaseresistenten Primer, der Folgendes umfasst:
eine erste Sequenz;
eine erste Spaltungsstelle, die an ein 3'-Ende der ersten Sequenz gebunden ist; und
eine erste nukleaseresistente Modifikation, die zwischen der ersten Sequenz und der ersten Spaltungsstelle eingebaut ist; und
einen zweiten nukleaseresistenten Primer, der Folgendes umfasst:
eine zweite Sequenz, die sich von der ersten Sequenz unterscheidet;
eine zweite nukleaseresistente Modifikation, die an einem 3'-Ende der zweiten Sequenz eingebaut ist; und
eine zweite Spaltungsstelle, die zwischen die zweite Sequenz und die zweite nukleaseresistente Modifikation gebunden ist, wobei sich die zweite Spaltungsstelle von der ersten Spaltungsstelle unterscheidet.

2. Primer-Set wie in Anspruch 1 definiert, wobei die erste nukleaseresistente Modifikation und die zweite nukleaseresistente Modifikation ein gleicher Typ von nukleaseresistenter Modifikation sind, wobei optional:
die erste und die zweite nukleaseresistente Modifikation aus der Gruppe ausgewählt sind, die aus einer Phosphorothioatbindung, Methylphosphonat, einem invertierten Nukleotid, einer Peptidnukleinsäure, einem Morpholino, 2'-O-Methyl und einem Nukleotid, das einen Phosphoramidit-C3-Spacer umfasst, besteht; und jede von der ersten und der zweiten Spaltungsstelle eine Nukleobase umfasst, die unabhängig aus der Gruppe ausgewählt ist, die aus einem Uracil, einem 8-Oxoguanin und einem Allylnukleotid besteht.

3. Eine wiederverwendbare Durchflusszelle, die Folgendes beinhaltet:
ein Substrat;
ein polymeres Hydrogel, das an mindestens einen Abschnitt des Substrats gebunden ist; und
ein Primer-Set, das an das polymere Hydrogel gebunden ist, wobei das Primer-Set aus der Gruppe ausgewählt ist, die aus Folgendem besteht:
i) einem ersten nukleaseresistenten Primer, der eine erste Spaltungsstelle umfasst; und
einem zweiten nukleaseresistenten Primer, der eine zweite Spaltungsstelle umfasst, die sich von der ersten Spaltungsstelle unterscheidet; und
ii) einem ersten nukleaseresistenten Primer, der eine Spaltungsstelle umfasst; und einem zweiten nukleaseresistenten Primer ohne eine Spaltungsstelle.

4. Wiederverwendbare Durchflusszelle wie in Anspruch 3 definiert, wobei:
(a) das Primer-Set i) ist;
der erste nukleaseresistente Primer Folgendes umfasst:
eine erste Sequenz;
die erste Spaltungsstelle, die an ein 3'-Ende der ersten Sequenz gebunden ist; und
eine erste nukleaseresistente Modifikation, die zwischen der ersten
Sequenz und der ersten Spaltungsstelle eingebaut ist; und
der zweite nukleaseresistente Primer Folgendes umfasst:
eine zweite Sequenz, die sich von der ersten Sequenz unterscheidet;
eine zweite nukleaseresistente Modifikation, die an einem 3'-Ende der zweiten Sequenz eingebaut ist; und
die zweite Spaltungsstelle, die zwischen die zweite Sequenz und die zweite nukleaseresistente Modifikation gebunden ist;
wobei optional:
die erste nukleaseresistente Modifikation und die zweite nukleaseresistente Modifikation ein gleicher Typ von nukleaseresistenter Modifikation sind;
die erste und die zweite nukleaseresistente Modifikation aus der Gruppe ausgewählt sind, die aus einer Phosphorothioatbindung, Methylphosphonat, einem invertierten Nukleotid, einer Peptidnukleinsäure, einem Morpholino, 2'-O-Methyl und einem Nukleotid, das einen Phosphoramidit-C3-Spacer umfasst, besteht; und
jede von der ersten und der zweiten Spaltungsstelle eine Nukleobase umfasst, die unabhängig aus der Gruppe ausgewählt ist, die aus einem Uracil, einem 8-Oxoguanin und einem Allylnukleotid besteht;
oder
(b) das Primer-Set ii) ist;
der erste nukleaseresistente Primer Folgendes umfasst:
eine erste Sequenz;
die Spaltungsstelle, die an ein 3'-Ende der ersten Sequenz gebunden ist; und
eine erste nukleaseresistente Modifikation, die zwischen der ersten Sequenz und der Spaltungsstelle eingebaut ist; und
der zweite nukleaseresistente Primer Folgendes umfasst:
eine zweite Sequenz, die sich von der ersten Sequenz unterscheidet; und
eine zweite nukleaseresistente Modifikation, die an einem 3'-Ende der zweiten Sequenz eingebaut ist;
wobei optional:
die erste nukleaseresistente Modifikation und die zweite nukleaseresistente Modifikation ein gleicher Typ von nukleaseresistenter Modifikation sind;
die erste und die zweite nukleaseresistente Modifikation aus der Gruppe ausgewählt sind, die aus einer Phosphorothioatbindung, Methylphosphonat, einem invertierten Nukleotid, einer Peptidnukleinsäure, einem Morpholino, 2'-O-Methyl und einem Nukleotid, das einen Phosphoramidit-C3-Spacer umfasst, besteht; und
die Spaltungsstelle des ersten nukleaseresistenten Primers eine Nukleobase umfasst, die aus der Gruppe ausgewählt ist, die aus einem Uracil, einem 8-Oxoguanin und einem Allylnukleotid besteht.

5. Ein Verfahren, das Folgendes beinhaltet:
(a) Einführen einer ersten Probe in eine Durchflusszelle, die Folgendes umfasst:
ein Substrat;
ein polymeres Hydrogel, das an mindestens einen Abschnitt des Substrats gebunden ist;
eine Vielzahl von Primer-Sets, die an das polymere Hydrogel gebunden sind, wobei jedes Primer-Set in der Vielzahl einen ersten Primer und einen zweiten Primer umfasst;
(b) Amplifizieren eines Bibliotheksfragments der ersten Probe, wodurch eine erste Vielzahl von Bibliotheksfragment-Amplikonen der ersten Probe, die jeweils an eine Vielzahl der ersten Primer gebunden sind, und eine zweite Vielzahl von Bibliotheksfragment-Amplikonen der ersten Probe, die jeweils an eine Vielzahl der zweiten Primer gebunden sind, erzeugt werden;
(c) Einführen eines Spaltfluids in die Durchflusszelle, wodurch die ersten Primer an Spaltungsstellen gespalten werden, wodurch die erste Vielzahl von Bibliotheksfragment-Amplikonen der ersten Probe entfernt wird; und
(d) Sequenzieren der zweiten Vielzahl von Bibliotheksfragment-Amplikonen der ersten Probe, wodurch eine Vielzahl naszierender Bibliotheksfragment-Stränge der ersten Probe erzeugt wird;
wobei entweder Option (I), (II) oder (III) gilt:
(I) in Schritt (a) der erste Primer ein nukleaseresistenter Primer ist, der eine erste Spaltungsstelle umfasst, und der zweite Primer ein zweiter nukleaseresistenter Primer ist, der eine zweite Spaltungsstelle umfasst, die sich von der ersten Spaltungsstelle unterscheidet, das in Schritt (b) amplifizierte Bibliotheksfragment ein erstes Bibliotheksfragment ist, in Schritt (c) das Spaltfluid ein Spaltfluid für die erste Spaltstelle ist und die Spaltungsstellen, an denen die ersten Primer gespalten werden, die ersten Spaltungsstellen sind; wobei das Verfahren ferner Folgendes beinhaltet:
(e) Einführen eines Nukleaseenzyms in die Durchflusszelle, wodurch die zweite Vielzahl von Bibliotheksfragment-Amplikonen der ersten Probe und die Vielzahl naszierender Bibliotheksfragment-Stränge der ersten Probe gespalten werden;
(f) Einführen einer zweiten Probe in die Durchflusszelle;
(g) Amplifizieren eines zweiten Bibliotheksfragments der zweiten Probe, wodurch eine erste Vielzahl von Bibliotheksfragment-Amplikonen der zweiten Probe, die jeweils an eine zweite Vielzahl der ersten nukleaseresistenten Primer gebunden sind, und eine zweite Vielzahl von Bibliotheksfragment-Amplikonen der zweiten Probe, die an eine zweite Vielzahl der zweiten nukleaseresistenten Primer gebunden sind, erzeugt werden;
(h) Einführen eines Spaltfluids für die zweite Spaltungsstelle in die Durchflusszelle, wodurch die zweiten nukleaseresistenten Primer an den zweiten Spaltungsstellen gespalten werden, wodurch die zweite Vielzahl von Bibliotheksfragment-Amplikonen der zweiten Probe entfernt wird; und
(i) Sequenzieren der ersten Vielzahl von Bibliotheksfragment-Amplikonen der zweiten Probe, wodurch eine Vielzahl naszierender Bibliotheksfragment-Stränge der zweiten Probe erzeugt wird;
ODER
(II) in Schritt (a) der erste Primer ein nukleaseresistenter Primer ist, der eine Spaltungsstelle umfasst, und der zweite Primer ein zweiter nukleaseresistenter Primer ohne Spaltungsstelle ist, wobei das Verfahren ferner Folgendes beinhaltet:
(e) Einführen eines Nukleaseenzyms in die Durchflusszelle, wodurch die zweite Vielzahl von Bibliotheksfragment-Amplikonen der ersten Probe und die Vielzahl naszierender Bibliotheksfragment-Stränge der ersten Probe gespalten werden;
(f) Einführen einer Vielzahl von Regenerationsoligonukleotiden in die Durchflusszelle, wodurch erste Abschnitte von mindestens einigen der Regenerationsoligonukleotide jeweils an verbleibende Abschnitte von mindestens einigen der ersten nukleaseresistenten Primer hybridisieren, wobei jedes Regenerationsoligonukleotid ferner einen zweiten Abschnitt umfasst, der zu einem abgespaltenen Abschnitt des ersten nukleaseresistenten Primers komplementär ist; und
(g) Initiieren einer Polymeraseverlängerung entlang jeweiliger zweiter Abschnitte der hybridisierten Regenerationsoligonukleotide, um den abgespaltenen Abschnitt der mindestens einigen der ersten nukleaseresistenten Primer zu regenerieren;
ODER
(III) in Schritt (a) der erste Primer eine erste Spaltungsstelle und eine zweite Spaltungsstelle, die sich von der ersten Spaltungsstelle unterscheidet, aufweist und der zweite Primer die erste Spaltungsstelle ohne die zweite Spaltungsstelle aufweist; in Schritt (c) die Spaltungsstellen, an denen die ersten Primer gespalten werden, die zweiten Spaltungsstellen sind; wobei das Verfahren ferner Folgendes beinhaltet:
(e) Einführen eines zweiten Spaltfluids in die Durchflusszelle, wodurch jeder von den ersten Primern und den zweiten Primern an ihren jeweiligen ersten Spaltungsstellen gespalten wird und verbleibende Abschnitte der ersten Primer und der zweiten Primer, die an das polymere Hydrogel gebunden sind, zurückbleiben; und
(f) sequentielles oder gleichzeitiges Regenerieren i) eines abgespaltenen Abschnitts von mindestens einigen der ersten Primer an den verbleibenden Abschnitten der mindestens einigen der ersten Primer und ii) eines abgespaltenen Abschnitts von mindestens einigen der zweiten Primer an den verbleibenden Abschnitten der mindestens einigen der zweiten Primer.

6. Verfahren wie in Anspruch 5 definiert, wobei das Verfahren das Verfahren von Option (I) ist und wobei:
jede der ersten Spaltungsstellen eine Uracil-Nukleobase umfasst;
das Spaltfluid für die erste Spaltungsstelle eine Mischung aus Uracil-DNA-Glycosylase und der DNA-Glycosylase-Lyase Endonuklease VIII oder ein aus Pyrococcus furiosus isoliertes Enzym umfasst;
jede der zweiten Spaltungsstellen eine 8-Oxoguanin-Nukleobase umfasst;
das Spaltfluid für die zweite Spaltungsstelle Formamidopyrimidin[fapy]-DNA-Glycosylase umfasst; und
das Nukleaseenzym aus der Gruppe ausgewählt ist, die aus Exonuklease I, thermolabiler Exonuklease I, Exonuklease T, Exonuklease VII, Mungobohnen-Nuklease, Nuklease P1, Exonuklease III, Exonuklease V, Nuklease BAL-31, DNase I und Mikrokokken-Nuklease besteht.

7. Verfahren gemäß Anspruch 5, wobei das Verfahren das Verfahren von Option (II) ist.

8. Verfahren wie in Anspruch 7 definiert, wobei:
(i) die Spaltungsstelle eine Uracil-Nukleobase umfasst;
das Spaltfluid eine Mischung aus Uracil-DNA-Glycosylase und der DNA-Glycosylase-Lyase Endonuklease VIII umfasst; und
das Nukleaseenzym aus der Gruppe ausgewählt ist, die aus Exonuklease I, thermolabiler Exonuklease I, Exonuklease T, Exonuklease VII, Mungobohnen-Nuklease, Nuklease P1, Exonuklease III, Exonuklease V, Nuklease BAL-31, DNase I und Mikrokokken-Nuklease besteht;
oder
(ii) die Polymeraseverlängerung entlang des zweiten Abschnitts des Regenerationsoligonukleotids mindestens die Spaltungsstelle des ersten nukleaseresistenten Primers regeneriert;
oder
(iii) das Verfahren ferner Folgendes beinhaltet:
Einführen einer zweiten Probe in die Durchflusszelle nach der Polymeraseverlängerung;
Amplifizieren eines Bibliotheksfragments der zweiten Probe, wodurch eine erste Vielzahl von Bibliotheksfragment-Amplikonen der zweiten Probe, die jeweils an eine zweite Vielzahl der ersten nukleaseresistenten Primer gebunden sind, und eine zweite Vielzahl von Bibliotheksfragment-Amplikonen der zweiten Probe, die an eine zweite Vielzahl der zweiten nukleaseresistenten Primer gebunden sind, erzeugt werden;
Einführen des Spaltfluids in die Durchflusszelle, wodurch die ersten nukleaseresistenten Primer an den Spaltungsstellen gespalten werden, wodurch die erste Vielzahl von Bibliotheksfragment-Amplikonen der zweiten Probe entfernt wird; und
Sequenzieren der zweiten Vielzahl von Bibliotheksfragment-Amplikonen der zweiten Probe, wodurch eine Vielzahl naszierender Bibliotheksfragment-Stränge der zweiten Probe erzeugt wird.

9. Eine Durchflusszelle, die Folgendes beinhaltet:
ein Substrat;
ein polymeres Hydrogel, das an mindestens einen Abschnitt des Substrats gebunden ist; und
ein Primer-Set, das an das polymere Hydrogel gebunden ist, wobei das Primer-Set Folgendes umfasst:
einen ersten Primer mit einer ersten Spaltungsstelle und einer zweiten Spaltungsstelle, die sich von der ersten Spaltungsstelle unterscheidet; und
einen zweiten Primer mit der ersten Spaltungsstelle ohne die zweite Spaltungsstelle.

10. Durchflusszelle wie in Anspruch 9 definiert, wobei:
(i) die erste Spaltungsstelle eine Restriktionsstelle ist; und
die zweite Spaltungsstelle eine Nukleobase umfasst, die aus der Gruppe ausgewählt ist, die aus einem Uracil, einem 8-Oxoguanin und einem Allylnukleotid besteht;
oder
(ii) die erste Spaltungsstelle und die zweite Spaltungsstelle jeweils eine Nukleobase umfassen, die unabhängig aus der Gruppe ausgewählt ist, die aus einem Uracil, einem 8-Oxoguanin und einem Allylnukleotid besteht;
oder
(iii) der erste und der zweite Primer nukleaseresistente Primer sind.

11. Verfahren gemäß Anspruch 5, wobei das Verfahren das Verfahren von Option (III) ist.

12. Verfahren wie in Anspruch 11 definiert, wobei:
jede der ersten Spaltungsstellen eine Restriktionsstelle ist;
das zweite Spaltfluid Folgendes umfasst:
eine Vielzahl erster Oligonukleotide, wobei jedes erste Oligonukleotid zu dem verbleibenden Abschnitt des ersten Primers komplementär ist;
eine Vielzahl zweiter Oligonukleotide, wobei jedes zweite Oligonukleotid zu dem verbleibenden Abschnitt des zweiten Primers komplementär ist; und
ein Restriktionsenzym; und
während sich das zweite Spaltfluid in der Durchflusszelle befindet, die Durchflusszelle einer ersten Temperatur ausgesetzt wird, um i) eine jeweilige Hybridisierung von Folgendem zu initiieren: von mindestens einigen der Vielzahl erster Oligonukleotide an mindestens einige der ersten Primer, die nach dem Sequenzieren einzelsträngig sind, von mindestens einigen der Vielzahl zweiter Oligonukleotide an mindestens einige der zweiten Primer, die nach dem Sequenzieren einzelsträngig sind, oder beides, und einer zweiten Temperatur ausgesetzt wird, um ii) eine doppelsträngige Spaltaktivität des Restriktionsenzyms zu initiieren.

13. Verfahren wie in Anspruch 12 definiert, das ferner Denaturieren beinhaltet, um die verbleibenden Abschnitte der mindestens einigen der ersten Primer und die verbleibenden Abschnitte von mindestens einigen der zweiten Primer einzelsträngig zu machen;
wobei optional:
(i) das Verfahren eine sequentielle Regeneration der abgespaltenen Abschnitte involviert; und
das sequentielle Regenerieren des abgespaltenen Abschnitts der mindestens einigen der ersten Primer und des abgespaltenen Abschnitts der mindestens einigen der zweiten Primer Folgendes involviert:
Einführen einer Vielzahl erster Regenerationsoligonukleotide in die Durchflusszelle, wodurch erste Abschnitte von mindestens einigen der ersten Regenerationsoligonukleotide jeweils an die verbleibenden Abschnitte der mindestens einigen der ersten Primer hybridisieren, wobei jedes erste Regenerationsoligonukleotid ferner einen zweiten Abschnitt umfasst, der zu dem abgespaltenen Abschnitt des ersten Primers komplementär ist;
Initiieren einer Polymeraseverlängerung entlang jeweiliger zweiter Abschnitte der hybridisierten ersten Regenerationsoligonukleotide, um den abgespaltenen Abschnitt der mindestens einigen der ersten Primer zu regenerieren;
Einführen einer Vielzahl zweiter Regenerationsoligonukleotide in die Durchflusszelle, wodurch erste Abschnitte von mindestens einigen der zweiten Regenerationsoligonukleotide jeweils an die verbleibenden Abschnitte der mindestens einigen der zweiten Primer hybridisieren, wobei jedes zweite Regenerationsoligonukleotid ferner einen zweiten Abschnitt umfasst, der zu dem abgespaltenen Abschnitt des zweiten Primers komplementär ist; und
Initiieren einer Polymeraseverlängerung entlang jeweiliger zweiter Abschnitte der hybridisierten zweiten Regenerationsoligonukleotide, um den abgespaltenen Abschnitt der mindestens einigen der zweiten Primer zu regenerieren;
oder
(ii) das Verfahren eine gleichzeitige Regeneration der abgespaltenen Abschnitte involviert; und
das gleichzeitige Regenerieren des abgespaltenen Abschnitts der mindestens einigen der ersten Primer und des abgespaltenen Abschnitts der mindestens einigen der zweiten Primer Folgendes involviert:
Einführen einer Vielzahl von Regenerationsplints, die erste Regenerationsplints und zweite Regenerationsplints umfassen, in die Durchflusszelle, wodurch Splintabschnitte der ersten Regenerationsplints jeweils an die verbleibenden Abschnitte der mindestens einigen der ersten Primer hybridisieren und Splintabschnitte der zweiten Regenerationsplints jeweils an die verbleibenden Abschnitte der mindestens einigen der zweiten Primer hybridisieren, wobei jeder erste Regenerationsplint ferner einen ersten Ligierabschnitt umfasst, der an den Splintabschnitt hybridisiert ist und eine Sequenz des abgespaltenen Abschnitts des ersten Primers aufweist, und jeder zweite Regenerationsplint ferner einen zweiten Ligierabschnitt umfasst, der an den Splintabschnitt hybridisiert ist und eine Sequenz des abgespaltenen Abschnitts des zweiten Primers aufweist; und
Initiieren einer Ligation von mindestens einigen der ersten Ligierabschnitte an die verbleibenden Abschnitte der mindestens einigen der ersten Primer und von mindestens einigen der zweiten Ligierabschnitte an die verbleibenden Abschnitte der mindestens einigen der zweiten Primer.

14. Verfahren wie in Anspruch 11 definiert, wobei:
das Verfahren eine sequentielle Regeneration der abgespaltenen Abschnitte involviert; jede der ersten Spaltungsstellen eine Nukleobase umfasst, die aus der Gruppe ausgewählt ist, die aus einem Uracil, einem 8-Oxoguanin und einem Allylnukleotid besteht; und
das zweite Spaltfluid Folgendes umfasst:
eine Vielzahl erster Oligonukleotide, wobei jedes erste Oligonukleotid zu dem verbleibenden Abschnitt des ersten Primers komplementär ist;
eine Vielzahl zweiter Oligonukleotide, wobei jedes zweite Oligonukleotid zu dem verbleibenden Abschnitt des zweiten Primers komplementär ist; und
ein Exzisionsenzym; und
während sich das zweite Spaltfluid in der Durchflusszelle befindet, die Durchflusszelle einer ersten Temperatur ausgesetzt wird, um i) eine jeweilige Hybridisierung von Folgendem zu initiieren: von mindestens einigen der Vielzahl erster Oligonukleotide an mindestens einige der ersten Primer, die nach dem Sequenzieren einzelsträngig sind, von mindestens einigen der Vielzahl zweiter Oligonukleotide an mindestens einige der zweiten Primer, die nach dem Sequenzieren einzelsträngig sind, oder beides, und einer zweiten Temperatur ausgesetzt wird, um ii) eine doppelsträngige Spaltaktivität des Exzisionsenzyms zu initiieren;
wobei optional:
(a) das Verfahren ferner Denaturieren beinhaltet, um die verbleibenden Abschnitte der mindestens einigen der ersten Primer und die verbleibenden Abschnitte von mindestens einigen der zweiten Primer einzelsträngig zu machen; oder
(b) das Verfahren ferner Denaturieren beinhaltet, um die verbleibenden Abschnitte der mindestens einigen der ersten Primer und die verbleibenden Abschnitte von mindestens einigen der zweiten Primer einzelsträngig zu machen, wobei ferner das sequentielle Regenerieren des abgespaltenen Abschnitts der mindestens einigen der ersten Primer und des abgespaltenen Abschnitts der mindestens einigen der zweiten Primer Folgendes involviert:
Einführen einer Vielzahl erster Regenerationsoligonukleotide in die Durchflusszelle, wodurch erste Abschnitte von mindestens einigen der ersten Regenerationsoligonukleotide jeweils an die verbleibenden Abschnitte der mindestens einigen der ersten Primer hybridisieren, wobei jedes erste Regenerationsoligonukleotid ferner einen zweiten Abschnitt umfasst, der zu dem abgespaltenen Abschnitt des ersten Primers komplementär ist;
Initiieren einer Polymeraseverlängerung entlang jeweiliger zweiter Abschnitte der hybridisierten ersten Regenerationsoligonukleotide, um den abgespaltenen Abschnitt der mindestens einigen der ersten Primer zu regenerieren;
Einführen einer Vielzahl zweiter Regenerationsoligonukleotide in die Durchflusszelle, wodurch erste Abschnitte von mindestens einigen der zweiten Regenerationsoligonukleotide jeweils an die verbleibenden Abschnitte der mindestens einigen der zweiten Primer hybridisieren, wobei jedes zweite Regenerationsoligonukleotid ferner einen zweiten Abschnitt umfasst, der zu dem abgespaltenen Abschnitt des zweiten Primers komplementär ist; und
Initiieren einer Polymeraseverlängerung entlang jeweiliger zweiter Abschnitte der hybridisierten zweiten Regenerationsoligonukleotide, um den abgespaltenen Abschnitt der mindestens einigen der zweiten Primer zu regenerieren.

15. Ein Durchflusszellenerzeugungskit, das Folgendes beinhaltet:
eine Durchflusszelle, die Folgendes umfasst:
ein Substrat;
ein polymeres Hydrogel, das an mindestens einen Abschnitt des Substrats gebunden ist; und
nukleaseresistente Ankeroligonukleotide, die an das polymere Hydrogel gebunden sind; und
ein Primerfluid, das Folgendes umfasst:
eine Vielzahl erster Primer, die jeweils an mindestens einige der nukleaseresistenten Ankeroligonukleotide ligiert werden sollen; und
eine Vielzahl zweiter Primer, die jeweils an mindestens einige andere der nukleaseresistenten Ankeroligonukleotide ligiert werden sollen.

16. Durchflusszellenerzeugungskit wie in Anspruch 15 definiert, wobei:
(a) jedes der nukleaseresistenten Ankeroligonukleotide am 3'-Ende hydroxylterminiert ist; und
jeder der ersten Primer und jeder der zweiten Primer am 3'-Ende phosphatterminiert und am 5'-Ende adenyliert ist;
oder
(b) jedes der nukleaseresistenten Ankeroligonukleotide am 3'-Ende hydroxylterminiert ist;
jeder der ersten Primer am 3'-Ende hydroxylterminiert und am 5'-Ende phosphatterminiert ist und an einen jeweiligen Splint hybridisiert ist, der einen Abschnitt aufweist, der an eines der nukleaseresistenten Ankeroligonukleotide hybridisieren soll; und
jeder der zweiten Primer am 3'-Ende hydroxylterminiert und am 5'-Ende phosphatterminiert ist und an einen jeweiligen Splint hybridisiert ist, der einen Abschnitt aufweist, der an eines der nukleaseresistenten Ankeroligonukleotide hybridisieren soll;
oder
(c) jedes der nukleaseresistenten Ankeroligonukleotide am 3'-Ende phosphatterminiert ist; und
jeder der ersten Primer und jeder der zweiten Primer am 3'-Ende hydroxylterminiert und am 5'-Ende hydroxylterminiert ist;
oder
(d) jedes der nukleaseresistenten Ankeroligonukleotide am 3'-Ende hydroxylterminiert ist; und
jeder der ersten Primer und jeder der zweiten Primer am 3'-Ende phosphatterminiert und am 5'-Ende phosphatterminiert ist;
oder
(e) jedes der nukleaseresistenten Ankeroligonukleotide eine nukleaseresistente Modifikation am 3'-Ende umfasst, die aus der Gruppe ausgewählt ist, die aus einer Phosphorothioatbindung, Methylphosphonat, einem invertierten Nukleotid, einer Peptidnukleinsäure, einem Morpholino, 2'-O-Methyl, einem phosphorylierten Nukleotid am 3'-Ende und einem Nukleotid, das einen Phosphoramidit-C3-Spacer umfasst, besteht;
oder
(f) (i) ein 3'-Ende jedes der nukleaseresistenten Ankeroligonukleotide ein RNA-Oligonukleotid oder ein 2'-O-Methyl umfasst; oder ii) ein 5'-Ende jedes der Vielzahl erster Primer und der Vielzahl zweiter Primer ein RNA-Oligonukleotid oder ein 2'-O-Methyl umfasst; oder iii) sowohl i) als auch ii).

17. Ein Verfahren, das Folgendes beinhaltet:
Einführen eines Primerfluids in eine Durchflusszelle, die Folgendes umfasst:
ein Substrat;
ein polymeres Hydrogel, das an mindestens einen Abschnitt des Substrats gebunden ist; und
nukleaseresistente Ankeroligonukleotide, die an das polymere Hydrogel gebunden sind;
Initiieren einer Ligation eines ersten Primers in dem Primerfluid an mindestens einige der nukleaseresistenten Ankeroligonukleotide und eines zweiten Primers in dem Primerfluid an mindestens einige andere der nukleaseresistenten Ankeroligonukleotide;
Durchführen eines Sequenzierungsvorgangs unter Verwendung des ersten und des zweiten Primers; und
Spalten des ersten und des zweiten Primers nach dem Sequenzierungsvorgang, um die nukleaseresistenten Ankeroligonukleotide freizulegen.

18. Verfahren wie in Anspruch 17 definiert, wobei:
(i) jedes der nukleaseresistenten Ankeroligonukleotide am 3'-Ende hydroxylterminiert ist;
jeder der ersten Primer und jeder der zweiten Primer am 3'-Ende phosphatterminiert und am 5'-Ende adenyliert ist;
das Initiieren einer Ligation der ersten und der zweiten Primer Folgendes involviert:
Einführen einer Ligase mit dem Primerfluid, wobei die Ligase aus der Gruppe ausgewählt ist, die aus T4-RNA-Ligase I und thermostabiler 5'-App-Ligase besteht; und
Einstellen einer Temperatur der Durchflusszelle auf eine Reaktionstemperatur für die Ligase; und
vor dem Durchführen des Sequenzierungsvorgangs das Verfahren ferner das Entschützen der ersten und der zweiten Primer an den 3'-Enden umfasst;
oder
(ii) jedes der nukleaseresistenten Ankeroligonukleotide am 3'-Ende hydroxylterminiert ist;
jeder der ersten Primer am 3'-Ende hydroxylterminiert und am 5'-Ende phosphatterminiert ist und an einen jeweiligen Splint hybridisiert ist, der einen Abschnitt aufweist, der an eines der nukleaseresistenten Ankeroligonukleotide hybridisieren soll;
jeder der zweiten Primer am 3'-Ende hydroxylterminiert und am 5'-Ende phosphatterminiert ist und an einen jeweiligen Splint hybridisiert ist, der einen Abschnitt aufweist, der an eines der nukleaseresistenten Ankeroligonukleotide hybridisieren soll; und
das Initiieren einer Ligation der ersten und der zweiten Primer Folgendes involviert:
Einführen einer Ligase mit dem Primerfluid, wobei die Ligase aus der Gruppe ausgewählt ist, die aus T4-RNA-Ligase II und PBCV-1-DNA-Ligase besteht; und
Einstellen einer Temperatur der Durchflusszelle auf eine Splint-Ligationsreaktionstemperatur für die Ligase;
oder
(iii) jedes der nukleaseresistenten Ankeroligonukleotide am 3'-Ende phosphatterminiert ist;
jeder der ersten Primer und jeder der zweiten Primer am 3'-Ende hydroxylterminiert und am 5'-Ende hydroxylterminiert ist; und
das Initiieren einer Ligation der ersten und der zweiten Primer Folgendes involviert:
Einführen einer RtcB-Ligase mit dem Primerfluid; und
Einstellen einer Temperatur der Durchflusszelle auf eine Reaktionstemperatur für die RtcB-Ligase;
oder
(iv) jedes der nukleaseresistenten Ankeroligonukleotide am 3'-Ende hydroxylterminiert ist;
jeder der ersten Primer und jeder der zweiten Primer am 3'-Ende phosphatterminiert und am 5'-Ende phosphatterminiert ist; und
das Initiieren einer Ligation der ersten und der zweiten Primer Folgendes involviert:
Einführen von T4-RNA-Ligase I mit dem Primerfluid; und
Einstellen einer Temperatur der Durchflusszelle auf eine Reaktionstemperatur für die T4-RNA-Ligase I;
oder
(v) Einführen eines zweiten Primerfluids in eine Durchflusszelle;
Initiieren einer Ligation eines dritten Primers in dem zweiten Primerfluid an mindestens einige der nukleaseresistenten Ankeroligonukleotide und eines vierten Primers in dem zweiten Primerfluid an einige andere der nukleaseresistenten Ankeroligonukleotide; und
Durchführen eines zweiten Sequenzierungsvorgangs unter Verwendung des dritten und des vierten Primers.

## Revendications

1. Un ensemble d'amorces, comprenant :
une première amorce résistante aux nucléases incluant :
une première séquence ;
un premier site de clivage attaché à une extrémité 3' de la première séquence ; et
une première modification résistante aux nucléases incorporée entre la première séquence et le premier site de clivage ; et
une deuxième amorce résistante aux nucléases incluant :
une deuxième séquence qui est différente de la première séquence ;
une deuxième modification résistante aux nucléases incorporée à une extrémité 3' de la deuxième séquence ; et
un deuxième site de clivage attaché entre la deuxième séquence et la deuxième modification résistante aux nucléases, dans lequel le deuxième site de clivage est différent du premier site de clivage.

2. L'ensemble d'amorces tel que défini dans la revendication 1, dans lequel la première modification résistante aux nucléases et la deuxième modification résistante aux nucléases sont un même type de modification résistante aux nucléases, facultativement dans lequel :
les première et deuxième modifications résistantes aux nucléases sont sélectionnées dans le groupe constitué d'une liaison phosphorothioate, de méthylphosphonate, d'un nucléotide inversé, d'un acide nucléique peptidique, d'un morpholino, de 2'-O-méthyle, et d'un nucléotide incluant un espaceur phosphoramidite C3 ; et chacun des premier et deuxième sites de clivage inclut une nucléobase indépendamment sélectionnée dans le groupe constitué d'un uracile, d'une 8-oxoguanine, et d'un allyl-nucléotide.

3. Une cellule d'écoulement réutilisable, comprenant :
un substrat ;
un hydrogel polymère attaché à au moins une portion du substrat ; et
un ensemble d'amorces attaché à l'hydrogel polymère, l'ensemble d'amorces étant sélectionné dans le groupe constitué de :
i) une première amorce résistante aux nucléases incluant un premier site de clivage ; et
une deuxième amorce résistante aux nucléases incluant un deuxième site de clivage qui est différent du premier site de clivage ; et
ii) une première amorce résistante aux nucléases incluant un site de clivage ; et une deuxième amorce résistante aux nucléases sans aucun site de clivage.

4. La cellule d'écoulement réutilisable telle que définie dans la revendication 3, dans laquelle :
(a) l'ensemble d'amorces est i) ;
la première amorce résistante aux nucléases inclut :
une première séquence ;
le premier site de clivage attaché à une extrémité 3' de la première séquence ; et
une première modification résistante aux nucléases incorporée entre la première séquence et le premier site de clivage ; et
la deuxième amorce résistante aux nucléases inclut :
une deuxième séquence qui est différente de la première séquence ;
une deuxième modification résistante aux nucléases incorporée à une extrémité 3' de la deuxième séquence ; et
le deuxième site de clivage attaché entre la deuxième séquence et la deuxième modification résistante aux nucléases ;
facultativement dans laquelle :
la première modification résistante aux nucléases et la deuxième modification résistante aux nucléases sont un même type de modification résistante aux nucléases ;
les première et deuxième modifications résistantes aux nucléases sont sélectionnées dans le groupe constitué d'une liaison phosphorothioate, de méthylphosphonate, d'un nucléotide inversé, d'un acide nucléique peptidique,
d'un morpholino, de 2'-O-méthyle, et d'un nucléotide incluant un espaceur phosphoramidite C3 ; et
chacun des premier et deuxième sites de clivage inclut une nucléobase indépendamment sélectionnée dans le groupe constitué d'un uracile, d'une 8-oxoguanine, et d'un allyl-nucléotide ;
ou
(b) l'ensemble d'amorces est ii) ;
la première amorce résistante aux nucléases inclut :
une première séquence ;
le site de clivage attaché à une extrémité 3' de la première séquence ; et
une première modification résistante aux nucléases incorporée entre la première séquence et le site de clivage ; et
la deuxième amorce résistante aux nucléases inclut :
une deuxième séquence qui est différente de la première séquence ; et
une deuxième modification résistante aux nucléases incorporée à une extrémité 3' de la deuxième séquence ;
facultativement dans laquelle :
la première modification résistante aux nucléases et la deuxième modification résistante aux nucléases sont un même type de modification résistante aux nucléases ;
les première et deuxième modifications résistantes aux nucléases sont sélectionnées dans le groupe constitué d'une liaison phosphorothioate, de méthylphosphonate, d'un nucléotide inversé, d'un acide nucléique peptidique, d'un morpholino, de 2'-O-méthyle, et d'un nucléotide incluant un espaceur phosphoramidite C3 ; et
le site de clivage de la première amorce résistante aux nucléases inclut une nucléobase sélectionnée dans le groupe constitué d'un uracile, d'une 8-oxoguanine, et d'un allyl-nucléotide.

5. Un procédé, comprenant :
(a) l'introduction d'un premier échantillon dans une cellule d'écoulement incluant :
un substrat ;
un hydrogel polymère attaché à au moins une portion du substrat ;
une pluralité d'ensembles d'amorces attachés à l'hydrogel polymère, chaque ensemble d'amorces dans la pluralité incluant une première amorce et une deuxième amorce ;
(b) l'amplification d'un fragment de bibliothèque du premier échantillon, générant ainsi une première pluralité d'amplicons de fragment de bibliothèque de premier échantillon respectivement attachés à une pluralité des premières amorces et une deuxième pluralité d'amplicons de fragment de bibliothèque de premier échantillon respectivement attachés à une pluralité des deuxièmes amorces ;
(c) l'introduction d'un fluide de clivage dans la cellule d'écoulement, clivant ainsi les premières amorces au niveau de sites de clivage, moyennant quoi la première pluralité d'amplicons de fragment de bibliothèque de premier échantillon sont retirés ; et
(d) le séquençage de la deuxième pluralité d'amplicons de fragment de bibliothèque de premier échantillon, générant ainsi une pluralité de brins naissants de fragment de bibliothèque de premier échantillon ;
dans lequel soit l'option (I), soit l'option (II), soit l'option (III) s'applique :
(I) à l'étape (a), ladite première amorce est une amorce résistante aux nucléases incluant un premier site de clivage et ladite deuxième amorce est une deuxième amorce résistante aux nucléases incluant un deuxième site de clivage qui est différent du premier site de clivage, ledit fragment de bibliothèque amplifié à l'étape (b) est un premier fragment de bibliothèque, à l'étape (c), ledit fluide de clivage est un fluide de clivage de premier site de clivage et les sites de clivage au niveau desquels les premières amorces sont clivées sont les premiers sites de clivage ; le procédé comprenant en outre :
(e) l'introduction d'une enzyme nucléase dans la cellule d'écoulement, clivant ainsi la deuxième pluralité d'amplicons de fragment de bibliothèque de premier échantillon et la pluralité de brins naissants de fragment de bibliothèque de premier échantillon ;
(f) l'introduction d'un deuxième échantillon dans la cellule d'écoulement ;
(g) l'amplification d'un deuxième fragment de bibliothèque du deuxième échantillon, générant ainsi une première pluralité d'amplicons de fragment de bibliothèque de deuxième échantillon respectivement attachés à une deuxième pluralité des premières amorces résistantes aux nucléases et une deuxième pluralité d'amplicons de fragment de bibliothèque de deuxième échantillon attachés à une deuxième pluralité des deuxièmes amorces résistantes aux nucléases ;
(h) l'introduction d'un fluide de clivage de deuxième site de clivage dans la cellule d'écoulement, clivant ainsi les deuxièmes amorces résistantes aux nucléases au niveau des deuxièmes sites de clivage, moyennant quoi la deuxième pluralité d'amplicons de fragment de bibliothèque de deuxième échantillon sont retirés ; et
(i) le séquençage de la première pluralité d'amplicons de fragment de bibliothèque de deuxième échantillon, générant ainsi une pluralité de brins naissants de fragment de bibliothèque de deuxième échantillon ;
OU
(II) à l'étape (a), ladite première amorce est une amorce résistante aux nucléases incluant un site de clivage et ladite deuxième amorce est une deuxième amorce résistante aux nucléases sans aucun site de clivage, le procédé comprenant en outre :
(e) l'introduction d'une enzyme nucléase dans la cellule d'écoulement, clivant ainsi la deuxième pluralité d'amplicons de fragment de bibliothèque de premier échantillon et la pluralité de brins naissants de fragment de bibliothèque de premier échantillon ;
(f) l'introduction d'une pluralité d'oligonucléotides de régénération dans la cellule d'écoulement, moyennant quoi des premières portions d'au moins certains des oligonucléotides de régénération s'hybrident respectivement à des portions restantes d'au moins certaines des premières amorces résistantes aux nucléases, chaque oligonucléotide de régénération incluant en outre une deuxième portion qui est complémentaire d'une portion clivée de la première amorce résistante aux nucléases ; et
(g) le commencement d'une extension polymérase le long de deuxièmes portions respectives des oligonucléotides de régénération hybridés pour régénérer la portion clivée des au moins certaines des premières amorces résistantes aux nucléases ;
OU
(III) à l'étape (a), ladite première amorce a un premier site de clivage et un deuxième site de clivage qui est différent du premier site de clivage, et ladite deuxième amorce a le premier site de clivage sans le deuxième site de clivage ; à l'étape (c), les sites de clivage au niveau desquels les premières amorces sont clivées sont les deuxièmes sites de clivage ; le procédé comprenant en outre :
(e) l'introduction d'un deuxième fluide de clivage dans la cellule d'écoulement, clivant ainsi chacune des premières amorces et des deuxièmes amorces au niveau de leurs premiers sites de clivage respectifs et laissant des portions restantes des premières amorces et des deuxièmes amorces attachées à l'hydrogel polymère ; et
(f) la régénération séquentielle ou simultanée i) d'une portion clivée d'au moins certaines des premières amorces au niveau des portions restantes des au moins certaines des premières amorces, et ii) d'une portion clivée d'au moins certaines des deuxièmes amorces au niveau des portions restantes des au moins certaines des deuxièmes amorces.

6. Le procédé tel que défini dans la revendication 5, le procédé étant le procédé de l'option (I) et dans lequel :
chacun des premiers sites de clivage inclut une nucléobase uracile ;
le fluide de clivage de premier site de clivage inclut un mélange d'ADN-glycosylase uracile et de l'endonucléase VIII d'ADN-glycosylase lyase ou d'une enzyme isolée de Pyrococcus furiosus ;
chacun des deuxièmes sites de clivage inclut une nucléobase 8-oxoguanine ;
le fluide de clivage de deuxième site de clivage inclut de l'ADN-glycosylase formamidopyrimidine [fapy] ; et
l'enzyme nucléase est sélectionnée dans le groupe constitué de l'exonucléase I, de l'exonucléase I thermolabile, de l'exonucléase T, de l'exonucléase VII, de la nucléase de haricot mungo, de la nucléase P1, de l'exonucléase III, de l'exonucléase V, de la nucléase BAL-31, de la DNase I, et de la nucléase micrococcale.

7. Le procédé selon la revendication 5, le procédé étant le procédé de l'option (II).

8. Le procédé tel que défini dans la revendication 7, dans lequel :
(i) le site de clivage inclut une nucléobase uracile ;
le fluide de clivage inclut un mélange d'ADN-glycosylase uracile et de l'endonucléase VIII d'ADN-glycosylase lyase ; et
l'enzyme nucléase est sélectionnée dans le groupe constitué de l'exonucléase I, de l'exonucléase I thermolabile, de l'exonucléase T, de l'exonucléase VII, de la nucléase de haricot mungo, de la nucléase P1, de l'exonucléase III, de l'exonucléase V, de la nucléase BAL-31, de la DNase I, et de la nucléase micrococcale ;
ou
(ii) l'extension polymérase le long de la deuxième portion de l'oligonucléotide de régénération régénère au moins le site de clivage de la première amorce résistante aux nucléases ;
ou
(iii) le procédé comprend en outre :
l'introduction d'un deuxième échantillon dans la cellule d'écoulement après l'extension polymérase ;
l'amplification d'un fragment de bibliothèque du deuxième échantillon, générant ainsi une première pluralité d'amplicons de fragment de bibliothèque de deuxième échantillon respectivement attachés à une deuxième pluralité des premières amorces résistantes aux nucléases et une deuxième pluralité d'amplicons de fragment de bibliothèque de deuxième échantillon attachés à une deuxième pluralité des deuxièmes amorces résistantes aux nucléases ;
l'introduction du fluide de clivage dans la cellule d'écoulement, clivant ainsi les premières amorces résistantes aux nucléases au niveau des sites de clivage,
moyennant quoi la première pluralité d'amplicons de fragment de bibliothèque de deuxième échantillon sont retirés ; et
le séquençage de la deuxième pluralité d'amplicons de fragment de bibliothèque de deuxième échantillon, générant ainsi une pluralité de brins naissants de fragment de bibliothèque de deuxième échantillon.

9. Une cellule d'écoulement, comprenant :
un substrat ;
un hydrogel polymère attaché à au moins une portion du substrat ; et
un ensemble d'amorces attaché à l'hydrogel polymère, l'ensemble d'amorces incluant :
une première amorce ayant un premier site de clivage et un deuxième site de clivage qui est différent du premier site de clivage ; et
une deuxième amorce ayant le premier site de clivage sans le deuxième site de clivage.

10. La cellule d'écoulement telle que définie dans la revendication 9, dans laquelle :
(i) le premier site de clivage est un site de restriction ; et
le deuxième site de clivage inclut une nucléobase sélectionnée dans le groupe constitué d'un uracile, d'une 8-oxoguanine, et d'un allyl-nucléotide ;
ou
(ii) le premier site de clivage et le deuxième site de clivage incluent chacun une nucléobase indépendamment sélectionnée dans le groupe constitué d'un uracile, d'une 8-oxoguanine, et d'un allyl-nucléotide ;
ou
(iii) les première et deuxième amorces sont des amorces résistantes aux nucléases.

11. Le procédé selon la revendication 5, le procédé étant le procédé de l'option (III).

12. Le procédé tel que défini dans la revendication 11, dans lequel :
chacun des premiers sites de clivage est un site de restriction ;
le deuxième fluide de clivage inclut :
une pluralité de premiers oligonucléotides, chaque premier oligonucléotide étant complémentaire de la portion restante de la première amorce ;
une pluralité de deuxièmes oligonucléotides, chaque deuxième oligonucléotide étant complémentaire de la portion restante de la deuxième amorce ; et
une enzyme de restriction ; et
pendant que le deuxième fluide de clivage est dans la cellule d'écoulement, la cellule d'écoulement est exposée à une première température i) pour commencer une hybridation respective : d'au moins certains de la pluralité de premiers oligonucléotides à au moins certaines des premières amorces qui sont simple brin après séquençage,
d'au moins certains de la pluralité de deuxièmes oligonucléotides à au moins certaines des deuxièmes amorces qui sont simple brin après séquençage, ou les deux, et à une deuxième température ii) pour commencer une activité de clivage double brin de l'enzyme de restriction.

13. Le procédé tel que défini dans la revendication 12, comprenant en outre une dénaturation pour rendre les portions restantes des au moins certaines des premières amorces et les portions restantes d'au moins certaines des deuxièmes amorces simple brin ;
facultativement :
(i) le procédé impliquant la régénération séquentielle des portions clivées ; et la régénération séquentielle de la portion clivée des au moins certaines des premières amorces et de la portion clivée des au moins certaines des deuxièmes amorces impliquant :
l'introduction d'une pluralité de premiers oligonucléotides de régénération dans la cellule d'écoulement, moyennant quoi des premières portions d'au moins certains des premiers oligonucléotides de régénération s'hybrident respectivement aux portions restantes des au moins certaines des premières amorces, chaque premier oligonucléotide de régénération incluant en outre une deuxième portion qui est complémentaire de la portion clivée de la première amorce ;
le commencement d'une extension polymérase le long de deuxièmes portions respectives des premiers oligonucléotides de régénération hybridés pour régénérer la portion clivée des au moins certaines des premières amorces ;
l'introduction d'une pluralité de deuxièmes oligonucléotides de régénération dans la cellule d'écoulement, moyennant quoi des premières portions d'au moins certains des deuxièmes oligonucléotides de régénération s'hybrident respectivement aux portions restantes des au moins certaines des deuxièmes amorces, chaque deuxième oligonucléotide de régénération incluant en outre une deuxième portion qui est complémentaire de la portion clivée de la deuxième amorce ; et
le commencement d'une extension polymérase le long de deuxièmes portions respectives des deuxièmes oligonucléotides de régénération hybridés pour régénérer la portion clivée des au moins certaines des
deuxièmes amorces ;
ou
(ii) le procédé impliquant la régénération simultanée des portions clivées ; et la régénération simultanée de la portion clivée des au moins certaines des premières amorces et de la portion clivée des au moins certaines des deuxièmes amorces impliquant :
l'introduction d'une pluralité d'attelles de régénération, incluant des premières attelles de régénération et des deuxièmes attelles de régénération, dans la cellule d'écoulement, moyennant quoi des portions d'attelle des premières attelles de régénération s'hybrident respectivement aux portions restantes des au moins certaines des premières amorces et
des portions d'attelle des deuxièmes attelles de régénération s'hybrident respectivement aux portions restantes des au moins certaines des deuxièmes amorces, chaque première attelle de régénération incluant en outre une première portion de ligature hybridée à la portion d'attelle et
ayant une séquence de la portion clivée de la première amorce, et chaque deuxième attelle de régénération incluant en outre une deuxième portion de ligature hybridée à la portion d'attelle et ayant une séquence de la portion clivée de la deuxième amorce ; et
le commencement d'une ligature d'au moins certaines des premières portions de ligature aux portions restantes des au moins certaines des premières amorces et d'au moins certaines des deuxièmes portions de ligature aux portions restantes des au moins certaines des deuxièmes amorces.

14. Le procédé tel que défini dans la revendication 11 :
le procédé impliquant la régénération séquentielle des portions clivées ;
chacun des premiers sites de clivage incluant une nucléobase sélectionnée dans le groupe constitué d'un uracile, d'une 8-oxoguanine, et d'un allyl-nucléotide ; et
le deuxième fluide de clivage incluant :
une pluralité de premiers oligonucléotides, chaque premier oligonucléotide étant complémentaire de la portion restante de la première amorce ;
une pluralité de deuxièmes oligonucléotides, chaque deuxième oligonucléotide étant complémentaire de la portion restante de la deuxième amorce ; et
une enzyme d'excision ; et
pendant que le deuxième fluide de clivage est dans la cellule d'écoulement, la cellule d'écoulement est exposée à une première température i) pour commencer une hybridation respective : d'au moins certains de la pluralité de premiers oligonucléotides à au moins certaines des premières amorces qui sont simple brin après séquençage,
d'au moins certains de la pluralité de deuxièmes oligonucléotides à au moins certaines des deuxièmes amorces qui sont simple brin après séquençage, ou les deux, et à une deuxième température ii) pour commencer une activité de clivage double brin de l'enzyme d'excision ;
facultativement :
(a) le procédé comprenant en outre une dénaturation pour rendre les portions restantes des au moins certaines des premières amorces et les portions restantes d'au moins certaines des deuxièmes amorces simple brin ; ou
(b) le procédé comprenant en outre une dénaturation pour rendre les portions restantes des au moins certaines des premières amorces et les portions restantes d'au moins certaines des deuxièmes amorces simple brin, en outre la régénération séquentielle de la portion clivée des au moins certaines des premières amorces et de la portion clivée des au moins certaines des deuxièmes amorces impliquant :
l'introduction d'une pluralité de premiers oligonucléotides de régénération dans la cellule d'écoulement, moyennant quoi des premières portions d'au moins certains des premiers oligonucléotides de régénération s'hybrident respectivement aux portions restantes des au moins certaines des premières amorces, chaque premier oligonucléotide de régénération incluant en outre une deuxième portion qui est complémentaire de la portion clivée de la première amorce ;
le commencement d'une extension polymérase le long de deuxièmes portions respectives des premiers oligonucléotides de régénération hybridés pour régénérer la portion clivée des au moins certaines des premières amorces ;
l'introduction d'une pluralité de deuxièmes oligonucléotides de régénération dans la cellule d'écoulement, moyennant quoi des premières portions d'au moins certains des deuxièmes oligonucléotides de régénération s'hybrident respectivement aux portions restantes des au moins certaines des deuxièmes amorces, chaque deuxième oligonucléotide de régénération incluant en outre une deuxième portion qui est complémentaire de la portion clivée de la deuxième amorce ; et
le commencement d'une extension polymérase le long de deuxièmes portions respectives des deuxièmes oligonucléotides de régénération hybridés pour régénérer la portion clivée des au moins certaines des deuxièmes amorces.

15. Un kit de génération de cellule d'écoulement, comprenant :
une cellule d'écoulement incluant :
un substrat ;
un hydrogel polymère attaché à au moins une portion du substrat ; et
des oligonucléotides d'ancrage résistants aux nucléases attachés à l'hydrogel polymère ; et
un fluide d'amorce incluant :
une pluralité de premières amorces devant être respectivement ligaturées à au moins certains des oligonucléotides d'ancrage résistants aux nucléases ; et
une pluralité de deuxièmes amorces devant être respectivement ligaturées à au moins certains autres des oligonucléotides d'ancrage résistants aux nucléases.

16. Le kit de génération de cellule d'écoulement tel que défini dans la revendication 15, dans lequel :
(a) chacun des oligonucléotides d'ancrage résistants aux nucléases est terminé par hydroxyle à l'extrémité 3' ; et
chacune des premières amorces et chacune des deuxièmes amorces est terminée par phosphate à l'extrémité 3' et adénylée à l'extrémité 5' ;
ou
(b) chacun des oligonucléotides d'ancrage résistants aux nucléases est terminé par hydroxyle à l'extrémité 3' ;
chacune des premières amorces est terminée par hydroxyle à l'extrémité 3' et terminée par phosphate à l'extrémité 5', et est hybridée à une attelle respective ayant une portion qui doit s'hybrider à l'un des oligonucléotides d'ancrage résistants aux nucléases ; et
chacune des deuxièmes amorces est terminée par hydroxyle à l'extrémité 3' et terminée par phosphate à l'extrémité 5', et est hybridée à une attelle respective ayant une portion qui doit s'hybrider à l'un des oligonucléotides d'ancrage résistants aux nucléases ;
ou
(c) chacun des oligonucléotides d'ancrage résistants aux nucléases est terminé par phosphate à l'extrémité 3' ; et
chacune des premières amorces et chacune des deuxièmes amorces est terminée par hydroxyle à l'extrémité 3' et terminée par hydroxyle à l'extrémité 5' ;
ou
(d) chacun des oligonucléotides d'ancrage résistants aux nucléases est terminé par hydroxyle à l'extrémité 3' ; et
chacune des premières amorces et chacune des deuxièmes amorces est terminée par phosphate à l'extrémité 3' et terminée par phosphate à l'extrémité 5' ;
ou
(e) chacun des oligonucléotides d'ancrage résistants aux nucléases inclut une modification résistante aux nucléases à l'extrémité 3' sélectionnée dans le groupe constitué d'une liaison phosphorothioate, de méthylphosphonate, d'un nucléotide inversé, d'un acide nucléique peptidique, d'un morpholino, de 2'-O-méthyle, d'un nucléotide phosphorylé à l'extrémité 3', et d'un nucléotide incluant un espaceur phosphoramidite C3 ;
ou
(f) (i) une extrémité 3' de chacun des oligonucléotides d'ancrage résistants aux nucléases inclut un oligonucléotide d'ARN ou un 2'-O-méthyle ; ou ii) une extrémité 5' de chacune de la pluralité de premières amorces et de la pluralité de deuxièmes amorces inclut un oligonucléotide d'ARN ou un 2'-O-méthyle ; ou iii) à la fois i) et ii).

17. Un procédé, comprenant :
l'introduction d'un fluide d'amorce dans une cellule d'écoulement incluant :
un substrat ;
un hydrogel polymère attaché à au moins une portion du substrat ; et
des oligonucléotides d'ancrage résistants aux nucléases attachés à l'hydrogel polymère ;
le commencement d'une ligature d'une première amorce dans le fluide d'amorce à au moins certains des oligonucléotides d'ancrage résistants aux nucléases et d'une deuxième amorce dans le fluide d'amorce à au moins certains autres des oligonucléotides d'ancrage résistants aux nucléases ;
la réalisation d'une opération de séquençage en utilisant les première et deuxième amorces ; et
le clivage des première et deuxième amorces après l'opération de séquençage pour exposer les oligonucléotides d'ancrage résistants aux nucléases.

18. Le procédé tel que défini dans la revendication 17, dans lequel :
(i) chacun des oligonucléotides d'ancrage résistants aux nucléases est terminé par hydroxyle à l'extrémité 3' ;
chacune des premières amorces et chacune des deuxièmes amorces est terminée par phosphate à l'extrémité 3' et adénylée à l'extrémité 5' ;
le commencement d'une ligature des première et deuxième amorces implique :
l'introduction d'une ligase avec le fluide d'amorce, la ligase étant sélectionnée dans le groupe constitué de l'ARN Ligase I T4 et de l'App Ligase 5' thermostable ; et
le réglage d'une température de la cellule d'écoulement sur une température de réaction pour la ligase ; et
avant la réalisation de l'opération de séquençage, le procédé inclut en outre la déprotection des première et deuxième amorces aux extrémités 3';
ou
(ii) chacun des oligonucléotides d'ancrage résistants aux nucléases est terminé par hydroxyle à l'extrémité 3' ;
chacune des premières amorces est terminée par hydroxyle à l'extrémité 3' et terminée par phosphate à l'extrémité 5', et est hybridée à une attelle respective ayant une portion qui doit s'hybrider à l'un des oligonucléotides d'ancrage résistants aux nucléases ;
chacune des deuxièmes amorces est terminée par hydroxyle à l'extrémité 3' et terminée par phosphate à l'extrémité 5', et est hybridée à une attelle respective ayant une portion qui doit s'hybrider à l'un des oligonucléotides d'ancrage résistants aux nucléases ; et
le commencement d'une ligature des première et deuxième amorces implique :
l'introduction d'une ligase avec le fluide d'amorce, la ligase étant sélectionnée dans le groupe constitué de l'ARN Ligase II T4 et de l'ADN Ligase PBCV-1 ; et
le réglage d'une température de la cellule d'écoulement sur une température de réaction de ligature d'attelle pour la ligase ;
ou
(iii) chacun des oligonucléotides d'ancrage résistants aux nucléases est terminé par phosphate à l'extrémité 3' ;
chacune des premières amorces et chacune des deuxièmes amorces est terminée par hydroxyle à l'extrémité 3' et terminée par hydroxyle à l'extrémité 5' ; et
le commencement d'une ligature des première et deuxième amorces implique :
l'introduction d'une ligase RtcB avec le fluide d'amorce ; et
le réglage d'une température de la cellule d'écoulement sur une température de réaction pour la ligase RtcB ;
ou
(iv) chacun des oligonucléotides d'ancrage résistants aux nucléases est terminé par hydroxyle à l'extrémité 3' ;
chacune des premières amorces et chacune des deuxièmes amorces est terminée par phosphate à l'extrémité 3' et terminée par phosphate à l'extrémité 5' ; et
le commencement d'une ligature des première et deuxième amorces implique :
l'introduction de l'ARN Ligase I T4 avec le fluide d'amorce ; et
le réglage d'une température de la cellule d'écoulement sur une température de réaction pour l'ARN Ligase I T4 ;
ou
(v) l'introduction d'un deuxième fluide d'amorce dans une cellule d'écoulement ;
le commencement d'une ligature d'une troisième amorce dans le deuxième fluide d'amorce à au moins certains des oligonucléotides d'ancrage résistants aux nucléases et d'une quatrième amorce dans le deuxième fluide d'amorce à certains autres des oligonucléotides d'ancrage résistants aux nucléases ; et
la réalisation d'une deuxième opération de séquençage en utilisant les troisième et quatrième amorces.
